# EUROPEAN PATENT SPECIFICATION

(11) **EP 0 665 739 B1**
(45) Date of publication and mention of the grant of the patent: **04.08.1999**
(21) Application number: 93922212.1
(22) Date of filing: 15.09.1993
(51) Int. Cl.: A61K 6/00, A61L 27/00

(54) **MORPHOGEN-INDUCED PERIODONTAL TISSUE REGENERATION**
DURCH MORPHOGEN INDUZIERTE PERIODONTOLGEWEBEREGENERATION
REGENERATION TISSULAIRE PERIODONTIQUE INDUITE PAR DES MORPHOGENES

(30) Priority: 15.09.1992 US 945285
(43) Date of publication of application: 09.08.1995
(73) Proprietor: CREATIVE BIOMOLECULES, INC., Hopkinton, MA 01748 (US)
(72) Inventor: KUBERASAMPATH, Thangavel, Medway, MA 02053 (US); RUEGER, David, C., Hopkinton, MA 01748 (US); OPPERMANN, Hermann, Medway, MA 02053 (US); COHEN, Charles, M., Weston, MA 02193 (US); PANG, Roy, H., L., Etna, NH 03750 (US); SMART, John, E., Weston, MA 02193 (US); OZKAYNAK, Engin, Milford, MA 01757 (US)
(74) Representative: Price, Vincent Andrew
(86) International application number: US9308742
(87) International publication number: WO9406399

(56) References cited:
- EP-A- 0 495 284
- WO-A-88/00205
- WO-A-90/10017
- WO-A-92/15323
- US-A- 5 011 691

SEQUENCE LISTING

(1) GENERAL INFORMATION:

(i) APPLICANT:
(A) NAME: CREATIVE BIOMOLECULES, INC.
(B) STREET: 45 SOUTH STREET
(C) CITY: HOPKINTON
(D) STATE: MA
(E) COUNTRY: USA
(F) POSTAL CODE (ZIP): 01748
(G) TELEPHONE: 1-508-435-9001
(H) TELEFAX: 1-508-435-0454
(I) TELEX:

(ii) TITLE OF INVENTION: MORPHOGEN-INDUCED PERIODONTAL
TISSUE REGENERATION

(iii) NUMBER OF SEQUENCES: 33

(iv) CORRESPONDENCE ADDRESS:
(A) ADDRESSEE: CREATIVE BIOMOLECULES, INC.
(B) STREET: 45 SOUTH STREET
(C) CITY: HOPKINTON
(D) STATE: MA
(E) COUNTRY: USA
(F) ZIP: 01748

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: KELLEY ESQ, ROBIN D.
(B) REGISTRATION NUMBER: 34,637
(C) REFERENCE/DOCKET NUMBER: CRP-067

(ix) TELECOMMUNICATION INFORMATION:
(A) TELEPHONE: 617/248-7477
(B) TELEFAX: 617/248-7100

(2) INFORMATION FOR SEQ ID NO:1:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ1
/note= "WHEREIN EACH XAA INDEPENDENTLY INDICATES
ONE OF THE 20 NATURALLY-OCCURING L-ISOMER, A-AMINO
ACIDS, OR A DERIVATIVE THEREOF."

(xi) SEQUENCE DESCRIPTION: SEQ ID NO:1:

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Cys Xaa Xaa Xaa Cys Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Cys Cys Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

(2) INFORMATION FOR SEQ ID NO:2:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ2
/note= "WHEREIN EACH XAA INDEPENDENTLY INDICATES
ONE OF THE 20 NATURALLY OCCURING L-ISOMER A-AMINO
ACIDS, OR A DERIVATIVE THEREOF."

(xi) SEQUENCE DESCRIPTION: SEQ ID NO:2:

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Cys Xaa Xaa Xaa Cys Xaa Xaa Xaa
            20                  25                  30

Xaa Xaa Xaa Cys Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Cys Cys Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

(2) INFORMATION FOR SEQ ID NO:3:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ3
/note= "WHEREIN EACH XAA IS INDEPENDENTLY SELECTED
FROM A GROUP OF ONE OR MORE SPECIFIED AMINO ACIDS
AS DEFINED IN THE SPECIFICATION."

(xi) SEQUENCE DESCRIPTION: SEQ ID NO:3:

Leu Tyr Val Xaa Phe Xaa Xaa Xaa Gly Trp Xaa Xaa Trp Xaa Xaa Ala
1               5                   10                  15

Pro Xaa Gly Xaa Xaa Ala Xaa Tyr Cys Xaa Gly Xaa Cys Xaa Xaa Pro
            20                  25                  30

Xaa Xaa Xaa Xaa Xaa Xaa Xaa Xaa Asn His Ala Xaa Xaa Xaa Xaa Leu
        35                  40                  45

Xaa Xaa Xaa Xaa Xaa Xaa Xaa Xaa Xaa Xaa Xaa Xaa Cys Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Val Xaa Leu Xaa Xaa Xaa Xaa Xaa Met Xaa Val Xaa Xaa Cys Gly Cys
                85                  90                  95

Xaa

(2) INFORMATION FOR SEQ ID NO:4:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102
(D) OTHER INFORMATION: /label= GENERIC-SEQ4
/note= "WHEREIN EACH XAA IS INDEPENDENTLY SELECTED
FROM A GROUP OF ONE OR MORE SPECIFIED AMINO ACIDS
AS DEFINED IN THE SPECIFICATION."

(xi) SEQUENCE DESCRIPTION: SEQ ID NO:4:

Cys Xaa Xaa Xaa Xaa Leu Tyr Val Xaa Phe Xaa Xaa Xaa Gly Trp Xaa
1               5                   10                  15

Xaa Trp Xaa Xaa Ala Pro Xaa Gly Xaa Xaa Ala Xaa Tyr Cys Xaa Gly
            20                  25                  30

Xaa Cys Xaa Xaa Pro Xaa Xaa Xaa Xaa Xaa Xaa Xaa Xaa Asn His Ala
        35                  40                  45

Xaa Xaa Xaa Xaa Leu Xaa Xaa Xaa Xaa Xaa Xaa Xaa Xaa Xaa Xaa Xaa
    50                  55                  60

Xaa Cys Cys Xaa Pro Xaa Xaa Xaa Xaa Xaa Xaa Xaa Xaa Leu Xaa Xaa
65                  70                  75                  80

Xaa Xaa Xaa Xaa Xaa Val Xaa Leu Xaa Xaa Xaa Xaa Xaa Met Xaa Val
                85                  90                  95

Xaa Xaa Cys Gly Cys Xaa
            100

(2) INFORMATION FOR SEQ ID NO:5:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: Homo sapiens
(F) TISSUE TYPE: HIPPOCAMPUS

(ix) FEATURE:
(A) NAME/KEY: Protein
(B) LOCATION: 1..139
(D) OTHER INFORMATION: /label= hOP1-MATURE

(xi) SEQUENCE DESCRIPTION: SEQ ID NO:5:

Ser Thr Gly Ser Lys Gln Arg Ser Gln Asn Arg Ser Lys Thr Pro Lys
1               5                   10                  15

Asn Gln Glu Ala Leu Arg Met Ala Asn Val Ala Glu Asn Ser Ser Ser
            20                  25                  30

Asp Gln Arg Gln Ala Cys Lys Lys His Glu Leu Tyr Val Ser Phe Arg
        35                  40                  45

Asp Leu Gly Trp Gln Asp Trp Ile Ile Ala Pro Glu Gly Tyr Ala Ala
    50                  55                  60

Tyr Tyr Cys Glu Gly Glu Cys Ala Phe Pro Leu Asn Ser Tyr Met Asn
65                  70                  75                  80

Ala Thr Asn His Ala Ile Val Gln Thr Leu Val His Phe Ile Asn Pro
                85                  90                  95

Glu Thr Val Pro Lys Pro Cys Cys Ala Pro Thr Gln Leu Asn Ala Ile
            100                 105                 110

Ser Val Leu Tyr Phe Asp Asp Ser Ser Asn Val Ile Leu Lys Lys Tyr
        115                 120                 125

Arg Asn Met Val Val Arg Ala Cys Gly Cys His
    130                 135

(2) INFORMATION FOR SEQ ID NO:6:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE
(F) TISSUE TYPE: EMBRYO

(ix) FEATURE:
(A) NAME/KEY: Protein
(B) LOCATION: 1..139
(D) OTHER INFORMATION: /label= MOP1-MATURE

(xi) SEQUENCE DESCRIPTION: SEQ ID NO:6:

Ser Thr Gly Gly Lys Gln Arg Ser Gln Asn Arg Ser Lys Thr Pro Lys
1               5                   10                  15

Asn Gln Glu Ala Leu Arg Met Ala Ser Val Ala Glu Asn Ser Ser Ser
            20                  25                  30

Asp Gln Arg Gln Ala Cys Lys Lys His Glu Leu Tyr Val Ser Phe Arg
        35                  40                  45

Asp Leu Gly Trp Gln Asp Trp Ile Ile Ala Pro Glu Gly Tyr Ala Ala
    50                  55                  60

Tyr Tyr Cys Glu Gly Glu Cys Ala Phe Pro Leu Asn Ser Tyr Met Asn
65                  70                  75                  80

Ala Thr Asn His Ala Ile Val Gln Thr Leu Val His Phe Ile Asn Pro
                85                  90                  95

Asp Thr Val Pro Lys Pro Cys Cys Ala Pro Thr Gln Leu Asn Ala Ile
            100                 105                 110

Ser Val Leu Tyr Phe Asp Asp Ser Ser Asn Val Ile Leu Lys Lys Tyr
        115                 120                 125

Arg Asn Met Val Val Arg Ala Cys Gly Cys His
    130                 135

(2) INFORMATION FOR SEQ ID NO:7:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS
(F) TISSUE TYPE: HIPPOCAMPUS

(ix) FEATURE:
(A) NAME/KEY: Protein
(B) LOCATION: 1..139
(D) OTHER INFORMATION: /label= HOP2-MATURE

(xi) SEQUENCE DESCRIPTION: SEQ ID NO:7:

Ala Val Arg Pro Leu Arg Arg Arg Gln Pro Lys Lys Ser Asn Glu Leu
1               5                   10                  15

Pro Gln Ala Asn Arg Leu Pro Gly Ile Phe Asp Asp Val His Gly Ser
            20                  25                  30

His Gly Arg Gln Val Cys Arg Arg His Glu Leu Tyr Val Ser Phe Gln
        35                  40                  45

Asp Leu Gly Trp Leu Asp Trp Val Ile Ala Pro Gln Gly Tyr Ser Ala
    50                  55                  60

Tyr Tyr Cys Glu Gly Glu Cys Ser Phe Pro Leu Asp Ser Cys Met Asn
65                  70                  75                  80

Ala Thr Asn His Ala Ile Leu Gln Ser Leu Val His Leu Met Lys Pro
                85                  90                  95

Asn Ala Val Pro Lys Ala Cys Cys Ala Pro Thr Lys Leu Ser Ala Thr
            100                 105                 110

Ser Val Leu Tyr Tyr Asp Ser Ser Asn Asn Val Ile Leu Arg Lys His
        115                 120                 125

Arg Asn Met Val Val Lys Ala Cys Gly Cys His
    130                 135

(2) INFORMATION FOR SEQ ID NO:8:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE
(F) TISSUE TYPE: EMBRYO

(ix) FEATURE:
(A) NAME/KEY: Protein
(B) LOCATION: 1..139
(D) OTHER INFORMATION: /label= MOP2-MATURE

(xi) SEQUENCE DESCRIPTION: SEQ ID NO:8:

Ala Ala Arg Pro Leu Lys Arg Arg Gln Pro Lys Lys Thr Asn Glu Leu
1               5                   10                  15

Pro His Pro Asn Lys Leu Pro Gly Ile Phe Asp Asp Gly His Gly Ser
            20                  25                  30

Arg Gly Arg Glu Val Cys Arg Arg His Glu Leu Tyr Val Ser Phe Arg
        35                  40                  45

Asp Leu Gly Trp Leu Asp Trp Val Ile Ala Pro Gln Gly Tyr Ser Ala
    50                  55                  60

Tyr Tyr Cys Glu Gly Glu Cys Ala Phe Pro Leu Asp Ser Cys Met Asn
65                  70                  75                  80

Ala Thr Asn His Ala Ile Leu Gln Ser Leu Val His Leu Met Lys Pro
                85                  90                  95

Asp Val Val Pro Lys Ala Cys Cys Ala Pro Thr Lys Leu Ser Ala Thr
            100                 105                 110

Ser Val Leu Tyr Tyr Asp Ser Ser Asn Asn Val Ile Leu Arg Lys His
        115                 120                 125

Arg Asn Met Val Val Lys Ala Cys Gly Cys His
    130                 135

(2) INFORMATION FOR SEQ ID NO:9:

    (i) SEQUENCE CHARACTERISTICS:
        (A) LENGTH: 101 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: bovinae

    (ix) FEATURE:
        (A) NAME/KEY: Protein
        (B) LOCATION: 1..101
        (D) OTHER INFORMATION: /label= CBMP-2A-FX

    (xi) SEQUENCE DESCRIPTION: SEQ ID NO:9:

Cys Lys Arg His Pro Leu Tyr Val Asp Phe Ser Asp Val Gly Trp Asn
1               5                   10                  15

Asp Trp Ile Val Ala Pro Pro Gly Tyr His Ala Phe Tyr Cys His Gly
            20                  25                  30

Glu Cys Pro Phe Pro Leu Ala Asp His Leu Asn Ser Thr Asn His Ala
        35                  40                  45

Ile Val Gln Thr Leu Val Asn Ser Val Asn Ser Lys Ile Pro Lys Ala
    50                  55                  60

Cys Cys Val Pro Thr Glu Leu Ser Ala Ile Ser Met Leu Tyr Leu Asp
65                  70                  75                  80

Glu Asn Glu Lys Val Val Leu Lys Asn Tyr Gln Asp Met Val Val Glu
                85                  90                  95

Gly Cys Gly Cys Arg
            100

(2) INFORMATION FOR SEQ ID NO:10:

    (i) SEQUENCE CHARACTERISTICS:
        (A) LENGTH: 101 amino acids
        (B) TYPE: amino acid
        (C) STRANDEDNESS: single
        (D) TOPOLOGY: linear

(ii) MOLECULE TYPE: protein

(vi) ORIGINAL SOURCE:
(A) ORGANISM: HOMO SAPIENS
(F) TISSUE TYPE: hippocampus

(ix) FEATURE:
(A) NAME/KEY: Protein
(B) LOCATION: 1..101
(D) OTHER INFORMATION: /label= CBMP-2B-FX

(xi) SEQUENCE DESCRIPTION: SEQ ID NO:10:

Cys Arg Arg His Ser Leu Tyr Val Asp Phe Ser Asp Val Gly Trp Asn
1               5                   10                  15

Asp Trp Ile Val Ala Pro Pro Gly Tyr Gln Ala Phe Tyr Cys His Gly
            20                  25                  30

Asp Cys Pro Phe Pro Leu Ala Asp His Leu Asn Ser Thr Asn His Ala
        35                  40                  45

Ile Val Gln Thr Leu Val Asn Ser Val Asn Ser Ser Ile Pro Lys Ala
    50                  55                  60

Cys Cys Val Pro Thr Glu Leu Ser Ala Ile Ser Met Leu Tyr Leu Asp
65                  70                  75                  80

Glu Tyr Asp Lys Val Val Leu Lys Asn Tyr Gln Glu Met Val Val Glu
                85                  90                  95

Gly Cys Gly Cys Arg
            100

(2) INFORMATION FOR SEQ ID NO:11:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: DROSOPHILA MELANOGASTER

(ix) FEATURE:
(A) NAME/KEY: Protein
(B) LOCATION: 1..101
(D) OTHER INFORMATION: /label= DPP-FX

(xi) SEQUENCE DESCRIPTION: SEQ ID NO:11:

Cys Arg Arg His Ser Leu Tyr Val Asp Phe Ser Asp Val Gly Trp Asp
1               5                   10                  15

Asp Trp Ile Val Ala Pro Leu Gly Tyr Asp Ala Tyr Tyr Cys His Gly
            20                  25                  30

Lys Cys Pro Phe Pro Leu Ala Asp His Phe Asn Ser Thr Asn His Ala
        35                  40                  45

Val Val Gln Thr Leu Val Asn Asn Asn Asn Pro Gly Lys Val Pro Lys
    50                  55                  60

Ala Cys Cys Val Pro Thr Gln Leu Asp Ser Val Ala Met Leu Tyr Leu
65                  70                  75                  80

Asn Asp Gln Ser Thr Val Val Leu Lys Asn Tyr Gln Glu Met Thr Val
                85                  90                  95

Val Gly Cys Gly Cys Arg
            100

(2) INFORMATION FOR SEQ ID NO:12:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: XENOPUS

(ix) FEATURE:
(A) NAME/KEY: Protein
(B) LOCATION: 1..102
(D) OTHER INFORMATION: /label= VGL-FX

(xi) SEQUENCE DESCRIPTION: SEQ ID NO:12:

Cys Lys Lys Arg His Leu Tyr Val Glu Phe Lys Asp Val Gly Trp Gln
1               5                   10                  15

Asn Trp Val Ile Ala Pro Gln Gly Tyr Met Ala Asn Tyr Cys Tyr Gly
            20                  25                  30

Glu Cys Pro Tyr Pro Leu Thr Glu Ile Leu Asn Gly Ser Asn His Ala
        35                  40                  45

Ile Leu Gln Thr Leu Val His Ser Ile Glu Pro Glu Asp Ile Pro Leu
    50                  55                  60

Pro Cys Cys Val Pro Thr Lys Met Ser Pro Ile Ser Met Leu Phe Tyr
65                  70                  75                  80

Asp Asn Asn Asp Asn Val Val Leu Arg His Tyr Glu Asn Met Ala Val
                85                  90                  95

Asp Glu Cys Gly Cys Arg
            100

(2) INFORMATION FOR SEQ ID NO:13:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE

(ix) FEATURE:
(A) NAME/KEY: Protein
(B) LOCATION: 1..102
(D) OTHER INFORMATION: /label= VGR-1-FX

(xi) SEQUENCE DESCRIPTION: SEQ ID NO:13:

Cys Lys Lys His Glu Leu Tyr Val Ser Phe Gln Asp Val Gly Trp Gln
1               5                   10                  15

Asp Trp Ile Ile Ala Pro Lys Gly Tyr Ala Ala Asn Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Val Met Asn Pro Glu Tyr Val Pro Lys
    50                  55                  60

Pro Cys Cys Ala Pro Thr Lys Val Asn Ala Ile Ser Val Leu Tyr Phe
65                  70                  75                  80

Asp Asp Asn Ser Asn Val Ile Leu Lys Lys Tyr Arg Asn Met Val Val
                85                  90                  95

Arg Ala Cys Gly Cys His
                100

(2) INFORMATION FOR SEQ ID NO:14:

    (i) SEQUENCE CHARACTERISTICS:
        (A) LENGTH: 106 amino acids
        (B) TYPE: amino acid
        (C) STRANDEDNESS: single
        (D) TOPOLOGY: linear

    (ii) MOLECULE TYPE: protein

    (iii) HYPOTHETICAL: NO

    (iv) ANTI-SENSE: NO

    (vi) ORIGINAL SOURCE:
        (A) ORGANISM: Homo sapiens
        (F) TISSUE TYPE: brain

    (ix) FEATURE:
        (A) NAME/KEY: Protein
        (B) LOCATION: 1..106
        (D) OTHER INFORMATION: /note= "GDF-1 (fx)"

    (xi) SEQUENCE DESCRIPTION: SEQ ID NO:14:

Cys Arg Ala Arg Arg Leu Tyr Val Ser Phe Arg Glu Val Gly Trp His
1               5                   10                  15

Arg Trp Val Ile Ala Pro Arg Gly Phe Leu Ala Asn Tyr Cys Gln Gly
            20                  25                  30

Gln Cys Ala Leu Pro Val Ala Leu Ser Gly Ser Gly Gly Pro Pro Ala
        35                  40                  45

Leu Asn His Ala Val Leu Arg Ala Leu Met His Ala Ala Ala Pro Gly
    50                  55                  60

Ala Ala Asp Leu Pro Cys Cys Val Pro Ala Arg Leu Ser Pro Ile Ser
65                  70                  75                  80

Val Leu Phe Phe Asp Asn Ser Asp Asn Val Val Leu Arg Gln Tyr Glu
                85                  90                  95

Asp Met Val Val Asp Glu Cys Gly Cys Arg
            100                 105

(2) INFORMATION FOR SEQ ID NO:15:

    (i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

Cys Xaa Xaa Xaa Xaa
1               5

(2) INFORMATION FOR SEQ ID NO:16:

(i) SEQUENCE CHARACTERISTICS:
(A) LENGTH: 1822 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: HOMO SAPIENS
(F) TISSUE TYPE: HIPPOCAMPUS

(ix) FEATURE:
(A) NAME/KEY: CDS
(B) LOCATION: 49..1341
(C) IDENTIFICATION METHOD: experimental
(D) OTHER INFORMATION: /function= "OSTEOGENIC PROTEIN"
/product= "OP1"
/evidence= EXPERIMENTAL
/standard_name= "OP1"

(xi) SEQUENCE DESCRIPTION: SEQ ID NO:16:

GGTGCGGGCC CGGAGCCCGG AGCCCGGGTA GCGCGTAGAG CCGGCGCG ATG CAC GTG      57
                                                     Met His Val
                                                      1

CGC TCA CTG CGA GCT GCG GCG CCG CAC AGC TTC GTG GCG CTC TGG GCA      105
Arg Ser Leu Arg Ala Ala Ala Pro His Ser Phe Val Ala Leu Trp Ala
    5                   10                  15

CCC CTG TTC CTG CTG CGC TCC GCC CTG GCC GAC TTC AGC CTG GAC AAC 153
Pro Leu Phe Leu Leu Arg Ser Ala Leu Ala Asp Phe Ser Leu Asp Asn
20                  25                  30                  35

GAG GTG CAC TCG AGC TTC ATC CAC CGG CGC CTC CGC AGC CAG GAG CGG 201
Glu Val His Ser Ser Phe Ile His Arg Arg Leu Arg Ser Gln Glu Arg
                40                  45                  50

CGG GAG ATG CAG CGC GAG ATC CTC TCC ATT TTG GGC TTG CCC CAC CGC 249
Arg Glu Met Gln Arg Glu Ile Leu Ser Ile Leu Gly Leu Pro His Arg
            55                  60                  65

CCG CGC CCG CAC CTC CAG GGC AAG CAC AAC TCG GCA CCC ATG TTC ATG 297
Pro Arg Pro His Leu Gln Gly Lys His Asn Ser Ala Pro Met Phe Met
        70                  75                  80

CTG GAC CTG TAC AAC GCC ATG GCG GTG GAG GAG GGC GGC GGG CCC GGC 345
Leu Asp Leu Tyr Asn Ala Met Ala Val Glu Glu Gly Gly Gly Pro Gly
    85                  90                  95

GGC CAG GGC TTC TCC TAC CCC TAC AAG GCC GTC TTC AGT ACC CAG GGC 393
Gly Gln Gly Phe Ser Tyr Pro Tyr Lys Ala Val Phe Ser Thr Gln Gly
100                 105                 110                 115

CCC CCT CTG GCC AGC CTG CAA GAT AGC CAT TTC CTC ACC GAC GCC GAC 441
Pro Pro Leu Ala Ser Leu Gln Asp Ser His Phe Leu Thr Asp Ala Asp
                120                 125                 130

ATG GTC ATG AGC TTC GTC AAC CTC GTG GAA CAT GAC AAG GAA TTC TTC 489
Met Val Met Ser Phe Val Asn Leu Val Glu His Asp Lys Glu Phe Phe
            135                 140                 145

CAC CCA CGC TAC CAC CAT CGA GAG TTC CGG TTT GAT CTT TCC AAG ATC 537
His Pro Arg Tyr His His Arg Glu Phe Arg Phe Asp Leu Ser Lys Ile
        150                 155                 160

CCA GAA GGG GAA GCT GTC ACG GCA GCC GAA TTC CGG ATC TAC AAG GAC 585
Pro Glu Gly Glu Ala Val Thr Ala Ala Glu Phe Arg Ile Tyr Lys Asp
    165                 170                 175

TAC ATC CGG GAA CGC TTC GAC AAT GAG ACG TTC CGG ATC AGC GTT TAT 633
Tyr Ile Arg Glu Arg Phe Asp Asn Glu Thr Phe Arg Ile Ser Val Tyr
180                 185                 190                 195

CAG GTG CTC CAG GAG CAC TTG GGC AGG GAA TCG GAT CTC TTC CTG CTC 681
Gln Val Leu Gln Glu His Leu Gly Arg Glu Ser Asp Leu Phe Leu Leu
                200                 205                 210

GAC AGC CGT ACC CTC TGG GCC TCG GAG GAG GGC TGG CTG GTG TTT GAC 729
Asp Ser Arg Thr Leu Trp Ala Ser Glu Glu Gly Trp Leu Val Phe Asp
            215                 220                 225

ATC ACA GCC ACC AGC AAC CAC TGG GTG GTC AAT CCG CGG CAC AAC CTG 777
Ile Thr Ala Thr Ser Asn His Trp Val Val Asn Pro Arg His Asn Leu
        230                 235                 240

GGC CTG CAG CTC TCG GTG GAG ACG CTG GAT GGG CAG AGC ATC AAC CCC 825
Gly Leu Gln Leu Ser Val Glu Thr Leu Asp Gly Gln Ser Ile Asn Pro
    245                 250                 255

AAG TTG GCG GGC CTG ATT GGG CGG CAC GGG CCC CAG AAC AAG CAG CCC 873
Lys Leu Ala Gly Leu Ile Gly Arg His Gly Pro Gln Asn Lys Gln Pro
260                 265                 270                 275

TTC ATG GTG GCT TTC TTC AAG GCC ACG GAG GTC CAC TTC CGC AGC ATC 921
Phe Met Val Ala Phe Phe Lys Ala Thr Glu Val His Phe Arg Ser Ile
                280                 285                 290

CGG TCC ACG GGG AGC AAA CAG CGC AGC CAG AAC CGC TCC AAG ACG CCC 969
Arg Ser Thr Gly Ser Lys Gln Arg Ser Gln Asn Arg Ser Lys Thr Pro
            295                 300                 305

AAG AAC CAG GAA GCC CTG CGG ATG GCC AAC GTG GCA GAG AAC AGC AGC 1017
Lys Asn Gln Glu Ala Leu Arg Met Ala Asn Val Ala Glu Asn Ser Ser
        310                 315                 320

AGC GAC CAG AGG CAG GCC TGT AAG AAG CAC GAG CTG TAT GTC AGC TTC 1065
Ser Asp Gln Arg Gln Ala Cys Lys Lys His Glu Leu Tyr Val Ser Phe
    325                 330                 335

CGA GAC CTG GGC TGG CAG GAC TGG ATC ATC GCG CCT GAA GGC TAC GCC 1113
Arg Asp Leu Gly Trp Gln Asp Trp Ile Ile Ala Pro Glu Gly Tyr Ala
340                 345                 350                 355

GCC TAC TAC TGT GAG GGG GAG TGT GCC TTC CCT CTG AAC TCC TAC ATG 1161
Ala Tyr Tyr Cys Glu Gly Glu Cys Ala Phe Pro Leu Asn Ser Tyr Met
                360                 365                 370

AAC GCC ACC AAC CAC GCC ATC GTG CAG ACG CTG GTC CAC TTC ATC AAC 1209
Asn Ala Thr Asn His Ala Ile Val Gln Thr Leu Val His Phe Ile Asn
            375                 380                 385

CCG GAA ACG GTG CCC AAG CCC TGC TGT GCG CCC ACG CAG CTC AAT GCC 1257
Pro Glu Thr Val Pro Lys Pro Cys Cys Ala Pro Thr Gln Leu Asn Ala
        390                 395                 400

ATC TCC GTC CTC TAC TTC GAT GAC AGC TCC AAC GTC ATC CTG AAG AAA 1305
Ile Ser Val Leu Tyr Phe Asp Asp Ser Ser Asn Val Ile Leu Lys Lys
    405                 410                 415

TAC AGA AAC ATG GTG GTC CGG GCC TGT GGC TGC CAC TAGCTCCTCC 1351
Tyr Arg Asn Met Val Val Arg Ala Cys Gly Cys His
420                 425                 430

GAGAATTCAG ACCCTTTGGG GCCAAGTTTT TCTGGATCCT CCATTGCTCG CCTTGGCCAG 1411

GAACCAGCAG ACCAACTGCC TTTTGTGAGA CCTTCCCCTC CCTATCCCCA ACTTTAAAGG 1471

TGTGAGAGTA TTAGGAAACA TGAGCAGCAT ATGGCTTTTG ATCAGTTTTT CAGTGGCAGC 1531

ATCCAATGAA CAAGATCCTA CAAGCTGTGC AGGCAAAACC TAGCAGGAAA AAAAAACAAC 1591

GCATAAAGAA AAATGGCCGG GCCAGGTCAT TGGCTGGGAA GTCTCAGCCA TGCACGGACT 1651

CGTTTCCAGA GGTAATTATG AGCGCCTACC AGCCAGGCCA CCCAGCCGTG GGAGGAAGGG 1711

GGCGTGGCAA GGGGTGGGCA CATTGGTGTC TGTGCGAAAG GAAAATTGAC CCGGAAGTTC 1771

CTGTAATAAA TGTCACAATA AAACGAATGA ATGAAAAAAA AAAAAAAAAA A 1822

(2) INFORMATION FOR SEQ ID NO:17:

(i) SEQUENCE CHARACTERISTICS:
(A) LENGTH: 431 amino acids
(B) TYPE: amino acid
(D) TOPOLOGY: linear

(ii) MOLECULE TYPE: protein

(xi) SEQUENCE DESCRIPTION: SEQ ID NO:17:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

(2) INFORMATION FOR SEQ ID NO:18:

(i) SEQUENCE CHARACTERISTICS:
(A) LENGTH: 1873 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: MURIDAE
(F) TISSUE TYPE: EMBRYO

(ix) FEATURE:
(A) NAME/KEY: CDS
(B) LOCATION: 104..1393
(D) OTHER INFORMATION: /function= "OSTEOGENIC PROTEIN"
/product= "MOP1"
/note= "MOP1 (CDNA)"

(xi) SEQUENCE DESCRIPTION: SEQ ID NO:18:

CTGCAGCAAG TGACCTCGGG TCGTGGACCG CTGCCCTGCC CCCTCCGCTG CCACCTGGGG 60

CGGCGCGGGC CCGGTGCCCC GGATCGCGCG TAGAGCCGGC GCG ATG CAC GTG CGC 115
Met His Val Arg
1

TCG CTG CGC GCT GCG GCG CCA CAC AGC TTC GTG GCG CTC TGG GCG CCT 163
Ser Leu Arg Ala Ala Ala Pro His Ser Phe Val Ala Leu Trp Ala Pro
5                   10                  15                  20

CTG TTC TTG CTG CGC TCC GCC CTG GCC GAT TTC AGC CTG GAC AAC GAG 211
Leu Phe Leu Leu Arg Ser Ala Leu Ala Asp Phe Ser Leu Asp Asn Glu
                25                  30                  35

GTG CAC TCC AGC TTC ATC CAC CGG CGC CTC CGC AGC CAG GAG CGG CGG 259
Val His Ser Ser Phe Ile His Arg Arg Leu Arg Ser Gln Glu Arg Arg
            40                  45                  50

GAG ATG CAG CGG GAG ATC CTG TCC ATC TTA GGG TTG CCC CAT CGC CCG 307
Glu Met Gln Arg Glu Ile Leu Ser Ile Leu Gly Leu Pro His Arg Pro
        55                  60                  65

CGC CCG CAC CTC CAG GGA AAG CAT AAT TCG GCG CCC ATG TTC ATG TTG 355
Arg Pro His Leu Gln Gly Lys His Asn Ser Ala Pro Met Phe Met Leu
    70                  75                  80

GAC CTG TAC AAC GCC ATG GCG GTG GAG GAG AGC GGG CCG GAC GGA CAG 403
Asp Leu Tyr Asn Ala Met Ala Val Glu Glu Ser Gly Pro Asp Gly Gln
85                  90                  95                  100

GGC TTC TCC TAC CCC TAC AAG GCC GTC TTC AGT ACC CAG GGC CCC CCT 451
Gly Phe Ser Tyr Pro Tyr Lys Ala Val Phe Ser Thr Gln Gly Pro Pro
                105                 110                 115

TTA GCC AGC CTG CAG GAC AGC CAT TTC CTC ACT GAC GCC GAC ATG GTC 499
Leu Ala Ser Leu Gln Asp Ser His Phe Leu Thr Asp Ala Asp Met Val
            120                 125                 130

ATG AGC TTC GTC AAC CTA GTG GAA CAT GAC AAA GAA TTC TTC CAC CCT 547
Met Ser Phe Val Asn Leu Val Glu His Asp Lys Glu Phe Phe His Pro
        135                 140                 145

CGA TAC CAC CAT CGG GAG TTC CGG TTT GAT CTT TCC AAG ATC CCC GAG 595
Arg Tyr His His Arg Glu Phe Arg Phe Asp Leu Ser Lys Ile Pro Glu
    150                 155                 160

GGC GAA CGG GTG ACC GCA GCC GAA TTC AGG ATC TAT AAG GAC TAC ATC 643
Gly Glu Arg Val Thr Ala Ala Glu Phe Arg Ile Tyr Lys Asp Tyr Ile
165                 170                 175                 180

CGG GAG CGA TTT GAC AAC GAG ACC TTC CAG ATC ACA GTC TAT CAG GTG 691
Arg Glu Arg Phe Asp Asn Glu Thr Phe Gln Ile Thr Val Tyr Gln Val
                185                 190                 195

CTC CAG GAG CAC TCA GGC AGG GAG TCG GAC CTC TTC TTG CTG GAC AGC 739
Leu Gln Glu His Ser Gly Arg Glu Ser Asp Leu Phe Leu Leu Asp Ser
            200                 205                 210

CGC ACC ATC TGG GCT TCT GAG GAG GGC TGG TTG GTG TTT GAT ATC ACA 787
Arg Thr Ile Trp Ala Ser Glu Glu Gly Trp Leu Val Phe Asp Ile Thr
        215                 220                 225

GCC ACC AGC AAC CAC TGG GTG GTC AAC CCT CGG CAC AAC CTG GGC TTA 835
Ala Thr Ser Asn His Trp Val Val Asn Pro Arg His Asn Leu Gly Leu
    230                 235                 240

CAG CTC TCT GTG GAG ACC CTG GAT GGG CAG AGC ATC AAC CCC AAG TTG 883
Gln Leu Ser Val Glu Thr Leu Asp Gly Gln Ser Ile Asn Pro Lys Leu
245                 250                 255                 260

GCA GGC CTG ATT GGA CGG CAT GGA CCC CAG AAC AAG CAA CCC TTC ATG 931
Ala Gly Leu Ile Gly Arg His Gly Pro Gln Asn Lys Gln Pro Phe Met
                265                 270                 275

GTG GCC TTC TTC AAG GCC ACG GAA GTC CAT CTC CGT AGT ATC CGG TCC 979
Val Ala Phe Phe Lys Ala Thr Glu Val His Leu Arg Ser Ile Arg Ser
            280                 285                 290

ACG GGG GGC AAG CAG CGC AGC CAG AAT CGC TCC AAG ACG CCA AAG AAC 1027
Thr Gly Gly Lys Gln Arg Ser Gln Asn Arg Ser Lys Thr Pro Lys Asn
        295                 300                 305

CAA GAG GCC CTG AGG ATG GCC AGT GTG GCA GAA AAC AGC AGC AGT GAC 1075
Gln Glu Ala Leu Arg Met Ala Ser Val Ala Glu Asn Ser Ser Ser Asp
    310                 315                 320

CAG AGG CAG GCC TGC AAG AAA CAT GAG CTG TAC GTC AGC TTC CGA GAC 1123
Gln Arg Gln Ala Cys Lys Lys His Glu Leu Tyr Val Ser Phe Arg Asp
325                 330                 335                 340

CTT GGC TGG CAG GAC TGG ATC ATT GCA CCT GAA GGC TAT GCT GCC TAC 1171
Leu Gly Trp Gln Asp Trp Ile Ile Ala Pro Glu Gly Tyr Ala Ala Tyr
                345                 350                 355

TAC TGT GAG GGA GAG TGC GCC TTC CCT CTG AAC TCC TAC ATG AAC GCC 1219
Tyr Cys Glu Gly Glu Cys Ala Phe Pro Leu Asn Ser Tyr Met Asn Ala
            360                 365                 370

ACC AAC CAC GCC ATC GTC CAG ACA CTG GTT CAC TTC ATC AAC CCA GAC 1267
Thr Asn His Ala Ile Val Gln Thr Leu Val His Phe Ile Asn Pro Asp
        375                 380                 385

ACA GTA CCC AAG CCC TGC TGT GCG CCC ACC CAG CTC AAC GCC ATC TCT 1315
Thr Val Pro Lys Pro Cys Cys Ala Pro Thr Gln Leu Asn Ala Ile Ser
    390                 395                 400

GTC CTC TAC TTC GAC GAC AGC TCT AAT GTC GAC CTG AAG AAG TAC AGA 1363
Val Leu Tyr Phe Asp Asp Ser Ser Asn Val Asp Leu Lys Lys Tyr Arg
405                 410                 415                 420

AAC ATG GTG GTC CGG GCC TGT GGC TGC CAC TAGCTCTTCC TGAGACCCTG 1413
Asn Met Val Val Arg Ala Cys Gly Cys His
                425                 430

ACCTTTGCGG GGCCACACCT TTCCAAATCT TCGATGTCTC ACCATCTAAG TCTCTCACTG 1473

CCCACCTTGG CGAGGAGAAC AGACCAACCT CTCCTGAGCC TTCCCTCACC TCCCAACCGG 1533

AAGCATGTAA GGGTTCCAGA AACCTGAGCG TGCAGCAGCT GATGAGCGCC CTTTCCTTCT 1593

GGCACGTGAC GGACAAGATC CTACCAGCTA CCACAGCAAA CGCCTAAGAG CAGGAAAAAT 1653

GTCTGCCAGG AAAGTGTCCA GTGTCCACAT GGCCCCTGGC GCTCTGAGTC TTTGAGGAGT 1713

AATCGCAAGC CTCGTTCAGC TGCAGCAGAA GGAAGGGCTT AGCCAGGGTG GGCGCTGGCG 1773

TCTGTGTTGA AGGGAAACCA AGCAGAAGCC ACTGTAATGA TATGTCACAA TAAAACCCAT 1833

GAATGAAAAA AAAAAAAAAA AAAAAAAAAA AAAAGAATTC 1873

(2) INFORMATION FOR SEQ ID NO:19:

(i) SEQUENCE CHARACTERISTICS:
(A) LENGTH: 430 amino acids
(B) TYPE: amino acid
(D) TOPOLOGY: linear

(ii) MOLECULE TYPE: protein

(xi) SEQUENCE DESCRIPTION: SEQ ID NO:19:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Ser Gly
                85                  90                  95

Pro Asp Gly Gln Gly Phe Ser Tyr Pro Tyr Lys Ala Val Phe Ser Thr
            100                 105                 110

Gln Gly Pro Pro Leu Ala Ser Leu Gln Asp Ser His Phe Leu Thr Asp
        115                 120                 125

Ala Asp Met Val Met Ser Phe Val Asn Leu Val Glu His Asp Lys Glu
    130                 135                 140

Phe Phe His Pro Arg Tyr His His Arg Glu Phe Arg Phe Asp Leu Ser
145                 150                 155                 160

Lys Ile Pro Glu Gly Glu Arg Val Thr Ala Ala Glu Phe Arg Ile Tyr
                165                 170                 175

Lys Asp Tyr Ile Arg Glu Arg Phe Asp Asn Glu Thr Phe Gln Ile Thr
            180                 185                 190

Val Tyr Gln Val Leu Gln Glu His Ser Gly Arg Glu Ser Asp Leu Phe
        195                 200                 205

Leu Leu Asp Ser Arg Thr Ile Trp Ala Ser Glu Glu Gly Trp Leu Val
    210                 215                 220

Phe Asp Ile Thr Ala Thr Ser Asn His Trp Val Val Asn Pro Arg His
225                 230                 235                 240

Asn Leu Gly Leu Gln Leu Ser Val Glu Thr Leu Asp Gly Gln Ser Ile
                245                 250                 255

Asn Pro Lys Leu Ala Gly Leu Ile Gly Arg His Gly Pro Gln Asn Lys
            260                 265                 270

Gln Pro Phe Met Val Ala Phe Phe Lys Ala Thr Glu Val His Leu Arg
        275                 280                 285

Ser Ile Arg Ser Thr Gly Gly Lys Gln Arg Ser Gln Asn Arg Ser Lys
    290                 295                 300

Thr Pro Lys Asn Gln Glu Ala Leu Arg Met Ala Ser Val Ala Glu Asn
305                 310                 315                 320

Ser Ser Ser Asp Gln Arg Gln Ala Cys Lys Lys His Glu Leu Tyr Val
                325                 330                 335

Ser Phe Arg Asp Leu Gly Trp Gln Asp Trp Ile Ile Ala Pro Glu Gly
            340                 345                 350

Tyr Ala Ala Tyr Tyr Cys Glu Gly Glu Cys Ala Phe Pro Leu Asn Ser
        355                 360                 365

Tyr Met Asn Ala Thr Asn His Ala Ile Val Gln Thr Leu Val His Phe
    370                 375                 380

Ile Asn Pro Asp Thr Val Pro Lys Pro Cys Cys Ala Pro Thr Gln Leu
385                 390                 395                 400

Asn Ala Ile Ser Val Leu Tyr Phe Asp Asp Ser Ser Asn Val Asp Leu
                405                 410                 415

Lys Lys Tyr Arg Asn Met Val Val Arg Ala Cys Gly Cys His
            420                 425                 430

(2) INFORMATION FOR SEQ ID NO:20:

     (i) SEQUENCE CHARACTERISTICS:
          (A) LENGTH: 1723 base pairs
          (B) TYPE: nucleic acid
          (C) STRANDEDNESS: single
          (D) TOPOLOGY: linear

    (ii) MOLECULE TYPE: cDNA

(vi) ORIGINAL SOURCE:
(A) ORGANISM: Homo sapiens
(F) TISSUE TYPE: HIPPOCAMPUS

(ix) FEATURE:
(A) NAME/KEY: CDS
(B) LOCATION: 490..1696
(D) OTHER INFORMATION: /function= "OSTEOGENIC PROTEIN"
/product= "hOP2-PP"
/note= "hOP2 (cDNA)"

(xi) SEQUENCE DESCRIPTION: SEQ ID NO:20:

GGCGCCGGCA GAGCAGGAGT GGCTGGAGGA GCTGTGGTTG GAGCAGGAGG TGGCACGGCA 60

GGGCTGGAGG GCTCCCTATG AGTGGCGGAG ACGGCCCAGG AGGCGCTGGA GCAACAGCTC 120

CCACACCGCA CCAAGCGGTG GCTGCAGGAG CTCGCCCATC GCCCCTGCGC TGCTCGGACC 180

GCGGCCACAG CCGGACTGGC GGGTACGGCG GCGACAGAGG CATTGGCCGA GAGTCCCAGT 240

CCGCAGAGTA GCCCCGGCCT CGAGGCGGTG GCGTCCCGGT CCTCTCCGTC CAGGAGCCAG 300

GACAGGTGTC GCGCGGCGGG GCTCCAGGGA CCGCGCCTGA GGCCGGCTGC CCGCCCGTCC 360

CGCCCCGCCC CGCCGCCCGC CGCCCGCCGA GCCCAGCCTC CTTGCCGTCG GGGCGTCCCC 420

AGGCCCTGGG TCGGCCGCGG AGCCGATGCG CGCCCGCTGA GCGCCCCAGC TGAGCGCCCC 480

CGGCCTGCC ATG ACC GCG CTC CCC GGC CCG CTC TGG CTC CTG GGC CTG 528
Met Thr Ala Leu Pro Gly Pro Leu Trp Leu Leu Gly Leu
1               5                   10

GCG CTA TGC GCG CTG GGC GGG GGC GGC CCC GGC CTG CGA CCC CCG CCC 576
Ala Leu Cys Ala Leu Gly Gly Gly Gly Pro Gly Leu Arg Pro Pro Pro
    15                  20                  25

GGC TGT CCC CAG CGA CGT CTG GGC GCG CGC GAG CGC CGG GAC GTG CAG 624
Gly Cys Pro Gln Arg Arg Leu Gly Ala Arg Glu Arg Arg Asp Val Gln
30                  35                  40                  45

CGC GAG ATC CTG GCG GTG CTC GGG CTG CCT GGG CGG CCC CGG CCC CGC 672
Arg Glu Ile Leu Ala Val Leu Gly Leu Pro Gly Arg Pro Arg Pro Arg
                50                  55                  60

GCG CCA CCC GCC GCC TCC CGG CTG CCC GCG TCC GCG CCG CTC TTC ATG 720
Ala Pro Pro Ala Ala Ser Arg Leu Pro Ala Ser Ala Pro Leu Phe Met
            65                  70                  75

CTG GAC CTG TAC CAC GCC ATG GCC GGC GAC GAC GAC GAG GAC GGC GCG 768
Leu Asp Leu Tyr His Ala Met Ala Gly Asp Asp Asp Glu Asp Gly Ala
        80                  85                  90

CCC GCG GAG CGG CGC CTG GGC CGC GCC GAC CTG GTC ATG AGC TTC GTT 816
Pro Ala Glu Arg Arg Leu Gly Arg Ala Asp Leu Val Met Ser Phe Val
    95                  100                 105

AAC ATG GTG GAG CGA GAC CGT GCC CTG GGC CAC CAG GAG CCC CAT TGG 864
Asn Met Val Glu Arg Asp Arg Ala Leu Gly His Gln Glu Pro His Trp
110                 115                 120                 125

AAG GAG TTC CGC TTT GAC CTG ACC CAG ATC CCG GCT GGG GAG GCG GTC 912
Lys Glu Phe Arg Phe Asp Leu Thr Gln Ile Pro Ala Gly Glu Ala Val
                130                 135                 140

ACA GCT GCG GAG TTC CGG ATT TAC AAG GTG CCC AGC ATC CAC CTG CTC 960
Thr Ala Ala Glu Phe Arg Ile Tyr Lys Val Pro Ser Ile His Leu Leu
            145                 150                 155

AAC AGG ACC CTC CAC GTC AGC ATG TTC CAG GTG GTC CAG GAG CAG TCC 1008
Asn Arg Thr Leu His Val Ser Met Phe Gln Val Val Gln Glu Gln Ser
        160                 165                 170

AAC AGG GAG TCT GAC TTG TTC TTT TTG GAT CTT CAG ACG CTC CGA GCT 1056
Asn Arg Glu Ser Asp Leu Phe Phe Leu Asp Leu Gln Thr Leu Arg Ala
    175                 180                 185

GGA GAC GAG GGC TGG CTG GTG CTG GAT GTC ACA GCA GCC AGT GAC TGC 1104
Gly Asp Glu Gly Trp Leu Val Leu Asp Val Thr Ala Ala Ser Asp Cys
190                 195                 200                 205

TGG TTG CTG AAG CGT CAC AAG GAC CTG GGA CTC CGC CTC TAT GTG GAG 1152
Trp Leu Leu Lys Arg His Lys Asp Leu Gly Leu Arg Leu Tyr Val Glu
                210                 215                 220

ACT GAG GAC GGG CAC AGC GTG GAT CCT GGC CTG GCC GGC CTG CTG GGT 1200
Thr Glu Asp Gly His Ser Val Asp Pro Gly Leu Ala Gly Leu Leu Gly
            225                 230                 235

CAA CGG GCC CCA CGC TCC CAA CAG CCT TTC GTG GTC ACT TTC TTC AGG 1248
Gln Arg Ala Pro Arg Ser Gln Gln Pro Phe Val Val Thr Phe Phe Arg
        240                 245                 250

GCC AGT CCG AGT CCC ATC CGC ACC CCT CGG GCA GTG AGG CCA CTG AGG 1296
Ala Ser Pro Ser Pro Ile Arg Thr Pro Arg Ala Val Arg Pro Leu Arg
    255                 260                 265

AGG AGG CAG CCG AAG AAA AGC AAC GAG CTG CCG CAG GCC AAC CGA CTC 1344
Arg Arg Gln Pro Lys Lys Ser Asn Glu Leu Pro Gln Ala Asn Arg Leu
270                 275                 280                 285

CCA GGG ATC TTT GAT GAC GTC CAC GGC TCC CAC GGC CGG CAG GTC TGC 1392
Pro Gly Ile Phe Asp Asp Val His Gly Ser His Gly Arg Gln Val Cys
                290                 295                 300

CGT CGG CAC GAG CTC TAC GTC AGC TTC CAG GAC CTC GGC TGG CTG GAC 1440
Arg Arg His Glu Leu Tyr Val Ser Phe Gln Asp Leu Gly Trp Leu Asp
            305                 310                 315

TGG GTC ATC GCT CCC CAA GGC TAC TCG GCC TAT TAC TGT GAG GGG GAG 1488
Trp Val Ile Ala Pro Gln Gly Tyr Ser Ala Tyr Tyr Cys Glu Gly Glu
        320                 325                 330

TGC TCC TTC CCA CTG GAC TCC TGC ATG AAT GCC ACC AAC CAC GCC ATC 1536
Cys Ser Phe Pro Leu Asp Ser Cys Met Asn Ala Thr Asn His Ala Ile
    335                 340                 345

CTG CAG TCC CTG GTG CAC CTG ATG AAG CCA AAC GCA GTC CCC AAG GCG 1584
Leu Gln Ser Leu Val His Leu Met Lys Pro Asn Ala Val Pro Lys Ala
350                 355                 360                 365

TGC TGT GCA CCC ACC AAG CTG AGC GCC ACC TCT GTG CTC TAC TAT GAC 1632
Cys Cys Ala Pro Thr Lys Leu Ser Ala Thr Ser Val Leu Tyr Tyr Asp
                370                 375                 380

AGC AGC AAC AAC GTC ATC CTG CGC AAA GCC CGC AAC ATG GTG GTC AAG 1680
Ser Ser Asn Asn Val Ile Leu Arg Lys Ala Arg Asn Met Val Val Lys
            385                 390                 395

GCC TGC GGC TGC CAC T GAGTCAGCCC GCCCAGCCCT ACTGCAG 1723
Ala Cys Gly Cys His
        400

(2) INFORMATION FOR SEQ ID NO:21:

(i) SEQUENCE CHARACTERISTICS:
(A) LENGTH: 402 amino acids
(B) TYPE: amino acid
(D) TOPOLOGY: linear

(ii) MOLECULE TYPE: protein

(xi) SEQUENCE DESCRIPTION: SEQ ID NO:21:

Met Thr Ala Leu Pro Gly Pro Leu Trp Leu Leu Gly Leu Ala Leu Cys
1               5                   10                  15

Ala Leu Gly Gly Gly Gly Pro Gly Leu Arg Pro Pro Pro Gly Cys Pro
            20                  25                  30

Gln Arg Arg Leu Gly Ala Arg Glu Arg Arg Asp Val Gln Arg Glu Ile
        35                  40                  45

Leu Ala Val Leu Gly Leu Pro Gly Arg Pro Arg Pro Arg Ala Pro Pro
    50                  55                  60

Ala Ala Ser Arg Leu Pro Ala Ser Ala Pro Leu Phe Met Leu Asp Leu
65                  70                  75                  80

Tyr His Ala Met Ala Gly Asp Asp Asp Glu Asp Gly Ala Pro Ala Glu
                85                  90                  95

Arg Arg Leu Gly Arg Ala Asp Leu Val Met Ser Phe Val Asn Met Val
            100                 105                 110

Glu Arg Asp Arg Ala Leu Gly His Gln Glu Pro His Trp Lys Glu Phe
        115                 120                 125

Arg Phe Asp Leu Thr Gln Ile Pro Ala Gly Glu Ala Val Thr Ala Ala
    130                 135                 140

Glu Phe Arg Ile Tyr Lys Val Pro Ser Ile His Leu Leu Asn Arg Thr
145                 150                 155                 160

Leu His Val Ser Met Phe Gln Val Val Gln Glu Gln Ser Asn Arg Glu
                165                 170                 175

Ser Asp Leu Phe Phe Leu Asp Leu Gln Thr Leu Arg Ala Gly Asp Glu
            180                 185                 190

Gly Trp Leu Val Leu Asp Val Thr Ala Ala Ser Asp Cys Trp Leu Leu
        195                 200                 205

Lys Arg His Lys Asp Leu Gly Leu Arg Leu Tyr Val Glu Thr Glu Asp
    210                 215                 220

Gly His Ser Val Asp Pro Gly Leu Ala Gly Leu Leu Gly Gln Arg Ala
225                 230                 235                 240

Pro Arg Ser Gln Gln Pro Phe Val Val Thr Phe Phe Arg Ala Ser Pro
                245                 250                 255

Ser Pro Ile Arg Thr Pro Arg Ala Val Arg Pro Leu Arg Arg Arg Gln
            260                 265                 270

Pro Lys Lys Ser Asn Glu Leu Pro Gln Ala Asn Arg Leu Pro Gly Ile
        275                 280                 285

Phe Asp Asp Val His Gly Ser His Gly Arg Gln Val Cys Arg Arg His
    290                 295                 300

Glu Leu Tyr Val Ser Phe Gln Asp Leu Gly Trp Leu Asp Trp Val Ile
305                 310                 315                 320

Ala Pro Gln Gly Tyr Ser Ala Tyr Tyr Cys Glu Gly Glu Cys Ser Phe
                325                 330                 335

Pro Leu Asp Ser Cys Met Asn Ala Thr Asn His Ala Ile Leu Gln Ser
            340                 345                 350

Leu Val His Leu Met Lys Pro Asn Ala Val Pro Lys Ala Cys Cys Ala
        355                 360                 365

Pro Thr Lys Leu Ser Ala Thr Ser Val Leu Tyr Tyr Asp Ser Ser Asn
    370                 375                 380

Asn Val Ile Leu Arg Lys Ala Arg Asn Met Val Val Lys Ala Cys Gly
385                 390                 395                 400

Cys His

(2) INFORMATION FOR SEQ ID NO:22:

     (i) SEQUENCE CHARACTERISTICS:
          (A) LENGTH: 1926 base pairs
          (B) TYPE: nucleic acid
          (C) STRANDEDNESS: single
          (D) TOPOLOGY: linear

    (vi) ORIGINAL SOURCE:
          (A) ORGANISM: MURIDAE
          (F) TISSUE TYPE: EMBRYO

    (ix) FEATURE:
          (A) NAME/KEY: CDS
          (B) LOCATION: 93..1289
          (D) OTHER INFORMATION: /function= "OSTEOGENIC PROTEIN"
                /product= "mOP2-PP"
                /note= "mOP2 cDNA"

    (xi) SEQUENCE DESCRIPTION: SEQ ID NO:22:

GCCAGGCACA GGTGCGCCGT CTGGTCCTCC CCGTCTGGCG TCAGCCGAGC CCGACCAGCT      60

ACCAGTGGAT GCGCGCCGGC TGAAAGTCCG AG ATG GCT ATG CGT CCC GGG CCA      113
                                    Met Ala Met Arg Pro Gly Pro
                                      1               5

CTC TGG CTA TTG GGC CTT GCT CTG TGC GCG CTG GGA GGC GGC CAC GGT      161
Leu Trp Leu Leu Gly Leu Ala Leu Cys Ala Leu Gly Gly Gly His Gly
        10                  15                  20

CCG CGT CCC CCG CAC ACC TGT CCC CAG CGT CGC CTG GGA GCG CGC GAG      209
Pro Arg Pro Pro His Thr Cys Pro Gln Arg Arg Leu Gly Ala Arg Glu
    25                  30                  35

CGC CGC GAC ATG CAG CGT GAA ATC CTG GCG GTG CTC GGG CTA CCG GGA      257
Arg Arg Asp Met Gln Arg Glu Ile Leu Ala Val Leu Gly Leu Pro Gly
 40                  45                  50                  55

CGG CCC CGA CCC CGT GCA CAA CCC GCC GCT GCC CGG CAG CCA GCG TCC 305
Arg Pro Arg Pro Arg Ala Gln Pro Ala Ala Ala Arg Gln Pro Ala Ser
                60                  65                  70

GCG CCC CTC TTC ATG TTG GAC CTA TAC CAC GCC ATG ACC GAT GAC GAC 353
Ala Pro Leu Phe Met Leu Asp Leu Tyr His Ala Met Thr Asp Asp Asp
            75                  80                  85

GAC GGC GGG CCA CCA CAG GCT CAC TTA GGC CGT GCC GAC CTG GTC ATG 401
Asp Gly Gly Pro Pro Gln Ala His Leu Gly Arg Ala Asp Leu Val Met
        90                  95                  100

AGC TTC GTC AAC ATG GTG GAA CGC GAC CGT ACC CTG GGC TAC CAG GAG 449
Ser Phe Val Asn Met Val Glu Arg Asp Arg Thr Leu Gly Tyr Gln Glu
    105                 110                 115

CCA CAC TGG AAG GAA TTC CAC TTT GAC CTA ACC CAG ATC CCT GCT GGG 497
Pro His Trp Lys Glu Phe His Phe Asp Leu Thr Gln Ile Pro Ala Gly
120                 125                 130                 135

GAG GCT GTC ACA GCT GCT GAG TTC CGG ATC TAC AAA GAA CCC AGC ACC 545
Glu Ala Val Thr Ala Ala Glu Phe Arg Ile Tyr Lys Glu Pro Ser Thr
                140                 145                 150

CAC CCG CTC AAC ACA ACC CTC CAC ATC AGC ATG TTC GAA GTG GTC CAA 593
His Pro Leu Asn Thr Thr Leu His Ile Ser Met Phe Glu Val Val Gln
            155                 160                 165

GAG CAC TCC AAC AGG GAG TCT GAC TTG TTC TTT TTG GAT CTT CAG ACG 641
Glu His Ser Asn Arg Glu Ser Asp Leu Phe Phe Leu Asp Leu Gln Thr
        170                 175                 180

CTC CGA TCT GGG GAC GAG GGC TGG CTG GTG CTG GAC ATC ACA GCA GCC 689
Leu Arg Ser Gly Asp Glu Gly Trp Leu Val Leu Asp Ile Thr Ala Ala
    185                 190                 195

AGT GAC CGA TGG CTG CTG AAC CAT CAC AAG GAC CTG GGA CTC CGC CTC 737
Ser Asp Arg Trp Leu Leu Asn His His Lys Asp Leu Gly Leu Arg Leu
200                 205                 210                 215

TAT GTG GAA ACC GCG GAT GGG CAC AGC ATG GAT CCT GGC CTG GCT GGT 785
Tyr Val Glu Thr Ala Asp Gly His Ser Met Asp Pro Gly Leu Ala Gly
                220                 225                 230

CTG CTT GGA CGA CAA GCA CCA CGC TCC AGA CAG CCT TTC ATG GTA ACC 833
Leu Leu Gly Arg Gln Ala Pro Arg Ser Arg Gln Pro Phe Met Val Thr
            235                 240                 245

TTC TTC AGG GCC AGC CAG AGT CCT GTG CGG GCC CCT CGG GCA GCG AGA 881
Phe Phe Arg Ala Ser Gln Ser Pro Val Arg Ala Pro Arg Ala Ala Arg
        250                 255                 260

CCA CTG AAG AGG AGG CAG CCA AAG AAA ACG AAC GAG CTT CCG CAC CCC 929
Pro Leu Lys Arg Arg Gln Pro Lys Lys Thr Asn Glu Leu Pro His Pro
    265                 270                 275

AAC AAA CTC CCA GGG ATC TTT GAT GAT GGC CAC GGT TCC CGC GGC AGA 977
Asn Lys Leu Pro Gly Ile Phe Asp Asp Gly His Gly Ser Arg Gly Arg
280                 285                 290                 295

GAG GTT TGC CGC AGG CAT GAG CTC TAC GTC AGC TTC CGT GAC CTT GGC 1025
Glu Val Cys Arg Arg His Glu Leu Tyr Val Ser Phe Arg Asp Leu Gly
                300                 305                 310

TGG CTG GAC TGG GTC ATC GCC CCC CAG GGC TAC TCT GCC TAT TAC TGT 1073
Trp Leu Asp Trp Val Ile Ala Pro Gln Gly Tyr Ser Ala Tyr Tyr Cys
            315                 320                 325

GAG GGG GAG TGT GCT TTC CCA CTG GAC TCC TGT ATG AAC GCC ACC AAC 1121
Glu Gly Glu Cys Ala Phe Pro Leu Asp Ser Cys Met Asn Ala Thr Asn
        330                 335                 340

CAT GCC ATC TTG CAG TCT CTG GTG CAC CTG ATG AAG CCA GAT GTT GTC 1169
His Ala Ile Leu Gln Ser Leu Val His Leu Met Lys Pro Asp Val Val
    345                 350                 355

CCC AAG GCA TGC TGT GCA CCC ACC AAA CTG AGT GCC ACC TCT GTG CTG 1217
Pro Lys Ala Cys Cys Ala Pro Thr Lys Leu Ser Ala Thr Ser Val Leu
360                 365                 370                 375

TAC TAT GAC AGC AGC AAC AAT GTC ATC CTG CGT AAA CAC CGT AAC ATG 1265
Tyr Tyr Asp Ser Ser Asn Asn Val Ile Leu Arg Lys His Arg Asn Met
                380                 385                 390

GTG GTC AAG GCC TGT GGC TGC CAC TGAGGCCCCG CCCAGCATCC TGCTTCTACT 1319
Val Val Lys Ala Cys Gly Cys His
            395

ACCTTACCAT CTGGCCGGGC CCCTCTCCAG AGGCAGAAAC CCTTCTATGT TATCATAGCT 1379

CAGACAGGGG CAATGGGAGG CCCTTCACTT CCCCTGGCCA CTTCCTGCTA AAATTCTGGT 1439

CTTTCCCAGT TCCTCTGTCC TTCATGGGGT TTCGGGGCTA TCACCCCGCC CTCTCCATCC 1499

TCCTACCCCA AGCATAGACT GAATGCACAC AGCATCCCAG AGCTATGCTA ACTGAGAGGT 1559

CTGGGGTCAG CACTGAAGGC CCACATGAGG AAGACTGATC CTTGGCCATC CTCAGCCCAC 1619

AATGGCAAAT TCTGGATGGT CTAAGAAGGC CCTGGAATTC TAAACTAGAT GATCTGGGCT 1679

CTCTGCACCA TTCATTGTGG CAGTTGGGAC ATTTTTAGGT ATAACAGACA CATACACTTA 1739

GATCAATGCA TCGCTGTACT CCTTGAAATC AGAGCTAGCT TGTTAGAAAA AGAATCAGAG 1799

CCAGGTATAG CGGTGCATGT CATTAATCCC AGCGCTAAAG AGACAGAGAC AGGAGAATCT 1859

CTGTGAGTTC AAGGCCACAT AGAAAGAGCC TGTCTCGGGA GCAGGAAAAA AAAAAAAAAC 1919

GGAATTC 1926

(2) INFORMATION FOR SEQ ID NO:23:

(i) SEQUENCE CHARACTERISTICS:
(A) LENGTH: 399 amino acids
(B) TYPE: amino acid
(D) TOPOLOGY: linear

(ii) MOLECULE TYPE: protein

(xi) SEQUENCE DESCRIPTION: SEQ ID NO:23:

Met Ala Met Arg Pro Gly Pro Leu Trp Leu Leu Gly Leu Ala Leu Cys
1               5                   10                  15

Ala Leu Gly Gly Gly His Gly Pro Arg Pro Pro His Thr Cys Pro Gln
            20                  25                  30

Arg Arg Leu Gly Ala Arg Glu Arg Arg Asp Met Gln Arg Glu Ile Leu
        35                  40                  45

Ala Val Leu Gly Leu Pro Gly Arg Pro Arg Pro Arg Ala Gln Pro Ala
    50                  55                  60

Ala Ala Arg Gln Pro Ala Ser Ala Pro Leu Phe Met Leu Asp Leu Tyr
65                  70                  75                  80

His Ala Met Thr Asp Asp Asp Asp Gly Gly Pro Pro Gln Ala His Leu
                85                  90                  95

Gly Arg Ala Asp Leu Val Met Ser Phe Val Asn Met Val Glu Arg Asp
            100                 105                 110

Arg Thr Leu Gly Tyr Gln Glu Pro His Trp Lys Glu Phe His Phe Asp
        115                 120                 125

Leu Thr Gln Ile Pro Ala Gly Glu Ala Val Thr Ala Ala Glu Phe Arg
    130                 135                 140

Ile Tyr Lys Glu Pro Ser Thr His Pro Leu Asn Thr Thr Leu His Ile
145                 150                 155                 160

Ser Met Phe Glu Val Val Gln Glu His Ser Asn Arg Glu Ser Asp Leu
                165                 170                 175

Phe Phe Leu Asp Leu Gln Thr Leu Arg Ser Gly Asp Glu Gly Trp Leu
            180                 185                 190

Val Leu Asp Ile Thr Ala Ala Ser Asp Arg Trp Leu Leu Asn His His
        195                 200                 205

Lys Asp Leu Gly Leu Arg Leu Tyr Val Glu Thr Ala Asp Gly His Ser
    210                 215                 220

Met Asp Pro Gly Leu Ala Gly Leu Leu Gly Arg Gln Ala Pro Arg Ser
225                 230                 235                 240

Arg Gln Pro Phe Met Val Thr Phe Phe Arg Ala Ser Gln Ser Pro Val
                245                 250                 255

Arg Ala Pro Arg Ala Ala Arg Pro Leu Lys Arg Arg Gln Pro Lys Lys
            260 ·               265                 270

Thr Asn Glu Leu Pro His Pro Asn Lys Leu Pro Gly Ile Phe Asp Asp
        275                 280                 285

Gly His Gly Ser Arg Gly Arg Glu Val Cys Arg Arg His Glu Leu Tyr
    290                 295                 300

Val Ser Phe Arg Asp Leu Gly Trp Leu Asp Trp Val Ile Ala Pro Gln
305                 310                 315                 320

Gly Tyr Ser Ala Tyr Tyr Cys Glu Gly Glu Cys Ala Phe Pro Leu Asp
                325                 330                 335

Ser Cys Met Asn Ala Thr Asn His Ala Ile Leu Gln Ser Leu Val His
            340                 345                 350

Leu Met Lys Pro Asp Val Val Pro Lys Ala Cys Cys Ala Pro Thr Lys
        355                 360                 365

Leu Ser Ala Thr Ser Val Leu Tyr Tyr Asp Ser Ser Asn Asn Val Ile
    370                 375                 380

Leu Arg Lys His Arg Asn Met Val Val Lys Ala Cys Gly Cys His
385                 390                 395

(2) INFORMATION FOR SEQ ID NO:24:

     (i) SEQUENCE CHARACTERISTICS:
          (A) LENGTH: 1368 base pairs
          (B) TYPE: nucleic acid
          (C) STRANDEDNESS: single
          (D) TOPOLOGY: linear

    (ii) MOLECULE TYPE: cDNA

    (ix) FEATURE:
          (A) NAME/KEY: CDS
          (B) LOCATION: 1..1368

(xi) SEQUENCE DESCRIPTION: SEQ ID NO:24:

ATG TCG GGA CTG CGA AAC ACC TCG GAG GCC GTT GCA GTG CTC GCC TCC 48
Met Ser Gly Leu Arg Asn Thr Ser Glu Ala Val Ala Val Leu Ala Ser
1               5                   10                  15

CTG GGA CTC GGA ATG GTT CTG CTC ATG TTC GTG GCG ACC ACG CCG CCG 96
Leu Gly Leu Gly Met Val Leu Leu Met Phe Val Ala Thr Thr Pro Pro
            20                  25                  30

GCC GTT GAG GCC ACC CAG TCG GGG ATT TAC ATA GAC AAC GGC AAG GAC 144
Ala Val Glu Ala Thr Gln Ser Gly Ile Tyr Ile Asp Asn Gly Lys Asp
        35                  40                  45

CAG ACG ATC ATG CAC AGA GTG CTG AGC GAG GAC GAC AAG CTG GAC GTC 192
Gln Thr Ile Met His Arg Val Leu Ser Glu Asp Asp Lys Leu Asp Val
    50                  55                  60

TCG TAC GAG ATC CTC GAG TTC CTG GGC ATC GCC GAA CGG CCG ACG CAC 240
Ser Tyr Glu Ile Leu Glu Phe Leu Gly Ile Ala Glu Arg Pro Thr His
65                  70                  75                  80

CTG AGC AGC CAC CAG TTG TCG CTG AGG AAG TCG GCT CCC AAG TTC CTG 288
Leu Ser Ser His Gln Leu Ser Leu Arg Lys Ser Ala Pro Lys Phe Leu
                85                  90                  95

CTG GAC GTC TAC CAC CGC ATC ACG GCG GAG GAG GGT CTC AGC GAT CAG 336
Leu Asp Val Tyr His Arg Ile Thr Ala Glu Glu Gly Leu Ser Asp Gln
            100                 105                 110

GAT GAG GAC GAC GAC TAC GAA CGC GGC CAT CGG TCC AGG AGG AGC GCC 384
Asp Glu Asp Asp Asp Tyr Glu Arg Gly His Arg Ser Arg Arg Ser Ala
        115                 120                 125

GAC CTC GAG GAG GAT GAG GGC GAG CAG CAG AAG AAC TTC ATC ACC GAC 432
Asp Leu Glu Glu Asp Glu Gly Glu Gln Gln Lys Asn Phe Ile Thr Asp
    130                 135                 140

CTG GAC AAG CGG GCC ATC GAC GAG AGC GAC ATC ATC ATG ACC TTC CTG 480
Leu Asp Lys Arg Ala Ile Asp Glu Ser Asp Ile Ile Met Thr Phe Leu
145                 150                 155                 160

AAC AAG CGC CAC CAC AAT GTG GAC GAA CTG CGT CAC GAG CAC GGC CGT 528
Asn Lys Arg His His Asn Val Asp Glu Leu Arg His Glu His Gly Arg
                165                 170                 175

CGC CTG TGG TTC GAC GTC TCC AAC GTG CCC AAC GAC AAC TAC CTG GTG 576
Arg Leu Trp Phe Asp Val Ser Asn Val Pro Asn Asp Asn Tyr Leu Val
            180                 185                 190

ATG GCC GAG CTG CGC ATC TAT CAG AAC GCC AAC GAG GGC AAG TGG CTG 624
Met Ala Glu Leu Arg Ile Tyr Gln Asn Ala Asn Glu Gly Lys Trp Leu
        195                 200                 205

ACC GCC AAC AGG GAG TTC ACC ATC ACG GTA TAC GCC ATT GGC ACC GGC 672
Thr Ala Asn Arg Glu Phe Thr Ile Thr Val Tyr Ala Ile Gly Thr Gly
    210                 215                 220

ACG CTG GGC CAG CAC ACC ATG GAG CCG CTG TCC TCG GTG AAC ACC ACC 720
Thr Leu Gly Gln His Thr Met Glu Pro Leu Ser Ser Val Asn Thr Thr
225                 230                 235                 240

GGG GAC TAC GTG GGC TGG TTG GAG CTC AAC GTG ACC GAG GGC CTG CAC 768
Gly Asp Tyr Val Gly Trp Leu Glu Leu Asn Val Thr Glu Gly Leu His
                245                 250                 255

GAG TGG CTG GTC AAG TCG AAG GAC AAT CAT GGC ATC TAC ATT GGA GCA 816
Glu Trp Leu Val Lys Ser Lys Asp Asn His Gly Ile Tyr Ile Gly Ala
            260                 265                 270

CAC GCT GTC AAC CGA CCC GAC CGC GAG GTG AAG CTG GAC GAC ATT GGA 864
His Ala Val Asn Arg Pro Asp Arg Glu Val Lys Leu Asp Asp Ile Gly
        275                 280                 285

CTG ATC CAC CGC AAG GTG GAC GAC GAG TTC CAG CCC TTC ATG ATC GGC 912
Leu Ile His Arg Lys Val Asp Asp Glu Phe Gln Pro Phe Met Ile Gly
    290                 295                 300

TTC TTC CGC GGA CCG GAG CTG ATC AAG GCG ACG GCC CAC AGC AGC CAC 960
Phe Phe Arg Gly Pro Glu Leu Ile Lys Ala Thr Ala His Ser Ser His
305                 310                 315                 320

CAC AGG AGC AAG CGA AGC GCC AGC CAT CCA CGC AAG CGC AAG AAG TCG 1008
His Arg Ser Lys Arg Ser Ala Ser His Pro Arg Lys Arg Lys Lys Ser
                325                 330                 335

GTG TCG CCC AAC AAC GTG CCG CTG CTG GAA CCG ATG GAG AGC ACG CGC 1056
Val Ser Pro Asn Asn Val Pro Leu Leu Glu Pro Met Glu Ser Thr Arg
            340                 345                 350

AGC TGC CAG ATG CAG ACC CTG TAC ATA GAC TTC AAG GAT CTG GGC TGG 1104
Ser Cys Gln Met Gln Thr Leu Tyr Ile Asp Phe Lys Asp Leu Gly Trp
        355                 360                 365

CAT GAC TGG ATC ATC GCA CCA GAG GGC TAT GGC GCC TTC TAC TGC AGC 1152
His Asp Trp Ile Ile Ala Pro Glu Gly Tyr Gly Ala Phe Tyr Cys Ser
    370                 375                 380

GGC GAG TGC AAT TTC CCG CTC AAT GCG CAC ATG AAC GCC ACG AAC CAT 1200
Gly Glu Cys Asn Phe Pro Leu Asn Ala His Met Asn Ala Thr Asn His
385                 390                 395                 400

GCG ATC GTC CAG ACC CTG GTC CAC CTG CTG GAG CCC AAG AAG GTG CCC     1248
Ala Ile Val Gln Thr Leu Val His Leu Leu Glu Pro Lys Lys Val Pro
                405                 410                 415

AAG CCC TGC TGC GCT CCG ACC AGG CTG GGA GCA CTA CCC GTT CTG TAC     1296
Lys Pro Cys Cys Ala Pro Thr Arg Leu Gly Ala Leu Pro Val Leu Tyr
            420                 425                 430

CAC CTG AAC GAC GAG AAT GTG AAC CTG AAA AAG TAT AGA AAC ATG ATT     1344
His Leu Asn Asp Glu Asn Val Asn Leu Lys Lys Tyr Arg Asn Met Ile
        435                 440                 445

GTG AAA TCC TGC GGG TGC CAT TGA                                     1368
Val Lys Ser Cys Gly Cys His
    450                 455

(2) INFORMATION FOR SEQ ID NO:25:

(i) SEQUENCE CHARACTERISTICS:
(A) LENGTH: 455 amino acids
(B) TYPE: amino acid
(D) TOPOLOGY: linear

(ii) MOLECULE TYPE: protein

(xi) SEQUENCE DESCRIPTION: SEQ ID NO:25:

Met Ser Gly Leu Arg Asn Thr Ser Glu Ala Val Ala Val Leu Ala Ser
  1               5                  10                  15

Leu Gly Leu Gly Met Val Leu Leu Met Phe Val Ala Thr Thr Pro Pro
             20                  25                  30

Ala Val Glu Ala Thr Gln Ser Gly Ile Tyr Ile Asp Asn Gly Lys Asp
         35                  40                  45

Gln Thr Ile Met His Arg Val Leu Ser Glu Asp Asp Lys Leu Asp Val
     50                  55                  60

Ser Tyr Glu Ile Leu Glu Phe Leu Gly Ile Ala Glu Arg Pro Thr His
 65                  70                  75                  80

Leu Ser Ser His Gln Leu Ser Leu Arg Lys Ser Ala Pro Lys Phe Leu
                 85                  90                  95

Leu Asp Val Tyr His Arg Ile Thr Ala Glu Glu Gly Leu Ser Asp Gln
            100                 105                 110

Asp Glu Asp Asp Asp Tyr Glu Arg Gly His Arg Ser Arg Arg Ser Ala
        115                 120                 125

Asp Leu Glu Glu Asp Glu Gly Glu Gln Gln Lys Asn Phe Ile Thr Asp
    130                 135                 140

Leu Asp Lys Arg Ala Ile Asp Glu Ser Asp Ile Ile Met Thr Phe Leu
145                 150                 155                 160

Asn Lys Arg His His Asn Val Asp Glu Leu Arg His Glu His Gly Arg
                165                 170                 175

Arg Leu Trp Phe Asp Val Ser Asn Val Pro Asn Asp Asn Tyr Leu Val
            180                 185                 190

Met Ala Glu Leu Arg Ile Tyr Gln Asn Ala Asn Glu Gly Lys Trp Leu
        195                 200                 205

Thr Ala Asn Arg Glu Phe Thr Ile Thr Val Tyr Ala Ile Gly Thr Gly
    210                 215                 220

Thr Leu Gly Gln His Thr Met Glu Pro Leu Ser Ser Val Asn Thr Thr
225                 230                 235                 240

Gly Asp Tyr Val Gly Trp Leu Glu Leu Asn Val Thr Glu Gly Leu His
                245                 250                 255

Glu Trp Leu Val Lys Ser Lys Asp Asn His Gly Ile Tyr Ile Gly Ala
            260                 265                 270

His Ala Val Asn Arg Pro Asp Arg Glu Val Lys Leu Asp Asp Ile Gly
        275                 280                 285

Leu Ile His Arg Lys Val Asp Asp Glu Phe Gln Pro Phe Met Ile Gly
    290                 295                 300

Phe Phe Arg Gly Pro Glu Leu Ile Lys Ala Thr Ala His Ser Ser His
305                 310                 315                 320

His Arg Ser Lys Arg Ser Ala Ser His Pro Arg Lys Arg Lys Lys Ser
                325                 330                 335

Val Ser Pro Asn Asn Val Pro Leu Leu Glu Pro Met Glu Ser Thr Arg
            340                 345                 350

Ser Cys Gln Met Gln Thr Leu Tyr Ile Asp Phe Lys Asp Leu Gly Trp
        355                 360                 365

His Asp Trp Ile Ile Ala Pro Glu Gly Tyr Gly Ala Phe Tyr Cys Ser
    370                 375                 380

Gly Glu Cys Asn Phe Pro Leu Asn Ala His Met Asn Ala Thr Asn His
385                 390                 395                 400

Ala Ile Val Gln Thr Leu Val His Leu Leu Glu Pro Lys Lys Val Pro
                405                 410                 415

Lys Pro Cys Cys Ala Pro Thr Arg Leu Gly Ala Leu Pro Val Leu Tyr
            420                     425                     430

His Leu Asn Asp Glu Asn Val Asn Leu Lys Lys Tyr Arg Asn Met Ile
        435                     440                     445

Val Lys Ser Cys Gly Cys His
    450                 455

(2) INFORMATION FOR SEQ ID NO:26:

     (i) SEQUENCE CHARACTERISTICS:
          (A) LENGTH: 104 amino acids
          (B) TYPE: amino acid
          (C) STRANDEDNESS: single
          (D) TOPOLOGY: linear

    (ii) MOLECULE TYPE: protein

    (ix) FEATURE:
          (A) NAME/KEY: Protein
          (B) LOCATION: 1..104
          (D) OTHER INFORMATION: /note= "BMP3"

    (xi) SEQUENCE DESCRIPTION: SEQ ID NO:26:

     Cys Ala Arg Arg Tyr Leu Lys Val Asp Phe Ala Asp Ile Gly Trp Ser
     1               5                   10                  15

     Glu Trp Ile Ile Ser Pro Lys Ser Phe Asp Ala Tyr Tyr Cys Ser Gly
                 20                  25                  30

     Ala Cys Gln Phe Pro Met Pro Lys Ser Leu Lys Pro Ser Asn His Ala
             35                  40                  45

     Thr Ile Gln Ser Ile Val Ala Arg Ala Val Gly Val Val Pro Gly Ile
         50                  55                  60

     Pro Glu Pro Cys Cys Val Pro Glu Lys Met Ser Ser Leu Ser Ile Leu
     65                  70                  75                  80

     Phe Phe Asp Glu Asn Lys Asn Val Val Leu Lys Val Tyr Pro Asn Met
                     85                  90                  95

     Thr Val Glu Ser Cys Ala Cys Arg
                 100

(2) INFORMATION FOR SEQ ID NO:27:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP5"

(xi) SEQUENCE DESCRIPTION: SEQ ID NO:27:

Cys Lys Lys His Glu Leu Tyr Val Ser Phe Arg Asp Leu Gly Trp Gln
1               5                   10                  15

Asp Trp Ile Ile Ala Pro Glu Gly Tyr Ala Ala Phe Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Phe Pro Asp His Val Pro Lys
    50                  55                  60

Pro Cys Cys Ala Pro Thr Lys Leu Asn Ala Ile Ser Val Leu Tyr Phe
65                  70                  75                  80

Asp Asp Ser Ser Asn Val Ile Leu Lys Lys Tyr Arg Asn Met Val Val
                85                  90                  95

Arg Ser Cys Gly Cys His
            100

(2) INFORMATION FOR SEQ ID NO:28:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP6"

(xi) SEQUENCE DESCRIPTION: SEQ ID NO:28:

Cys Arg Lys His Glu Leu Tyr Val Ser Phe Gln Asp Leu Gly Trp Gln
1               5                   10                  15

Asp Trp Ile Ile Ala Pro Lys Gly Tyr Ala Ala Asn Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Asn Pro Glu Tyr Val Pro Lys
    50                  55                  60

Pro Cys Cys Ala Pro Thr Lys Leu Asn Ala Ile Ser Val Leu Tyr Phe
65                  70                  75                  80

Asp Asp Asn Ser Asn Val Ile Leu Lys Lys Tyr Arg Trp Met Val Val
                85                  90                  95

Arg Ala Cys Gly Cys His
            100

(2) INFORMATION FOR SEQ ID NO:29:

(i) SEQUENCE CHARACTERISTICS:
(A) LENGTH: 102 amino acids
(B) TYPE: amino acid
(D) TOPOLOGY: linear

(ii) MOLECULE TYPE: protein

(ix) FEATURE:
(A) NAME/KEY: Protein
(B) LOCATION: 1..102
(D) OTHER INFORMATION: /label= OPX
/note= "WHEREIN EACH XAA IS INDEPENDENTLY SELECTED
FROM A GROUP OF ONE OR MORE SPECIFIED AMINO ACIDS
AS DEFINED IN THE SPECIFICATION (SECTION II.B.2.)"

(xi) SEQUENCE DESCRIPTION: SEQ ID NO:29:

Cys Xaa Xaa His Glu Leu Tyr Val Xaa Phe Xaa Asp Leu Gly Trp Xaa
1               5                   10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
65                  70                  75                  80

Asp Xaa Ser Xaa Asn Val Xaa Leu Xaa Lys Xaa Arg Asn Met Val Val
                85                  90                  95

Xaa Ala Cys Gly Cys His
            100

(2) INFORMATION FOR SEQ ID NO:30:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ5
/note= "WHEREIN EACH XAA IS INDEPENDENTLY SELECTED
FROM A GROUP OF ONE OR MORE SPECIFIED AMINO ACIDS
AS DEFINED IN THE SPECIFICATION."

(xi) SEQUENCE DESCRIPTION: SEQ ID NO:30:

Leu Xaa Xaa Xaa Phe Xaa Xaa Xaa Gly Trp Xaa Xaa Trp Xaa Xaa Xaa
1               5                   10                  15

Pro Xaa Xaa Xaa Xaa Ala Xaa Tyr Cys Xaa Gly Xaa Cys Xaa Xaa Pro
            20                  25                  30

Xaa Xaa Xaa Xaa Xaa Xaa Xaa Xaa Asn His Ala Xaa Xaa Xaa Xaa Xaa
        35                  40                  45

Xaa Xaa Xaa Xaa Xaa Xaa Xaa Xaa Xaa Xaa Xaa Xaa Cys Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Val Xaa Leu Xaa Xaa Xaa Xaa Xaa Met Xaa Val Xaa Xaa Cys Xaa Cys
                85                  90                  95

Xaa

(2) INFORMATION FOR SEQ ID NO:31:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102
(D) OTHER INFORMATION: /label= GENERIC-SEQ6
/note= "WHEREIN EACH XAA IS INDEPENDENTLY SELECTED
FROM A GROUP OF ONE OR MORE SPECIFIED AMINO ACIDS
AS DEFINED IN THE SPECIFICATION. "

(xi) SEQUENCE DESCRIPTION: SEQ ID NO:31:

Cys Xaa Xaa Xaa Xaa Leu Xaa Xaa Xaa Phe Xaa Xaa Xaa Gly Trp Xaa
1               5                   10                  15

Xaa Trp Xaa Xaa Xaa Pro Xaa Xaa Xaa Xaa Ala Xaa Tyr Cys Xaa Gly
            20                  25                  30

Xaa Cys Xaa Xaa Pro Xaa Xaa Xaa Xaa Xaa Xaa Xaa Xaa Asn His Ala
        35                  40                  45

Xaa Xaa Xaa Xaa Xaa Xaa Xaa Xaa Xaa Xaa Xaa Xaa Xaa Xaa Xaa Xaa
    50                  55                  60

Xaa Cys Cys Xaa Pro Xaa Xaa Xaa Xaa Xaa Xaa Xaa Xaa Leu Xaa Xaa
65                  70                  75                  80

Xaa Xaa Xaa Xaa Xaa Val Xaa Leu Xaa Xaa Xaa Xaa Xaa Met Xaa Val
                85                  90                  95

Xaa Xaa Cys Xaa Cys Xaa
            100

(2) INFORMATION FOR SEQ ID NO:32:

(i) SEQUENCE CHARACTERISTICS:
(A) LENGTH: 1247 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: HOMO SAPIENS
(F) TISSUE TYPE: BRAIN

(ix) FEATURE:
(A) NAME/KEY: CDS
(B) LOCATION: 84..1199
(D) OTHER INFORMATION: /product= "GDF-1"
/note= "GDF-1 CDNA"

(xi) SEQUENCE DESCRIPTION: SEQ ID NO:32:

GGGGACACCG GCCCCGCCCT CAGCCCACTG GTCCCGGGCC GCCGCGGACC CTGCGCACTC 60

TCTGGTCATC GCCTGGGAGG AAG ATG CCA CCG CCG CAG CAA GGT CCC TGC 110
Met Pro Pro Pro Gln Gln Gly Pro Cys
1               5

GGC CAC CAC CTC CTC CTC CTC CTG GCC CTG CTG CTG CCC TCG CTG CCC 158
Gly His His Leu Leu Leu Leu Leu Ala Leu Leu Leu Pro Ser Leu Pro
10                  15                  20                  25

CTG ACC CGC GCC CCC GTG CCC CCA GGC CCA GCC GCC GCC CTG CTC CAG 206
Leu Thr Arg Ala Pro Val Pro Pro Gly Pro Ala Ala Ala Leu Leu Gln
                30                  35                  40

GCT CTA GGA CTG CGC GAT GAG CCC CAG GGT GCC CCC AGG CTC CGG CCG 254
Ala Leu Gly Leu Arg Asp Glu Pro Gln Gly Ala Pro Arg Leu Arg Pro
            45                  50                  55

GTT CCC CCG GTC ATG TGG CGC CTG TTT CGA CGC CGG GAC CCC CAG GAG 302
Val Pro Pro Val Met Trp Arg Leu Phe Arg Arg Arg Asp Pro Gln Glu
        60                  65                  70

ACC AGG TCT GGC TCG CGG CGG ACG TCC CCA GGG GTC ACC CTG CAA CCG 350
Thr Arg Ser Gly Ser Arg Arg Thr Ser Pro Gly Val Thr Leu Gln Pro
    75                  80                  85

TGC CAC GTG GAG GAG CTG GGG GTC GCC GGA AAC ATC GTG CGC CAC ATC 398
Cys His Val Glu Glu Leu Gly Val Ala Gly Asn Ile Val Arg His Ile
90                  95                  100                 105

CCG GAC CGC GGT GCG CCC ACC CGG GCC TCG GAG CCT GTC TCG GCC GCG 446
Pro Asp Arg Gly Ala Pro Thr Arg Ala Ser Glu Pro Val Ser Ala Ala
                110                 115                 120

GGG CAT TGC CCT GAG TGG ACA GTC GTC TTC GAC CTG TCG GCT GTG GAA 494
Gly His Cys Pro Glu Trp Thr Val Val Phe Asp Leu Ser Ala Val Glu
            125                 130                 135

CCC GCT GAG CGC CCG AGC CGG GCC CGC CTG GAG CTG CGT TTC GCG GCG 542
Pro Ala Glu Arg Pro Ser Arg Ala Arg Leu Glu Leu Arg Phe Ala Ala
        140                 145                 150

GCG GCG GCG GCA GCC CCG GAG GGC GGC TGG GAG CTG AGC GTG GCG CAA 590
Ala Ala Ala Ala Ala Pro Glu Gly Gly Trp Glu Leu Ser Val Ala Gln
    155                 160                 165

GCG GGC CAG GGC GCG GGC GCG GAC CCC GGG CCG GTG CTG CTC CGC CAG 638
Ala Gly Gln Gly Ala Gly Ala Asp Pro Gly Pro Val Leu Leu Arg Gln
170                 175                 180                 185

TTG GTG CCC GCC CTG GGG CCG CCA GTG CGC GCG GAG CTG CTG GGC GCC 686
Leu Val Pro Ala Leu Gly Pro Pro Val Arg Ala Glu Leu Leu Gly Ala
                190                 195                 200

GCT TGG GCT CGC AAC GCC TCA TGG CCG CGC AGC CTC CGC CTG GCG CTG 734
Ala Trp Ala Arg Asn Ala Ser Trp Pro Arg Ser Leu Arg Leu Ala Leu
            205                 210                 215

GCG CTA CGC CCC CGG GCC CCT GCC GCC TGC GCG CGC CTG GCC GAG GCC 782
Ala Leu Arg Pro Arg Ala Pro Ala Ala Cys Ala Arg Leu Ala Glu Ala
        220                 225                 230

TCG CTG CTG CTG GTG ACC CTC GAC CCG CGC CTG TGC CAC CCC CTG GCC 830
Ser Leu Leu Leu Val Thr Leu Asp Pro Arg Leu Cys His Pro Leu Ala
    235                 240                 245

CGG CCG CGG CGC GAC GCC GAA CCC GTG TTG GGC GGC GGC CCC GGG GGC 878
Arg Pro Arg Arg Asp Ala Glu Pro Val Leu Gly Gly Gly Pro Gly Gly
250                 255                 260                 265

GCT TGT CGC GCG CGG CGG CTG TAC GTG AGC TTC CGC GAG GTG GGC TGG 926
Ala Cys Arg Ala Arg Arg Leu Tyr Val Ser Phe Arg Glu Val Gly Trp
                270                 275                 280

CAC CGC TGG GTC ATC GCG CCG CGC GGC TTC CTG GCC AAC TAC TGC CAG 974
His Arg Trp Val Ile Ala Pro Arg Gly Phe Leu Ala Asn Tyr Cys Gln
            285                 290                 295

GGT CAG TGC GCG CTG CCC GTC GCG CTG TCG GGG TCC GGG GGG CCG CCG 1022
Gly Gln Cys Ala Leu Pro Val Ala Leu Ser Gly Ser Gly Gly Pro Pro
        300                 305                 310

GCG CTC AAC CAC GCT GTG CTG CGC GCG CTC ATG CAC GCG GCC GCC CCG     1070
Ala Leu Asn His Ala Val Leu Arg Ala Leu Met His Ala Ala Ala Pro
    315                 320                 325

GGA GCC GCC GAC CTG CCC TGC TGC GTG CCC GCG CGC CTG TCG CCC ATC     1118
Gly Ala Ala Asp Leu Pro Cys Cys Val Pro Ala Arg Leu Ser Pro Ile
330                 335                 340                 345

TCC GTG CTC TTC TTT GAC AAC AGC GAC AAC GTG GTG CTG CGG CAG TAT     1166
Ser Val Leu Phe Phe Asp Asn Ser Asp Asn Val Val Leu Arg Gln Tyr
                350                 355                 360

GAG GAC ATG GTG GTG GAC GAG TGC GGC TGC CGC TAACCCGGGG CGGGCAGGGA     1219
Glu Asp Met Val Val Asp Glu Cys Gly Cys Arg
            365                 370

CCCGGGCCCA ACAATAAATG CCGCGTGG                                        1247

(2) INFORMATION FOR SEQ ID NO:33:

      (i) SEQUENCE CHARACTERISTICS:
           (A) LENGTH: 372 amino acids
           (B) TYPE: amino acid
           (D) TOPOLOGY: linear

     (ii) MOLECULE TYPE: protein

     (xi) SEQUENCE DESCRIPTION: SEQ ID NO:33:

Met Pro Pro Pro Gln Gln Gly Pro Cys Gly His His Leu Leu Leu Leu
  1               5                  10                  15

Leu Ala Leu Leu Leu Pro Ser Leu Pro Leu Thr Arg Ala Pro Val Pro
             20                  25                  30

Pro Gly Pro Ala Ala Ala Leu Leu Gln Ala Leu Gly Leu Arg Asp Glu
         35                  40                  45

Pro Gln Gly Ala Pro Arg Leu Arg Pro Val Pro Pro Val Met Trp Arg
     50                  55                  60

Leu Phe Arg Arg Arg Asp Pro Gln Glu Thr Arg Ser Gly Ser Arg Arg
 65                  70                  75                  80

Thr Ser Pro Gly Val Thr Leu Gln Pro Cys His Val Glu Glu Leu Gly
                 85                  90                  95

Val Ala Gly Asn Ile Val Arg His Ile Pro Asp Arg Gly Ala Pro Thr
            100                 105                 110

Arg Ala Ser Glu Pro Val Ser Ala Ala Gly His Cys Pro Glu Trp Thr
        115                 120                 125

Val Val Phe Asp Leu Ser Ala Val Glu Pro Ala Glu Arg Pro Ser Arg
    130                 135                 140

Ala Arg Leu Glu Leu Arg Phe Ala Ala Ala Ala Ala Ala Ala Pro Glu
145                 150                 155                 160

Gly Gly Trp Glu Leu Ser Val Ala Gln Ala Gly Gln Gly Ala Gly Ala
                165                 170                 175

Asp Pro Gly Pro Val Leu Leu Arg Gln Leu Val Pro Ala Leu Gly Pro
            180                 185                 190

Pro Val Arg Ala Glu Leu Leu Gly Ala Ala Trp Ala Arg Asn Ala Ser
        195                 200                 205

Trp Pro Arg Ser Leu Arg Leu Ala Leu Ala Leu Arg Pro Arg Ala Pro
    210                 215                 220

Ala Ala Cys Ala Arg Leu Ala Glu Ala Ser Leu Leu Leu Val Thr Leu
225                 230                 235                 240

Asp Pro Arg Leu Cys His Pro Leu Ala Arg Pro Arg Arg Asp Ala Glu
                245                 250                 255

Pro Val Leu Gly Gly Gly Pro Gly Gly Ala Cys Arg Ala Arg Arg Leu
            260                 265                 270

Tyr Val Ser Phe Arg Glu Val Gly Trp His Arg Trp Val Ile Ala Pro
        275                 280                 285

Arg Gly Phe Leu Ala Asn Tyr Cys Gln Gly Gln Cys Ala Leu Pro Val
    290                 295                 300

Ala Leu Ser Gly Ser Gly Gly Pro Pro Ala Leu Asn His Ala Val Leu
305                 310                 315                 320

Arg Ala Leu Met His Ala Ala Ala Pro Gly Ala Ala Asp Leu Pro Cys
                325                 330                 335

Cys Val Pro Ala Arg Leu Ser Pro Ile Ser Val Leu Phe Phe Asp Asn
            340                 345                 350

Ser Asp Asn Val Val Leu Arg Gln Tyr Glu Asp Met Val Val Asp Glu
        355                 360                 365

Cys Gly Cys Arg
    370

## Description

This invention relates generally to the dental arts and more specifically to methods and compositions for treating and regenerating periodontal tissue.

The peridontium is the cushioning tissue which anchors the tooth root to the mandibular or maxillar jawbone tissue by suspending the tooth in the tooth socket ("alveolus"). Periodontal tissue includes both the periodontal ligament, a collagen-containing tissue that is in contact with the bone tissue, and cementum, a mineralized tissue that covers the dental root surface. These two hard tissues are connected through the periodontal ligament fibers that run in a perpendicular direction to the two surfaces and thereby serve to anchor and suspend the tooth in the tooth socket, providing a shock-absorptive cushion between the tooth and the jawbone that accommodates the pressure applied to teeth when food is being chewed.

Periodontal tissue loss may occur as a result of disease, including infectious diseases (e.g., gingivitis, caused by bacteria), nutritional diseases, e.g., scurvy, resulting from a vitamin deficiency, and a number of neoplastic diseases, including acute leukemia and lymphomas. The diseases are characterized by inflammation, bleeding and ulceration. Periodontal disease also may result from an opportunistic infection, e.g., in an immune-compromised individual. Left untreated, these diseases can cause significant periodontal tissue loss which loosen the tooth and ultimately can result in loss of the tooth and the alveolar bone tissue (periodontitis.) Chronic periodontitis is the primary cause of tooth loss in adults. Current treatments include professional cleaning to remove plaque and tartar, use of oral antiseptics, local and/or systemic antibiotic therapies, and/or surgical procedures to remove periodontal pockets formed from periodontal tissue lesions and necrosis. Typically, where a tooth has been lost as a result of periodontitis, a prosthetic tooth or removable bridge is substituted for the natural tooth.

Periodontal tissue loss also may occur as a result of mechanical injury to the tissue or to the tooth itself, particularly one causing tooth loss. Tooth loss also may occur as a result of any of a number of dental diseases, e.g., dental caries, pulpitis, or osteomyelitis.

A viable tooth can be reimplanted if implantation occurs quickly after loss, e.g. , within thirty minutes, and if the periodontal tissue within the tooth socket is still healthy. However, if a significant period of time is allowed to elapse, the living periodontal tissue lining the tooth socket will be resorbed. In addition, the tooth itself begins to degenerate and a prosthetic tooth or removable bridge must be implanted. In the absence of healthy periodontal tissue the prosthetic implant is integrated directly into the jaw bone tissue in a condition called ankylosis (bone tissue in direct contact with dentin tissue.) The life of such prosthetic tooth implants often is limited due to the absence of viable periodontal tissue to enhance tooth anchoring and to absorb the impact of mastication on the prosthesis.

PCT Publication No. WO 88/00205 (Wang et al., inventors) discloses cDNA sequences for four mammalian proteins said to be capable of inducing cartilage and bone formation. Nucleic acid encoding three of these proteins, BMP-1, BMP-2 Class I(BMP-2) and BMP-2 Class II (BMP-4), was isolated from human and bovine expression libraries. Nucleic acid encoding BMP-3 was isolated from a bovine expression library. WO 88/00205 teaches that each of the four proteins may be embloyed in methods for treating bone defects, including fracture and craniofacial defect repair, fixation of artificial joints, treatment of periodontal disease and tooth repair processes: Data presented in the publication indicates that purified, recombinant human BMP-1, admixed with a rat matrix and implanted in vivo according to the method of Sampath and Reddi (1983), Proc. Natl. Acad. Sci. USA, 80:6591-6595, induces the formation of cartilage-like nodules.

PCT Publication No. WO 90/10017 (Terranova, inventor) discloses a periodontal ligament cell-attractant factor (PDL-CTX), which was shown to induce migration and proliferation of periodontal ligament cells on an artificial basement membrane. WO 90/10017 accordingly teaches that PDL-CTX may be useful for regenerating bone or periodontium. The factor is characterized as a tetramer of polypeptides, each having a molecular weight of about 12,500 d. The publication further presents guidelines for a method of periodontal regeneration, in which periodontal ligament cells are retrieved from a patient, cultured, and selected for chemotactic responsiveness to PDL-CTX. Then, a solution of PDL-CTX followed by a suspension of the selected cells are applied to the patient's tooth, after which the treated area is covered with an artificial basement membrane. WO 90/10017 does not, however, disclose results of this regeneration method.

It is an object of this invention to provide a means for inhibiting periodontal tissue loss, as well as means for inducing regeneration of damaged periodontal tissue. Another object is to provide means for inhibiting the periodontal tissue damage and tooth loss associated with periodontal and other gum diseases. Yet another object is to enhance integration of an implanted tooth, including a reimplanted natural tooth or tooth prosthesis, in the tooth socket. Still another object is to promote periodontal tissue growth around an implanted tooth. Another object is to inhibit ankylosis of an implanted tooth or tooth prosthesis.

These and other objects and features of the invention will be obvious from the specification, drawings and claims, which follow.

### Summary of the Invention

The invention provides compositions, uses and protheses as defined in the claims. These find application in inhibiting periodontal tissue loss in a mammal, particularly humans, including regenerating damaged tissue and/or inhibiting additional damage thereto. The invention also finds application in the prevention and/or inhibition of tooth loss, as well as enhancement of integration of an implanted tooth.

As used herein, "implanted tooth" includes a natural tooth which has grown naturally in the tooth socket, a natural tooth which is reimplanted in a tooth socket, and a prosthetic tooth, which includes both natural teeth from which the root has been removed and replaced with an inert, biocompatible material, and "complete" prostheses made of natural or synthetic, non dentin-containing materials. In all cases, "tooth" refers to a natural or synthetic composition essentially defining the shape of a natural tooth, having a solid tooth body, including a crown and tooth root. "Reimplanted natural tooth" includes both an allogenic tooth, e.g., selected from a tooth bank; and a tooth autologous to the mammal, such as a tooth which has fallen out, been knocked out, or otherwise removed from the individual into which it is now being reimplanted. "Integrated tooth" means an implanted tooth with a living, substantially healthy periodontal tissue, including periodontal ligament and cementum, anchoring the tooth to the jaw bone. "Viable" tissue means living, substantially healthy tissue. "Viable tooth" refers to an implanted natural tooth with a living tooth root. "Periodontium" defines the tissues which surround the tooth in the tooth socket and includes both periodontal ligament and cementum. "Inhibit loss" of periodontal tissue, as used herein, means inhibiting damage to, and/or loss of, periodontal tissue, including periodontal ligament and/or cementum, and includes regenerating lost or damaged tissue and/or inhibiting additional damage thereto. "Symptom alleviating cofactor" refers to one or more pharmaceuticals which may be administered together with the therapeutic agents of this invention and which alleviate or mitigate one or more of the symptoms typically associated with periodontal tissue loss. Exemplary cofactors include antibiotics, antiseptics, non-steroidal antiinflammatory agents, anaesthetics and analgesics.

The methods and compositions of this invention include a morphogenic protein ("morphogen"), as described herein, which, when provided to the tooth and/or jawbone surfaces in a tooth socket is capable of inducing periodontal tissue formation where periodontal tissue has been lost or damaged, and enhancing integration of an implanted tooth thereby.

In one aspect, the invention relates to a composition as defined in claim 1.

In another aspect, the invention relates to a use as defined in claim 7.

In another aspect, the invention relates to a prosthetic or replacement tooth as defined in claim 13. The invention finds application in enhancing the integration of an implanted tooth, particularly where the tooth socket is substantially reduced in viable periodontal tissue. In fact, the processes and compositions of the invention work well when a tooth socket has lost 30-50% of the periodontal ligament, and as much as 50-100% of the periodontal ligament. Applications include providing to the tooth or tooth socket surface a therapeutically effective concentration of a morphogen or morphogen-stimulating agent sufficient to induce morphogenesis of periodontal tissue. The implanted tooth may be an implanted tooth which has grown naturally in the socket and which is loose as a result of, for example, mechanical injury or due to a dental or periodontal disease. Alternatively, the implanted tooth may be a lost tooth or a tooth prosthesis which has been reimplanted in a vacant tooth socket. The tooth prosthesis may include a natural tooth from which a damaged or diseased root has been removed and replaced with a biocompatible, biologically inert material, as is created in a root canal procedure. The prosthetic tooth also may be composed of synthetic, non dentin-containing materials.

The morphogen may be provided directly to the tooth surface to be implanted, and/or to the tooth socket to which the tooth is to be implanted. Where the morphogen is to be provided to the tissue socket, it may be provided by topical administration to the tooth socket surface or by local injection to periodontal or alveolar bone tissue associated with the socket. Alternatively, an agent capable of stimulating the production and/or secretion of a therapeutically effective concentration of an endogenous morphogen also may be provided to the tooth or tooth socket. Where the morphogen or morphogen stimulating agent (referred to herein collectively as "therapeutic agent") is provided to the tooth surface, it preferably is dispersed in a biocompatible, bioresorbable carrier, most preferably a carrier capable of retaining the therapeutic agent at the tissue surface and/or providing a controlled delivery of the agent to the tooth socket. The therapeutic agent also may be provided to the tooth socket itself, also preferably in association with a carrier capable of maintaining the agent in the tooth socket, and/or capable of enhancing the controlled delivery of the agent to the socket. Useful carriers include compositions having a high viscosity, such as that provided by glycerol and the like, as well as carrier materials formulated from extracellular matrices and/or which contain laminin, collagen, and/or biocompatible synthetic polymers, such as polybutyric, polylactic, polyglycolic acids and copolymers thereof. In addition, or alternatively, an acellular carrier material may be formulated from bone, dentin, cementum or periodontal tissue by demineralizing and guanidine-extracting the tissue essentially as described herein and/or in international application US92/01968 (WO92/15323). Particularly useful acellular matrices include dentin-derived, periodontal ligament-derived and cementum-derived matrices.

In addition, the tooth to be implanted preferably comprises a porous exterior surface onto which the therapeutic agent may be adsorbed, and into which progenitor and differentiating cementoblasts can infiltrate and proliferate. Useful surfaces include natural tooth root surfaces, and porous prosthetic surfaces, including surfaces composed of matrix materials such as collagen, laminin, biocompatible polymers or metals such as titanium oxide. Where a natural tooth or dentin-containing prosthesis is to be implanted, the surface to be implanted first may be partially demineralized, e.g., by transient exposure to an acid to enhance the porosity of the tooth root surface.

Preferably, where the tooth is to be implanted into a tooth socket, the socket has been freed of fibrous tissue which may have formed following tooth loss and periodontal tissue resorption. For example, the tooth socket may have undergone a healing period of several months after loss or removal of the tooth such that scar tissue has formed over the wound. In this case the healed socket preferably is surgically prepared for tooth implantation by removing the scar and other undesired tissue to expose the alveolar bone surface.

Preferably, where the therapeutic agent is to be provided to enhance periodontal tissue viability surrounding an implanted tooth, the therapeutic agent is provided topically to the tissue surfaces between the tooth and gingiva. Alternatively, the agent may be injected locally , e.g., into the gingiva itself.

The morphogens described herein may be used to inhibit periodontal tissue loss and/or to enhance viability of periodontal tissue at risk of damage due to a periodontal disease. The periodontal disease may be caused by an infectious agent, such as a bacterial, fungal or viral agent, or by a nutritional deficiency, including a vitamin deficiency. The morphogens also may be used to regenerate periodontal tissue lost as a result of a neoplastic disease, including squamous cell carcinomas, acute leukemias, lymphomas and metastatic tumors. A detailed description of diseases which damage or destroy periodontal tissue can be found, for example, in Harrison's Principles of Internal Medicine, 243-248, (McGraw-Hill 12th ed. 1991), the disclosure of which is incorporated herein by reference. The efficacy of the morphogens described herein in modulating an inflammatory response are described in detail in international application US92/07358 (WO93/04692).

Although all individuals, and particularly adults, are at risk for periodontal tissue damage due to periodontal disease, a population most particularly at risk are immune-compromised individuals, such as individuals suffering from autoimmune diseases and/or whose immune system has been suppressed as part of a clinical procedure or therapy. Thus, in another aspect, the invention provides methods and compositions for inhibiting periodontal tissue loss in immune-compromised individuals.

As described in international application WO92/15323, and Example 2, below, the morphogens described herein also can induce formation of damaged or lost dentin tissue. Accordingly, where a natural tooth or dentin-containing prosthesis is to be implanted, a morphogen or morphogen-stimulating agent also may be provided to damaged areas of the tooth to induce dentin regeneration of damaged or lost dentin tissue. The morphogen may be provided topically or otherwise administered to the tooth tissue. For example, the morphogen may be dispersed in a biocompatible, porous carrier material that then is provided topically to the damaged dentin tissue. A useful carrier may be formulated from dentin by demineralizing and guanidine-extracting the tissue to create an acellular matrix.

The compositions of the invention may be provided to the periodontium together with other molecules ("cofactors") known to have a beneficial effect in treating damaged periodontal tissue, particularly cofactors capable of mitigating or alleviating symptoms typically associated with periodontal tissue damage and/or loss. Examples of such cofactors include antiseptics such as chlorohexidine and tibezonium iodide, antibiotics, including tetracycline, aminoglycosides, macrolides, penicillins and cephalosporins, anaesthetics and analgesics, and other non-steroidal anti-inflammatory agents (with the promise that tetracycline and salicylic acid are excluded).

Among the morphogens useful in this invention are proteins originally identified as osteogenic proteins (see U.S. Patent 5,011,691, incorporated herein by reference), such as the OP-1, OP-2 and CBMP2 proteins, as well as amino acid sequence-related proteins such as DPP (from Drosophila), Vgl (from Xenopus), Vgr-1 (from mouse), GDF-1 (from mouse, see Lee (1991) PNAS 88:4250-4254), all of which are presented in Table II and Seq. ID Nos. 5-14, and the recently identified 60A protein (from Drosophila, Seq. ID No. 24, see Wharton et al. (1991) PNAS 88:9214-9218.) The members of this family, which include members of the TGF-β super-family of proteins, share substantial amino acid sequence homology in their C-terminal regions. The proteins are translated as a precursor, having an N-terminal signal peptide sequence, typically less than about 30 residues, followed by a "pro" domain that is cleaved to yield the mature sequence. The "pro" form of the protein includes the pro domain and the mature domain, and forms a soluble species that appears to be the primary form secreted from cultured mammalian cells. The signal peptide is cleaved rapidly upon translation, at a cleavage site that can be predicted in a given sequence using the method of Von Heijne ((1986) Nucleic Acids Research 14:4683-4691.) Table I, below, describes the various morphogens identified to date, including their nomenclature as used herein, their Seq. ID references, and publication sources for the amino acid sequences for the full length proteins not included in the Seq. Listing. The disclosure of these publications is incorporated herein by reference.

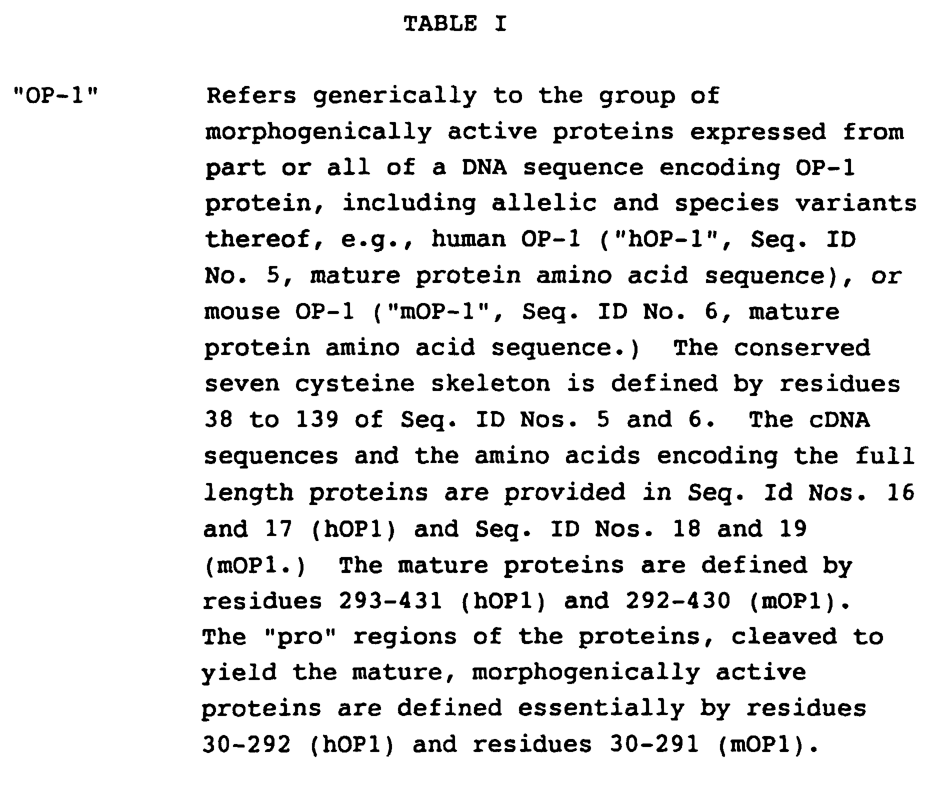

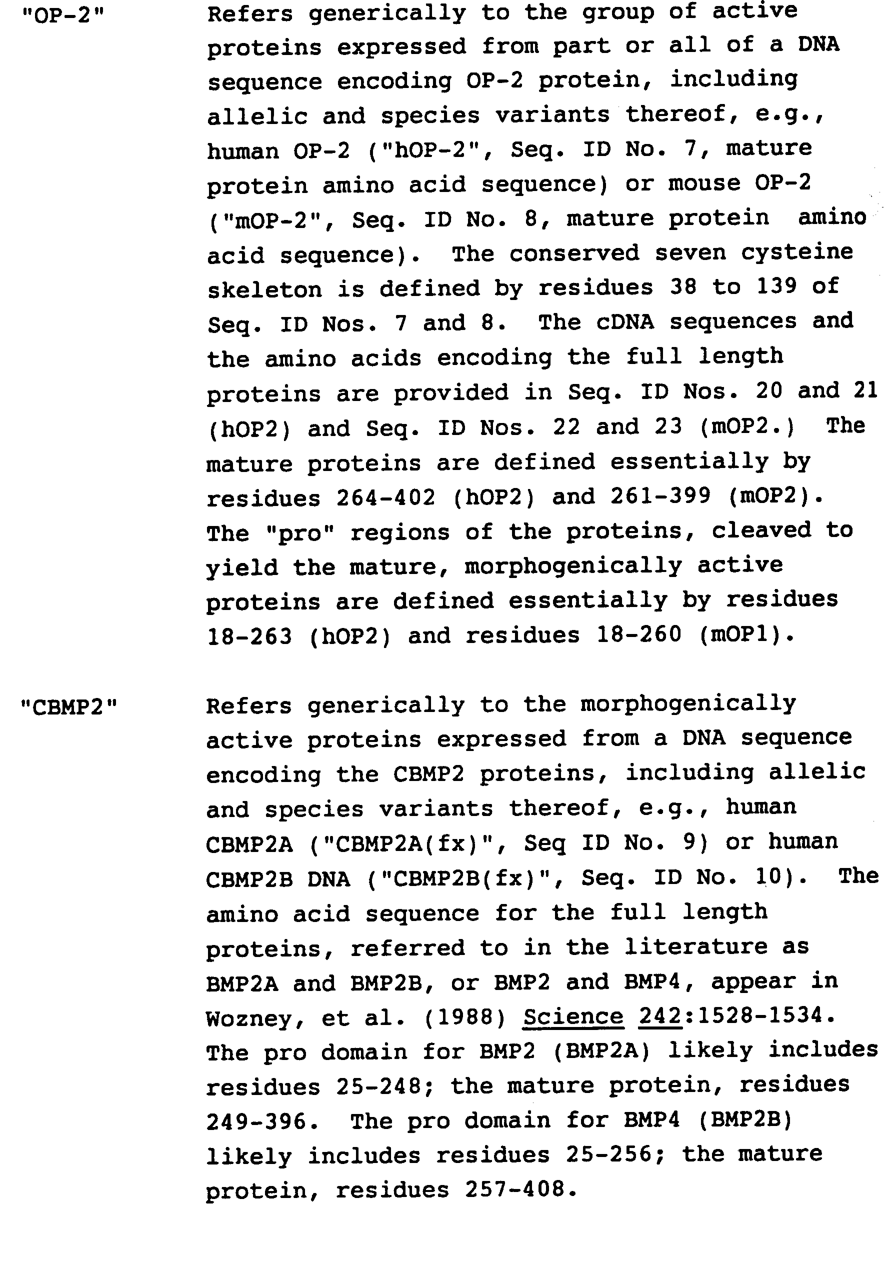

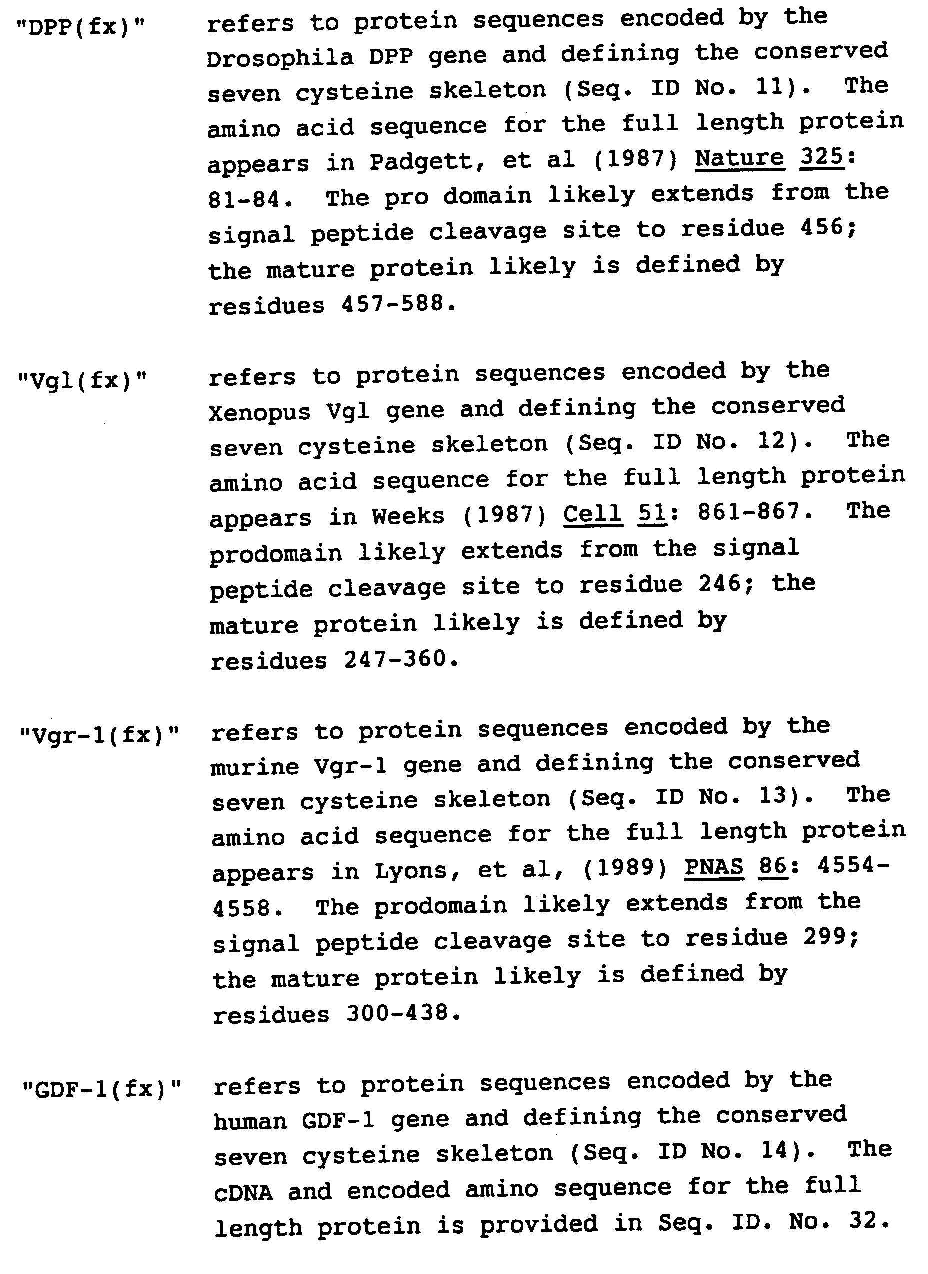

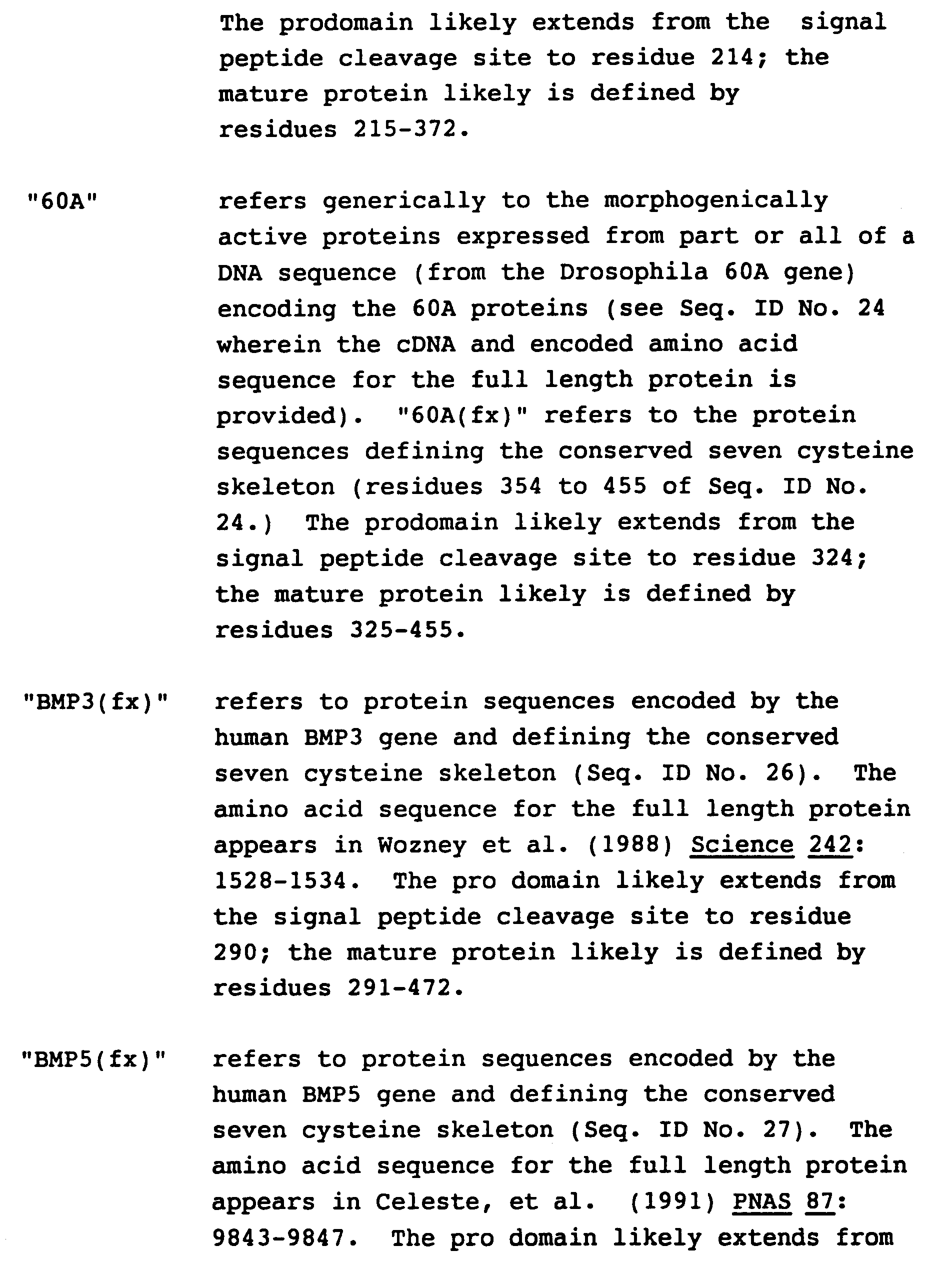

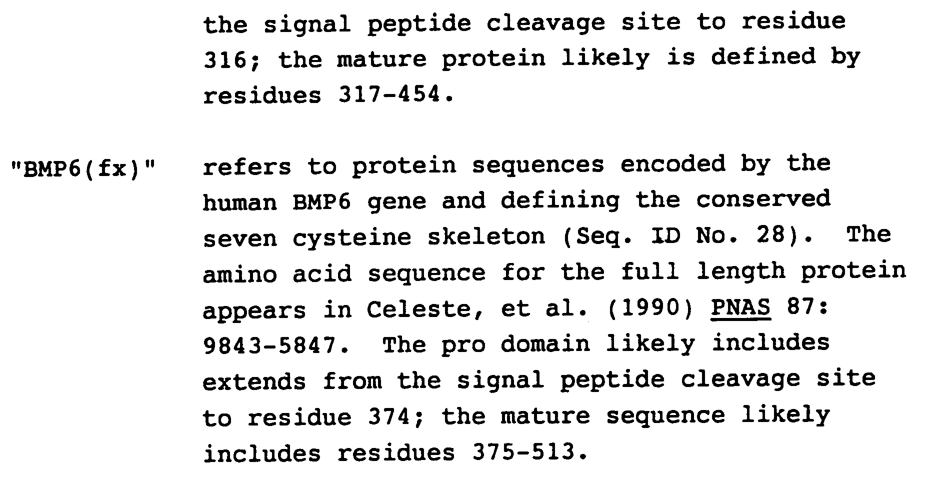

The OP-2 proteins have an additional cysteine residue in the conserved region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens of this invention. Thus, as defined herein, a morphogen is a dimeric protein comprising a pair of polypeptide chains, wherein each polypeptide chain comprises at least the C-terminal six cysteine skeleton defined by residues 43-139 of Seq. ID No. 5, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- and/or inter-chain disulfide bonds such that the protein is capable of acting as a morphogen as defined herein. Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells. In addition, it is also anticipated that these morphogens are capable of inducing redifferentiation of committed cells under appropriate environmental conditions.

In one preferred aspect, the morphogens of this invention comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36, Seq. ID No. 2). In another preferred aspect, these sequences further comprise the following additional sequence at their N-terminus:

Cys Xaa Xaa Xaa Xaa (Seq. ID No. 15)
1 5

Preferred amino acid sequences within the foregoing generic sequences include: Generic Sequence 3 (Seq. ID No. 3), Generic Sequence 4 (Seq. ID No. 4), Generic Sequence 5 (Seq. ID No. 30) and Generic Sequence 6 (Seq. ID No. 31), listed below. These Generic Sequences accommodate the homologies shared among the various preferred members of this morphogen family identified in Table II, as well as the amino acid sequence variation among them. Generic Sequences 3 and 4 are composite amino acid sequences of the proteins presented in Table II and identified in Seq. ID Nos. 5-14, specifically: human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14.) The generic sequences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins. 
Generic Sequence 3
Leu Tyr Val Xaa Phe
1               5
Xaa Xaa Xaa Gly Trp Xaa Xaa Trp Xaa
                10
Xaa Ala Pro Xaa Gly Xaa Xaa Ala
15                  20
Xaa Tyr Cys Xaa Gly Xaa Cys Xaa
        25                  30
Xaa Pro Xaa Xaa Xaa Xaa Xaa
                35
Xaa Xaa Xaa Asn His Ala Xaa Xaa
        40                  45
Xaa Xaa Leu Xaa Xaa Xaa Xaa Xaa
                50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Gly Cys Xaa
        95
 wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (Ile or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or Ile); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (Ile or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (Ile, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or Ile); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (Ile, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (Ile or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg); 
Generic Sequence 4

Cys Xaa Xaa Xaa Xaa Leu Tyr Val Xaa Phe
1               5                   10
Xaa Xaa Xaa Gly Trp Xaa Xaa Trp Xaa
                15
Xaa Ala Pro Xaa Gly Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Leu Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
 
Xaa Xaa Xaa Xaa Val Xaa Leu Xaa
                85
Xaa Xaa Xaa Xaa Met Xaa Val Xaa
 90                     95
Xaa Cys Gly Cys Xaa
        100
 wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (Ile or Val); Xaa at res.20 = (Ile or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or lie); Xaa at res.42 = (Met, Phe, Gly or Leu); Xaa at res.44 = (Ala, Ser or Gly); Xaa at res.45 = (Thr, Leu or Ser); Xaa at res.49 = (Ile or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (Ile, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or Ile); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Glu, Lys, Arg or Glu); Xaa at res.72 = (Leu, Met or Val); Xaa at res.73 = (Ash, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (Ile or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Similarly, Generic Sequence 5 (Seq. ID No. 30) and Generic Sequence 6 (Seq. ID No. 31) accommodate the homologies shared among all the morphogen protein family members identified in Table II. Specifically, Generic Sequences 5 and 6 are composite amino acid sequences of human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14), human BMP3 (Seq. ID No. 26), human BMP5 (Seq. ID No. 27), human BMP6 (Seq. ID No. 28) and 60(A) (from Drosophila, Seq. ID Nos. 24-25). The generic sequences include both the amino acid identity shared by these sequences in the C-terminal domain, defined by the six and seven cysteine skeletons (Generic Sequences 5 and 6, respectively), as well as alternative residues for the variable positions within the sequence. As for Generic Sequences 3 and 4, Generic Sequences 5 and 6 allow for an additional cysteine at position 41 (Generic Sequence 5) or position 46 (Generic Sequence 6), providing an appropriate cysteine skeleton where inter- or intramolecular disulfide bonds can form, and containing certain critical amino acids which influence the tertiary structure of the proteins. 
Generic Sequence 5

Leu Xaa Xaa Xaa Phe
1               5
Xaa Xaa Xaa Gly Trp Xaa Xaa Trp Xaa
                10
Xaa Xaa Pro Xaa Xaa Xaa Xaa Ala
15                  20
Xaa Tyr Cys Xaa Gly Xaa Cys Xaa
        25                  30
Xaa Pro Xaa Xaa Xaa Xaa Xaa
                35
Xaa Xaa Xaa Asn His Ala Xaa Xaa
        40                  45
Xaa Xaa Xaa Xaa Xaa Xaa Xaa Xaa
                50
 
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Xaa Cys Xaa
        95
 wherein each Xaa is independently selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp, Glu or Lys); Xaa at res.8 = (Leu, Val or Ile); Xaa at res.11 = (Gln, Leu, Asp, His, Asn or Ser); Xaa at res.12 = (Asp, Arg, Asn or Glu); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln or Ser); Xaa at res.28 = (Glu, Lys, Asp, Gln or Ala); Xaa at res.30 = (Ala, Ser, Pro, Gln or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val or Lys); Xaa at res.56 = (Thr, Ala, Val, Lys, Asp, Tyr, Ser, Ala, Pro or His); Xaa at res.57 = (Val, Ala or Ile); Xaa at res.58 = (Pro or Asp); Xaa at res.59 = (Lys, Leu or Glu); Xaa at res.60 = (Pro or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His or Val); Xaa at res.86 = (Tyr or His); Xaa at res.87 = (Arg, Gln, Glu or Pro); Xaa at res.88 = (Asn, Glu or Asp); Xaa at res.90 = (Val, Thr, Ala or Ile); Xaa at res.92 = (Arg, Lys, Val, Asp or Glu); Xaa at res.93 = (Ala, Gly, Glu or Ser); Xaa at res.95 = (Gly or Ala) and Xaa at res.97 = (His or Arg). 
Generic Sequence 6

Cys Xaa Xaa Xaa Xaa Leu Xaa Xaa Xaa Phe
1               5                   10
Xaa Xaa Xaa Gly Trp Xaa Xaa Trp Xaa
                15
Xaa Xaa Pro Xaa Xaa Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Xaa Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
Xaa Xaa Xaa Xaa Val Xaa Leu Xaa
            85
Xaa Xaa Xaa Xaa Met Xaa Val Xaa
90                  95
Xaa Cys Xaa Cys Xaa
        100
 wherein each Xaa is independently selected from a group of one or more specified amino acids as defined by the following: "Res." means "residue" and Xaa at res.2 = (Lys, Arg, Ala or Gln); Xaa at res.3 = (Lys, Arg or Met); Xaa at res.4 = (His, Arg or Gln); Xaa at res.5 = (Glu, Ser, His, Gly, Arg, Pro, Thr, or Tyr); Xaa at res.7 = (Tyr or Lys); Xaa at res.8 = (Val or Ile); Xaa at res.9 = (Ser, Asp or Glu); Xaa at res.11 = (Arg, Gln, Ser, Lys or Ala); Xaa at res.12 = (Asp, Glu, or Lys); Xaa at res.13 = (Leu, Val or Ile); Xaa at res.16 = (Gln, Leu, Asp, His, Asn or Ser); Xaa at res.17 = (Asp, Arg, Asn or Glu); Xaa at res.19 = (Ile or Val); Xaa at res.20 = (Ile or Val); Xaa at res.21 = (Ala or Ser); Xaa at res.23 = (Glu, Gln, Leu, Lys, Pro or Arg); Xaa at res.24 = (Gly or Ser); Xaa at res.25 = (Tyr or Phe); Xaa at res.26 = (Ala, Ser, Asp, Met, His, Gln, Leu, or Gly); Xaa at res.28 = (Tyr, Asn or Phe); Xaa at res.31 = (Glu, His, Tyr, Asp, Gln or Ser); Xaa at res.33 = Glu, Lys, Asp, Gln or Ala); Xaa at res.35 = (Ala, Ser, Pro, Gln or Asn); Xaa at res.36 = (Phe, Leu or Tyr); Xaa at res.38 = (Leu, Val or Met); Xaa at res.39 = (Asn, Asp, Ala, Thr or Pro); Xaa at res.40 = (Ser, Asp, Glu, Leu, Ala or Lys); Xaa at res.41 = (Tyr, Cys, His, Ser or Ile); Xaa at res.42 = (Met, Phe, Gly or Leu); Xaa at res.43 = (Asn, Ser or Lys); Xaa at res.44 = (Ala, Ser, Gly or Pro); Xaa at res.45 = (Thr, Leu or Ser); Xaa at res.49 = (Ile, Val or Thr); Xaa at res.50 = (Val, Leu or Ile); Xaa at res.51 = (Gln or Arg); Xaa at res.52 = (Thr, Ala or Ser); Xaa at res.53 = (Leu or Ile); Xaa at res.54 = (Val or Met); Xaa at res.55 = (His, Asn or Arg); Xaa at res.56 = (Phe, Leu, Asn, Ser, Ala or Val); Xaa at res.57 = (Ile, Met, Asn, Ala, Val or Leu); Xaa at res.58 = (Asn, Lys, Ala, Glu, Gly or Phe); Xaa at res.59 = (Pro, Ser or Val); Xaa at res.60 = (Glu, Asp, Gly, Val or Lys); Xaa at res.61 = (Thr, Ala, Val, Lys, Asp, Tyr, Ser, Ala, Pro or His); Xaa at res.62 = (Val, Ala or Ile); Xaa at res.63 = (Pro or Asp); Xaa at res.64 = (Lys, Leu or Glu); Xaa at res.65 = (Pro or Ala); Xaa at res.68 = (Ala or Val); Xaa at res.70 = (Thr, Ala or Glu); Xaa at res.71 = (Gln, Lys, Arg or Glu); Xaa at res.72 = (Leu, Met or Val); Xaa at res.73 = (Asn, Ser, Asp or Gly); Xaa at res.74 = (Ala, Pro or Ser); Xaa at res.75 = (Ile, Thr, Val or Leu); Xaa at res.76 = (Ser, Ala or Pro); Xaa at res.77 = (Val, Met or Ile); Xaa at res.79 = (Tyr or Phe); Xaa at res.80 = (Phe, Tyr, Leu or His); Xaa at res.81 = (Asp, Asn or Leu); Xaa at res.82 = (Asp, Glu, Asn or Ser); Xaa at res.83 = (Ser, Gln, Asn, Tyr or Asp); Xaa at res.84 = (Ser, Asn, Asp, Glu or Lys); Xaa at res.85 = (Asn, Thr or Lys); Xaa at res.87 = (Ile, Val or Asn); Xaa at res.89 = (Lys or Arg); Xaa at res.90 = (Lys, Asn, Gln, His or Val); Xaa at res.91 = (Tyr or His); Xaa at res.92 = (Arg, Gln, Glu or Pro); Xaa at res.93 = (Asn, Glu or Asp); Xaa at res.95 = (Val, Thr, Ala or Ile); Xaa at res.97 = (Arg, Lys, Val, Asp or Glu); Xaa at res.98 = (Ala, Gly, Glu or Ser); Xaa at res.100 = (Gly or Ala); and Xaa at res.102 = (His or Arg).

Particularly useful sequences for use as morphogens in this invention include the C-terminal domains, e.g., the C-terminal 96-102 amino acid residues of Vgl, Vgr-1, DPP, OP-1, OP-2, CBMP-2A, CBMP-2B, GDF-1 (see Table II, below, and Seq. ID Nos. 5-14), as well as proteins comprising the C-terminal domains of 60A, BMP3, BMP5 and BMP6 (see Seq. ID Nos. 24-28), all of which include at least the conserved six or seven cysteine skeleton. In addition, biosynthetic constructs designed from the generic sequences, such as COP-1, 3-5, 7, 16, disclosed in U.S. Pat. No. 5,011,691, also are useful. Other sequences include the inhibins/activin proteins (see, for example, U.S. Pat. Nos. 4,968,590 and 5,011,691). Accordingly, other useful sequences are those sharing at least 70% amino acid sequence homology or "similarity", and preferably 80% homology or similarity with any of the sequences above. These are anticipated to include allelic, species variants and other sequence variants (e.g., including "muteins" or "mutant proteins"), whether naturally-occurring or biosynthetically produced, as well as novel members of this morphogenic family of proteins. As used herein, "amino acid sequence homology" is understood to mean amino acid sequence similarity, and homologous sequences share identical or similar amino acids, where similar amino acids are conserved amino acids as defined by Dayoff et al., Atlas of Protein Sequence and Structure; vol.5, Suppl.3, pp.345-362 (M.O. Dayoff, ed., Nat'l BioMed. Research Fdn., Washington D.C. 1978.) Thus, a candidate sequence sharing 70% amino acid homology with a reference sequence requires that, following alignment of the candidate sequence with the reference sequence, 70% of the amino acids in the candidate sequence are identical to the corresponding amino acid in the reference sequence, or constitute a conserved amino acid change thereto. "Amino acid sequence identity" is understood to require identical amino acids between two aligned sequences. Thus, a candidate sequence sharing 60% amino acid identity with a reference sequence requires that, following alignment of the candidate sequence with the reference sequence, 60% of the amino acids in the candidate sequence are identical to the corresponding amino acid in the reference sequence.

As used herein, all homologies and identities calculated use OP-1 as the reference sequence. Also as used herein, sequences are aligned for homology and identity calculations using the method of Needleman et al. (1970) J.Mol. Biol. 48:443-453 and identities calculated by the Align program (DNAstar, Inc.) In all cases, internal gaps and amino acid insertions in the candidate sequence as aligned are ignored when making the homology/identity calculation.

The currently most preferred protein sequences useful as morphogens in this invention include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, in another preferred aspect of the invention, useful morphogens include active proteins comprising species of polypeptide chains having the generic amino acid sequence herein referred to as "OPX" (Seq. ID No. 29), which defines the seven cysteine skeleton and accommodates the homologies between the various identified species of OP1 and OP2. As described therein, each Xaa at a given position independently is selected from the residues occurring at the corresponding position in the C-terminal sequence of mouse or human OP1 or OP2 (see Seq. ID Nos. 5-8 and/or Seq. ID Nos. 16-23).

In still another preferred aspect of the invention, useful morphogens include dimeric proteins comprising amino acid sequences encoded by nucleic acids that hybridize to DNA or RNA sequences encoding the C-terminal sequences defining the conserved seven cysteine domain of OP1 or OP2, e.g., nucleotides 1036-1341 and nucleotides 1390-1695 of Seq. ID No. 16 and 20, respectively, under stringent hybridization conditions. As used herein, stringent hybridisation conditions are defined as hybridization in 40% formamide, 5 X SSPE, 5 X Denhardt's Solution, and 0.1% SDS at 37°C overnight, and washing in 0.1 X SSPE, 0.1% SDS at 50°C.

The morphogens useful in the methods, compositions and devices of this invention include proteins comprising any of the polypeptide chains described above, whether isolated from naturally-occurring sources, or produced by recombinant DNA or other synthetic techniques, and includes allelic and species variants of these proteins, naturally-occurring or biosynthetic mutants thereof, as well as various truncated and fusion constructs. Deletion or addition mutants also are envisioned to be active, including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells. A detailed description of the morphogens useful in the methods, compositions and devices of this invention is disclosed in copending US patent application Serial Nos. 752,764, filed August 30, 1991, and 667,724, filed March 11, 1991, the disclosures of which are incorporated herein by reference.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of stimulating the morphogenesis of, and/or inhibiting damage to, periodontal tissue.

Other features and advantages of the invention will be apparent from the following description of the preferred embodiments, and from the claims.

### Brief Description of the Drawings

The foregoing and other objects and features of this invention, as well as the invention itself, may be more fully understood from the following description, when read together with the accompanying drawings, in which:
Fig. 1 is a schematic illustration of a healthy tooth in the tooth socket; and
Fig. 2 (A and B) are photomicrographs demonstrating the effect of morphogen (2A) or carrier alone (2B) on periodontal tissue regeneration in a surgically prepared canine tooth socket.
Fig.3 (A and B) are photomicrographs demonstrating the effect of morphogen (3A) or carrier alone (3B) on dentine tissue regeneration in a surgically exposed dental pulp experiment.

### Detailed Description

It has been discovered that the morphogens described herein can stimulate periodontal tissue formation, including regenerating lost or damaged periodontal ligament and/or cementum. The invention may be used for tooth implant integration as well as to inhibit and/or repair periodontal tissue loss due to disease or mechanical injury.

Provided below is a description of tooth anatomy and useful morphogens, including methods for their production and formulation, as well as exemplary, non-limiting examples which (1) demonstrate the suitability of the morphogens described herein in the methods of the invention, and (2) provide assays with which to test candidate morphogens for their efficacy.

### I. Tooth Anatomy

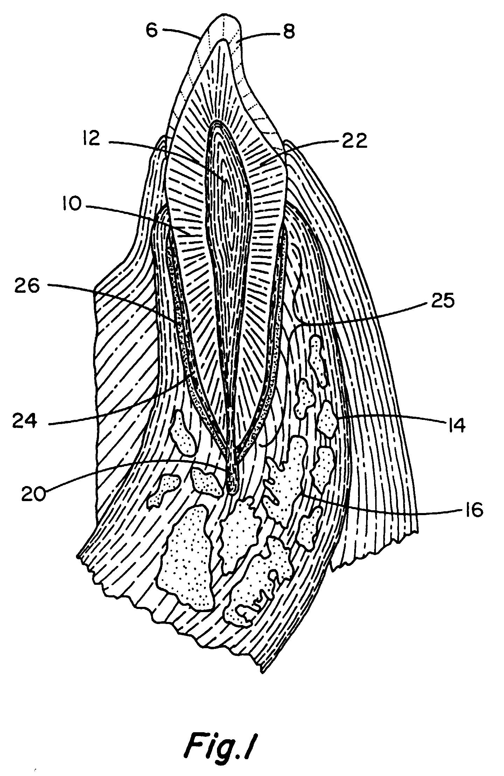

A vertical section of a tooth in the tooth socket is shown schematically in Fig. 1. The crown 6 of the tooth is composed of enamel 8 and dentin 22. The pulp chamber 12 is seen in the interior of the crown 6 and the center of the root 10; it extends downward into the bony area 14, 16, 18 and opens by a minute orifice, the apical foramen 20, at the extremity of the root 10. The pulp chamber 12 contains dental pulp, a loose connective tissue richly supplied with vessels and nerves, which enter the cavity through the apical foramen 20. Some of the cells of the pulp, i.e., odontoblasts, the precursors of dentin 22, are arranged as a layer on the wall of the pulp chamber 12. During development of the tooth, odontoblasts are columnar, but later, after the dentin 22 is fully formed, they become flattened and resemble osteoblasts.

The solid portion of the mature tooth includes dentin 22, enamel 8, and a thin layer of cementum 24, which is disposed on the surface of the root 25. Enamel 8 is formed during development of the tooth from amyloblasts, and cementum 24 is formed from cementoblasts. In a fully developed tooth, the principal mass of the tooth comprises dentin 22, which is made up of hydroxyapatite crystals embedded in a strong meshwork of collagen fibers. The dentin includes a number of minute wavy and branching tubes called dental canaliculi, embedded in a dense homogeneous substance, the matrix. The dental canaliculi are parallel with one another and open at their inner ends into the pulp chamber 12. The dentin matrix is translucent and comprises the majority of the inorganic mass of the dentin. It includes a number of fine fibrils, which are continuous with the fibrils of the dental pulp. After the organic matter has been removed by steeping a tooth in weak acid, the remaining organic matter may be torn into laminae that run parallel with the pulp chamber 12 across the direction of the tubes.

The cementum 24 is disposed as a thin mineralized layer covering the tooth root. It extends from where the enamel terminates to the apex of each root, where it is usually very thick. Cementum resembles bone in structure and chemical composition in that it contains, sparingly, the lacunae and canaliculi that characterize true bone; in the thicker portions of the cementum, the lamellae and Haversian canals peculiar to bone are also found. As a result of aging, the cementum increases in thickness and the pulp chamber also becomes partially filled with a hard substance that is intermediate in structure between dentin and bone.

It appears to be formed by a slow conversion of the dental pulp, which shrinks or even disappears.

The periodontal ligament, or periodontal membrane 26, is the layer of periodontal tissue which forms a cushion between the cementum 24 and the bone 14, 16, 18; it holds the tooth in position by suspending it in the socket (alveolus) of the jawbone. The periodontal ligament is a highly organized tissue which is formed from periodontal fibroblasts. It organizes the collagen fibers which pass directly from the bone of the jaw into the cementum.

### II. Useful Morphogens

As defined herein a protein is morphogenic if it is capable of inducing the developmental cascade of cellular and molecular events that culminate in the formation of new, organ-specific tissue and comprises at least the conserved C-terminal six cysteine skeleton or its functional equivalent (see supra). Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells. Details of how the morphogens useful in the method of this invention first were identified, as well as a description on how to make, use and test them for morphogenic activity are disclosed in international application (US92/01968 (WO92/15323), the disclosure of which is incorporated hereinabove by reference. As disclosed therein, the morphogens may be purified from naturally-sourced material or recombinantly produced from procaryotic or eucaryotic host cells, using the genetic sequences disclosed therein.

Alternatively, novel morphogenic sequences may be identified following the procedures disclosed therein.

Particularly useful proteins include those which comprise the naturally derived sequences disclosed in Table II. Other useful sequences include biosynthetic constructs such as those disclosed in U.S. Pat. 5,011,691, the disclosure of which is incorporated herein by reference (e.g., COP-1, COP-3, COP-4, COP-5, COP-7, and COP-16).

Accordingly, the morphogens useful in the methods and compositions of this invention also may be described by morphogenically active proteins having amino acid sequences sharing 70% or, preferably, 80% homology (similarity) with any of the sequences described above, where "homology" is as defined herein above.

The morphogens useful in the method of this invention also can be described by any of the 6 generic sequences described herein (Generic Sequences 1, 2, 3, 4, 5 and 6). Generic sequences 1 and 2 also may include, at their N-terminus, the sequence

Cys Xaa Xaa Xaa Xaa (Seq. ID No. 15)
  1               5

Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-23), CBMP2A (Seq. ID No. 9), CBMP2B (Seq. ID No. 10), BMP3 (Seq. ID No. 26), DPP (from Drosophila, Seq. ID No. 11), Vgl, (from Xenopus, Seq. ID No. 12), Vgr-1 (from mouse, Seq. ID No. 13), GDF-1 (from mouse, Seq. ID Nos. 14, 32 and 33), 60A protein (from Drosophila, Seq. ID Nos. 24 and 25), BMP5 (Seq. ID No. 27) and BMP6 (Seq. ID No. 28). The sequences are aligned essentially following the method of Needleman et al. (1970) J. Mol. Biol., 48:443-453, calculated using the Align Program (DNAstar, Inc.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and Ile, whereas CBMP-2B comprises Ser and Ile.

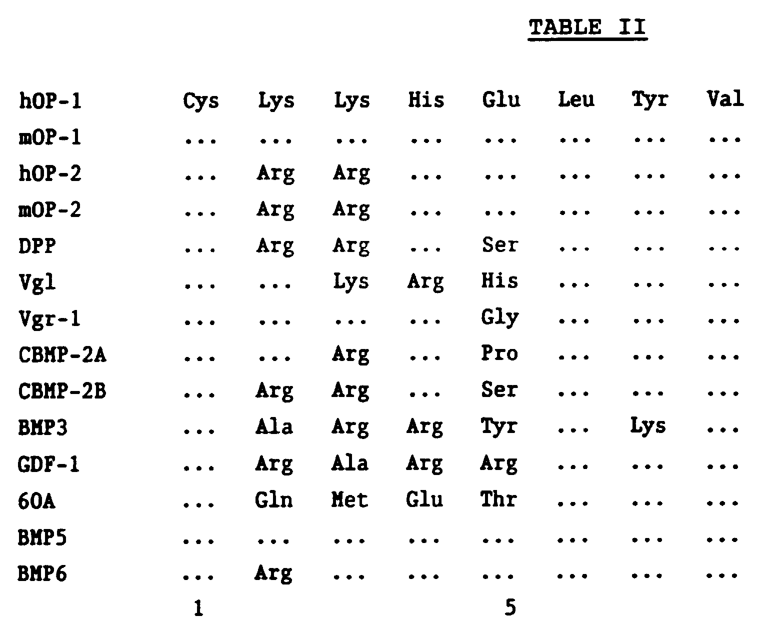

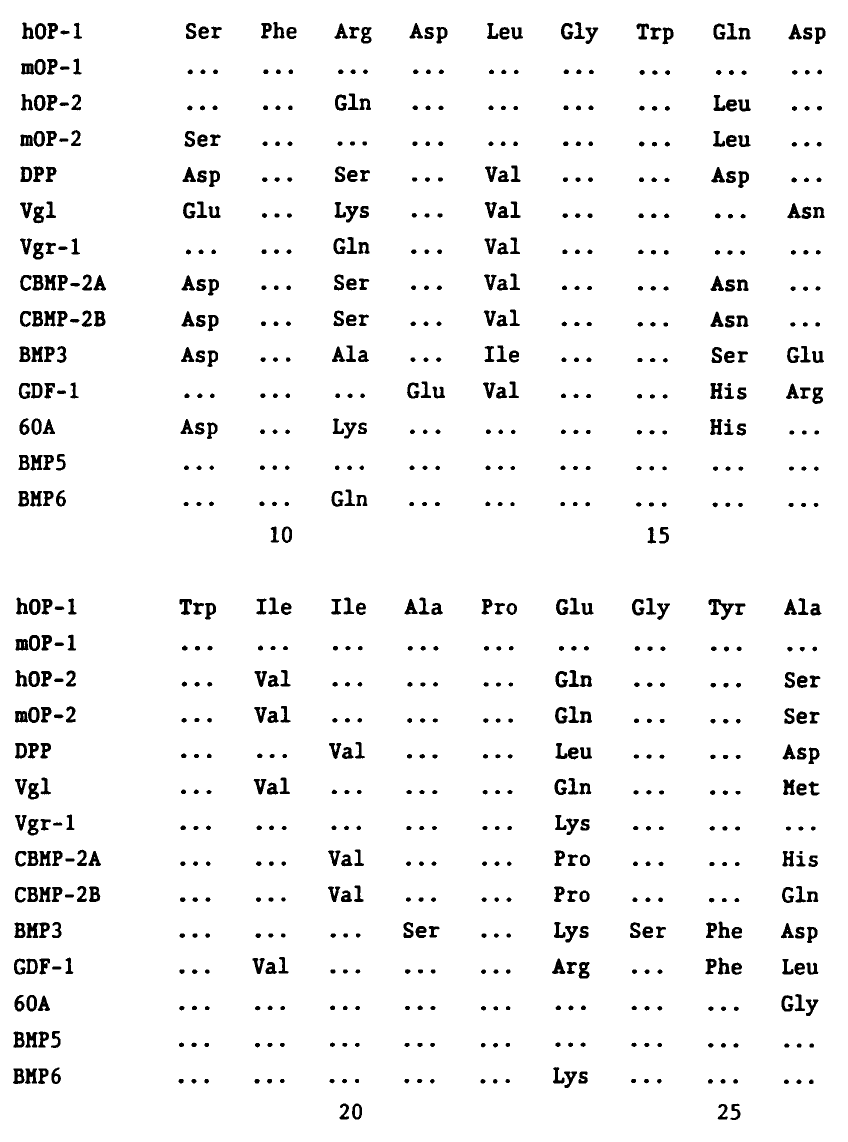

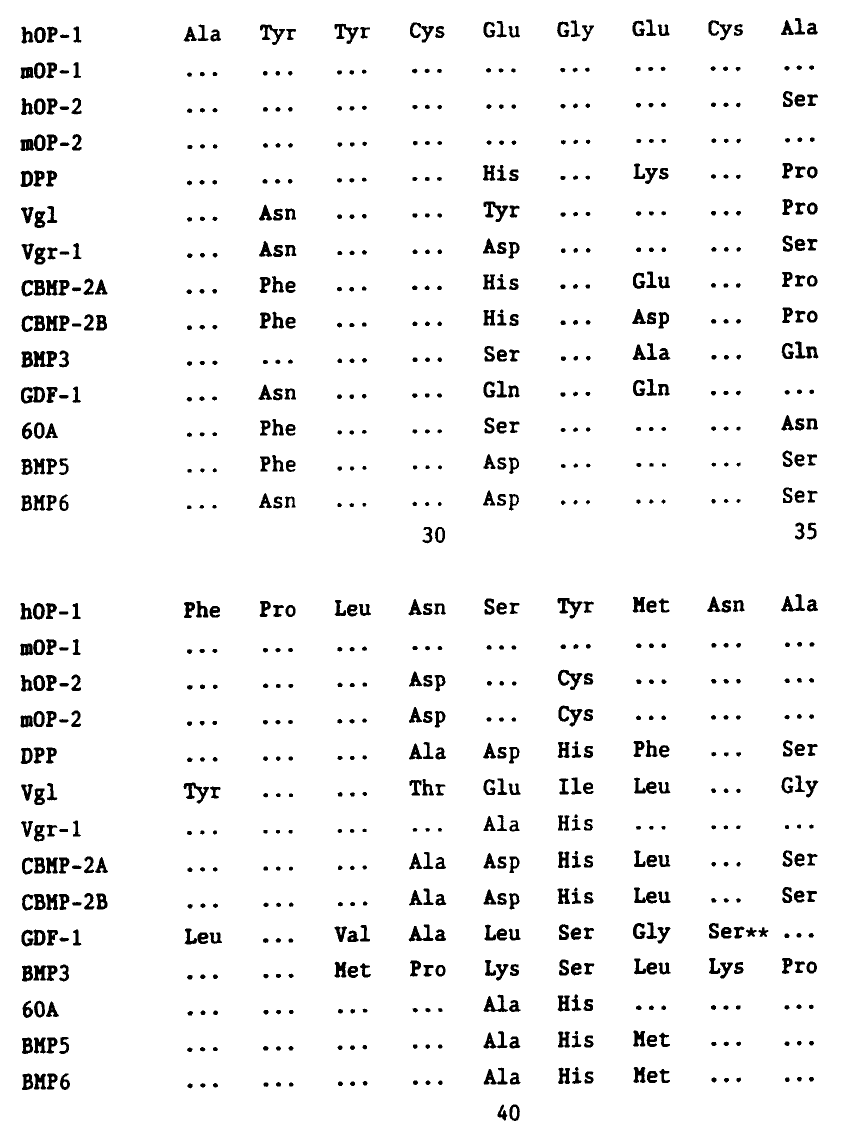

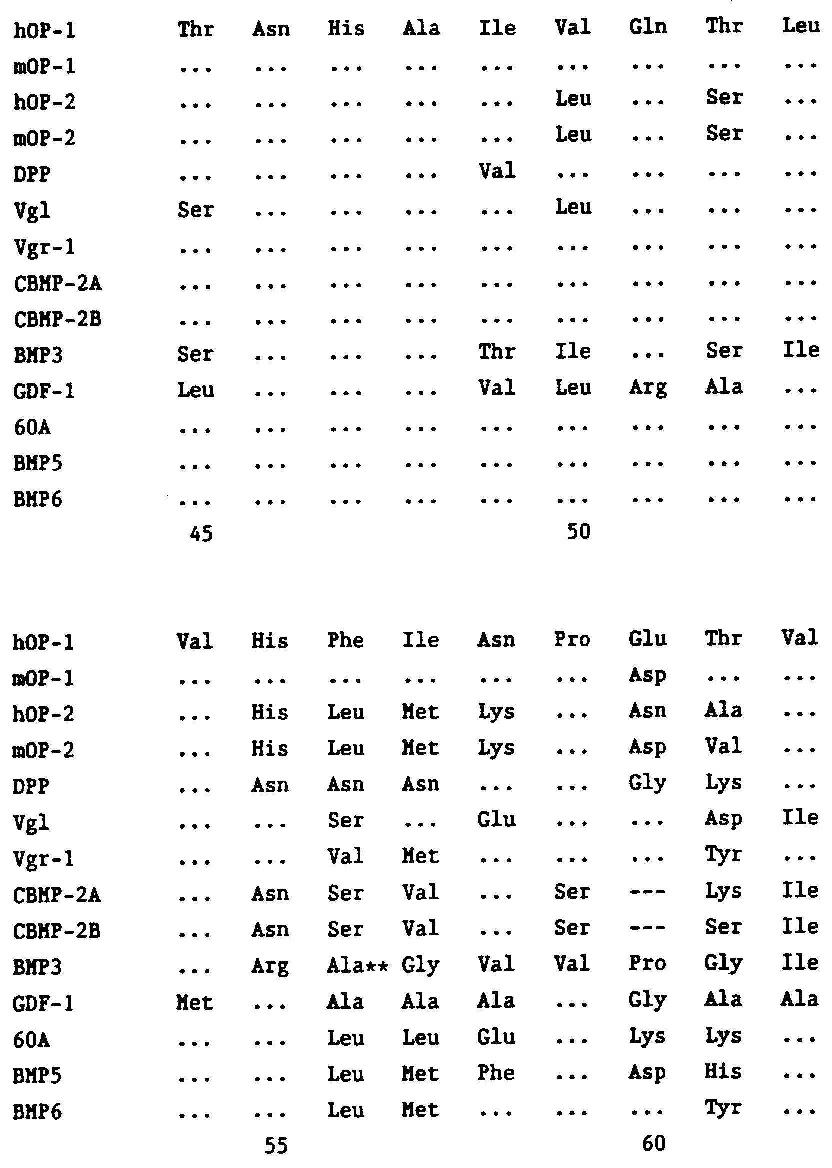

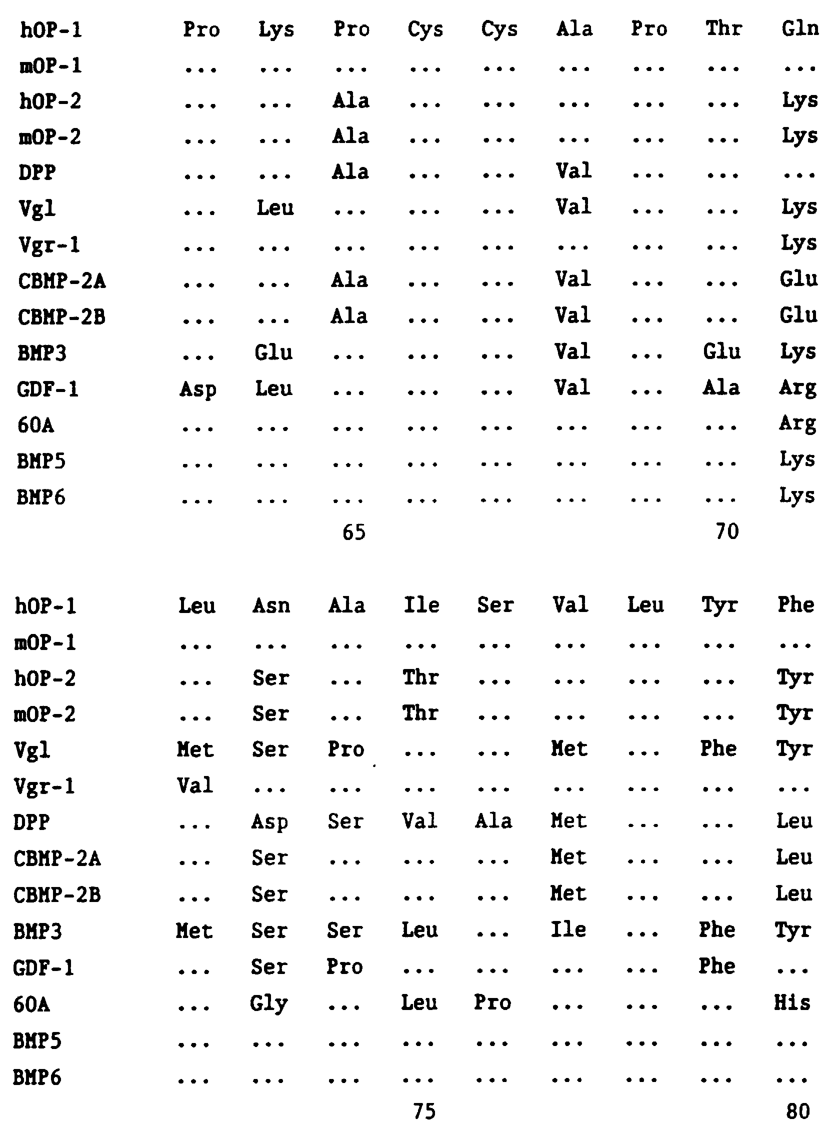

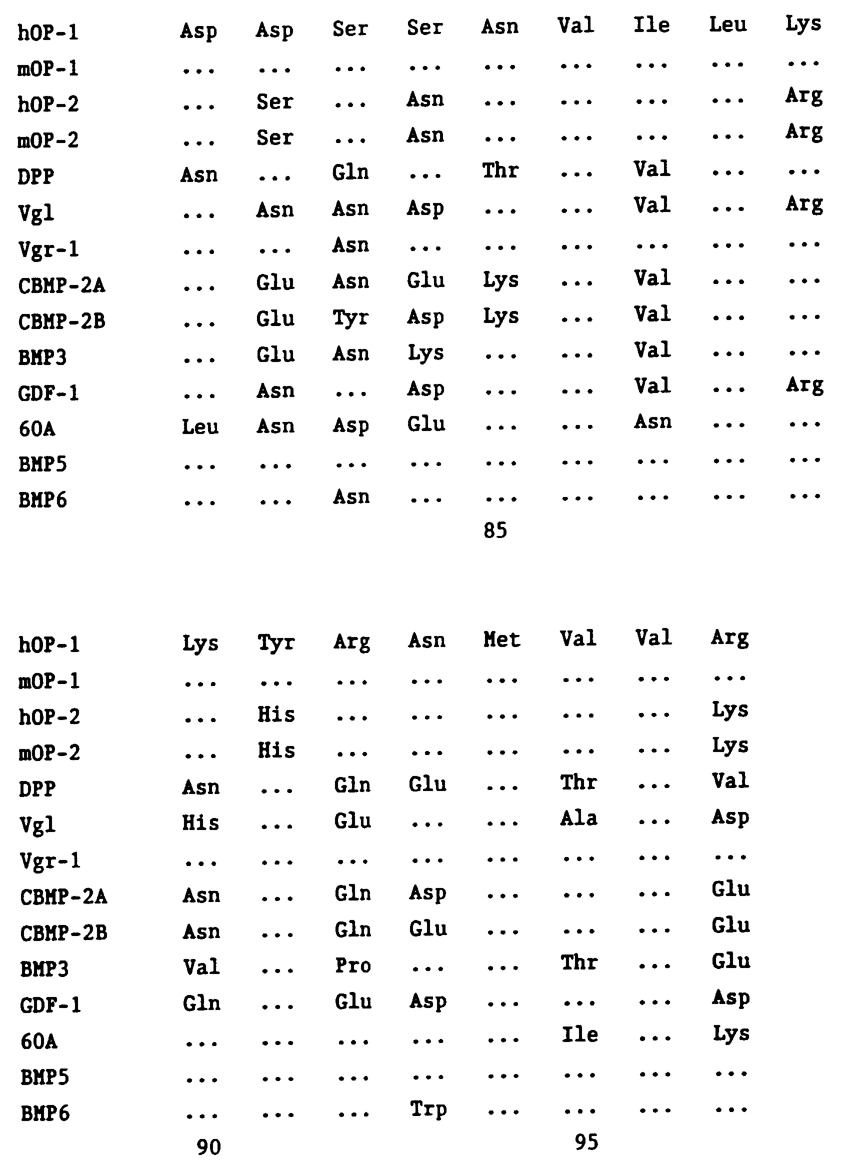

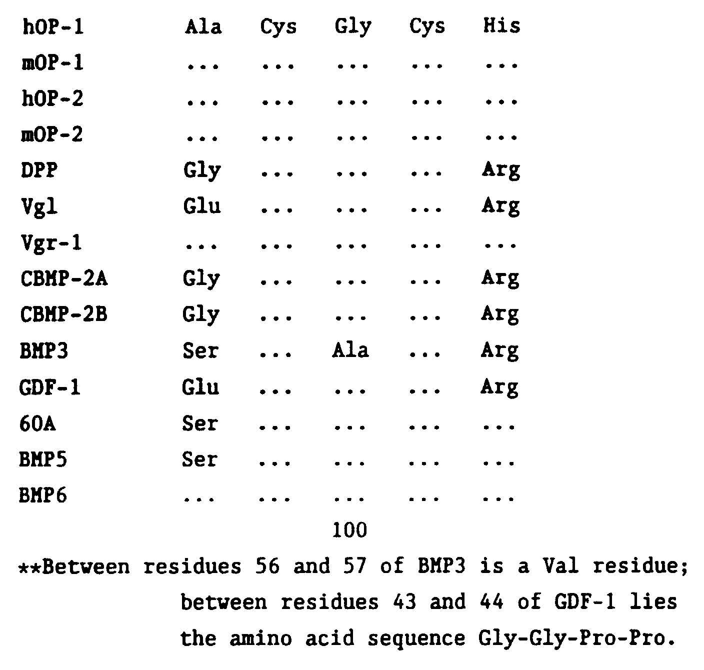

As is apparent from the foregoing amino acid sequence comparisons, significant amino acid changes can be made within the generic sequences while retaining the morphogenic activity. For example, while the GDF-1 protein sequence depicted in Table II shares only about 50% amino acid identity with the hOP1 sequence described therein, the GDF-1 sequence shares greater than 70% amino acid sequence homology (or "similarity") with the hOP1 sequence, where "homology" or "similarity" includes allowed conservative amino acid changes within the sequence as defined by Dayoff, et al., Atlas of Protein Sequence and Structure vol.5, supp.3, pp.345-362, (M.O. Dayoff, ed., Nat'l BioMed. Res. Fd'n, Washington D.C. 1979.)

The currently most preferred protein sequences useful as morphogens in this invention include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, in still another preferred aspect, the invention includes morphogens comprising species of polypeptide chains having the generic amino acid sequence referred to herein as "OPX", which defines the seven cysteine skeleton and accommodates the identities between the various identified mouse and human OP1 and OP2 proteins. OPX is presented in Seq. ID No. 29. As described therein, each Xaa at a given position independently is selected from the residues occurring at the corresponding position in the C-terminal sequence of mouse or human OP1 or OP2 (see Seq. ID Nos. 5-8 and/or Seq. ID Nos. 16-23).

### III. Formulations and Methods for Administration

### 1. Therapeutic Agent Considerations

The morphogens may be provided to the tooth root and/or tooth socket surface by any suitable means. Preferably, the morphogen, or a morphogen-stimulating agent, (collectively, the therapeutic agent) is provided directly to the tissue surface by topical administration. Alternatively, the therapeutic agent may be provided to the tissue by, for example, local injection. While not currently preferred, systemic injection also may be a viable administration route for certain applications, such as periodontal tissue maintenance in older adults, immuno-suppressed individuals, or others at chronic risk for periodontal tissue loss. A detailed description of considerations for systemic administration, including oral and parenteral administration, is disclosed, for example, in international application US92/07358 (WO93/04692), incorporated hereinabove by reference.

Where the therapeutic agent is provided directly to the tooth socket, the therapeutic agent may be provided to the socket surface as part of a biocompatible formulation that may be a liquid, gel or solid. The therapeutic agent further may be dispersed in and associated with a carrier capable of maintaining the morphogen at the administered locus. Useful formulations include viscous compositions. Biocompatible compositions that increase the viscosity of the formulation include glycerol, polyalkylene glycols such as polyethylene glycol, oils of vegetable origin, hydrogenated naphthalenes, and the like.

The formulation also may include an in vivo bioresorbable carrier material that acts as a controlled release delivery vehicle. Useful carriers may include biocompatible, preferably biodegradable structural components from, e.g., an extracellular matrix, such as collagen, laminin, hyaluronic acid, and the like, or polymeric materials, such as polylactic, polybutyric and polyglycolic acids. The carrier also may comprise an acellular tissue matrix, substantially depleted in nonstructural components, such as a demineralized, guanidine-extracted dentin, periodontal ligament or cementum matrix. Details for preparing such matrices are disclosed in international application US92/01968 (WO93/15323). Other useful controlled release carriers in which the therapeutic agent may be dispersed are described in U.S. Pat. Nos. 4,975,526 and 4,919,939, the disclosures of which are incorporated herein by reference.

Where the morphogen is to be provided to a tooth root surface, it may be formulated in a composition for controlled delivery as described above and applied topically to the tooth root surface as described below. Alternatively, or in addition, the therapeutic agent may be dispersed in a liquid formulation into which at least the tooth root surface is placed and the liquid lyophilized to adsorb the therapeutic agent onto the tooth surface.

Where the agent is administered to inhibit periodontal tissue loss and/or to regenerate periodontal tissue surrounding an implanted tooth, the agent may be provided to the area between the tooth and gum (gingiva) by injection or by topical application.

Where the morphogen is to be provided directly (e.g., locally, as by injection, e.g., to a periodontal or alveolar tissue site), the morphogen preferably comprises part of an aqueous solution which also may contain a carrier material. The solution is physiologically acceptable so that in addition to delivery of the desired morphogen to the patient, the solution does not otherwise adversely affect the patient's electrolyte and volume balance. The aqueous medium for the morphogen thus may comprise normal physiologic saline (0.85% NaCl, 0.15M), pH 7-7.4. The aqueous solution containing the morphogen can be made, for example, by dissolving the protein in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further may include 0.1-0.2% human serum albumin (HSA). The resultant solution preferably is vortexed extensively. If desired, a given morphogen may be made more soluble in the solution by association with a suitable molecule. For example, the pro form of the morphogenic protein comprises a species that is soluble in physiological solutions. In fact, the endogenous protein is thought to be transported (e.g., secreted and circulated) in this form. This soluble form of the protein may be obtained from the culture medium of morphogen-secreting mammalian cells. Alternatively, a soluble species may be formulated by complexing the mature dimer (or an active fragment thereof) with part or all of a pro domain. Other components, including various serum proteins, also may be useful. A more detailed description for formulating soluble morphogen complexes appears in Example 4, below.

Finally, the morphogens or morphogen-stimulating agents provided herein may be administered alone or in combination with other molecules, particularly symptom alleviating cofactors. Useful pharmaceutical cofactors include antiseptics, antibiotics, anaesthetics and analgesics. Preferred antiseptics for use in the present system include chlorhexidine and tibezonium iodide; preferred antibiotics include tetracyciine, aminoglycosides such as neomycin, gentamycin, kanamycin, tobramycin, netilmicin, siscmicin, amicamycin, their sulfates or other derivatives, macrolides such as erythromycin, its salts and other derivatives, spiramycin, josamicin or miocamicin, penicillins such as ampicillin, amoxicillin and the like, and cephalosporins, for example, cefaclor, cefadroxil, cefazolin, cefoperazone, cefotaxime, cephalothin, cefalexin, ceforanide, cefonicide or ceftriaxone. Preferred anaesthetics/analgesics include amide-type local anaesthetics such as lidocaine, mepivacaine, pyrrocaine, bupivacaine, prilocaine, etidocaine, or other widely used anaesthetics such as procaine. In the compositions of the invention, the cofactors do not include tetracycline or salicyclic acid.

Other cofactors include non-steroidal anti-inflammatory agents. However, the morphogens described herein themselves modulate the body's inflammatory/immune response to an initial tissue injury. Specifically, and as described in detail in international application US92/07358 (WO93/04692), in the presence of a morphogen, progenitor inflammatory effector cells induced to migrate to a site of tissue injury do not become significantly activated. Without being limited to any given theory, it is thought that, in the presence of the morphogen, damaged tissue is induced to undergo a recapitulation of tissue morphogenesis, where progenitor cells are induced to proliferate and differentiate in a tissue-specific manner, and new, functional, organized tissue is formed to replace the damaged or lost tissue, rather than disorganized, fibrous scar tissue.

The formulated compositions contain therapeutically effective amounts of the morphogen, e.g., amounts which provide appropriate concentrations of the morphogen to the tooth surface for a time sufficient to stimulate growth and development of periodontal tissues, including morphogenesis of periodontal ligament and/or cementum, and/or to substantially inhibit periodontal tissue loss.

As will be appreciated by those skilled in the art, the concentration of the compounds described in a therapeutic composition will vary depending upon a number of factors, including the biological efficacy of the selected morphogen, the chemical characteristics (e.g., hydrophobicity) of the compounds employed, the formulation of the compound excipients, the administration route, and the treatment envisioned. The preferred dosage to be administered also is likely to depend on such variables such as the condition of the tissues within the tooth socket, the size of the tooth or tooth socket, the length of time after tooth loss, extent of periodontal tissue loss and the overall health status of the particular patient. The amount of morphogen applied also will depend on the tooth size. In general, 0.1-1000 µg of morphogen are sufficient with 1-100 µg being preferable. For example, for a large tooth, e.g., an incisor or large molar, about 10-100 µg, and preferably 50 µg of morphogen, may be used to advantage; a medium tooth may be treated with approximately 5-50 µg , and preferably 25 µg; and a small tooth, with approximately 1-25, preferably 5-10 µg morphogen. No obvious morphogen induced pathological lesions are induced when mature morphogen (e.g., OP-1, 20 µg) is administered daily to normal growing rats for 21 consecutive days. Moreover, 10 µg systemic injections of morphogen (e.g., OP-1) injected daily for 10 days into normal newborn mice does not produce any gross abnormalities.

### 2. Tooth Preparation

Tooth loss may be repaired by implanting a viable tooth having a healthy root and pulp system or by implanting a tooth prosthesis. The prosthesis may be a tooth from which the root has been removed and replaced with a biocompatible, biologically inert material, e.g., as typically is replaced in a root canal procedure, or may be a completely synthetic prosthesis coated, for example, with a porous material to enhance tooth integration in the tooth socket. Useful prosthesis coating materials include collagen fibers, ceramics and metals, such as titanium oxide. The root of the implanted tooth first may be partially demineralized as described below. Alternatively, a clean, mineralized natural tooth or dentin-containing prosthetic tooth may be implanted.

A tooth to be implanted first is obtained, e.g., by loss or removal of a natural tooth from the tooth socket, e.g., using standard tooth extraction means well known to one skilled in the dentistry art. Alternatively, an allogenic tooth may be obtained from a tooth bank. The natural, mineralized tooth or tooth root may be coated as is with a morphogen and implanted as described below. Alternatively, the mineralized, natural tooth root surface first may be scored or scraped to expose dentin tissue beneath the enamel. Natural, mineralized teeth also may be treated briefly with an acidic solution (e.g., sodium citrate, about pH 3.5) to remove a thin external layer, e.g., about 1-5 cells in thickness from at least the root surface. Preferred treatment times are from about 0.5 to 5 minutes. The treated teeth preferably then are washed, dried and coated with morphogen as described below. Alternatively, the tooth root portion may be at least partially demineralized according to any conventional procedure prior to implantation. A currently preferred demineralization method is to soak the tooth in a demineralizing solution for a length of time sufficient to remove at least some mineral components from the tooth. For example, at least the root portion of the tooth may be placed in a volume, e.g., 0.025-1 liter of a demineralizing agent such as hydrochloric acid (HCl) at a cool temperature for a time sufficient to achieve partial demineralization, e.g., 0.5-0.6 M HCl at 4°C for a prescribed number of minutes (e.g., preferably within the range of about 10-200 minutes.) Essentially complete demineralization may be achieved by acid exposure for 1-7 days. If desired, several changes of the demineralizing agent may be performed. The partially demineralized tooth will be of the same shape as prior to demineralization, but will weigh less due to the absence of the mineral content. The tooth then may be dried by lyophilization.

The tooth or tooth prosthesis may be treated with morphogenic protein as follows. The morphogen may be applied to the tooth or tooth prosthesis root surface by any means known in the art for adsorbing a protein to a surface. A currently preferred method is to suspend the morphogen in a small volume sufficient to cover the tooth surface, e.g., 200-300µl, freeze the tooth in solution, and then lyophilize the frozen liquid. A currently preferred solution is ethanol (e.g. 50%) or acetonitrile/trifluroactic acid (TFA), other solutions include HCL/TFA, buffered saline, and the like. Alternatively, or in addition, the therapeutic agent may be provided to the tooth root surface dispersed in a suitable carrier material as described above. Similarly, and as described above, the therapeutic agent may be provided to the tooth socket surface and the tooth to be implanted embedded in the morphogen composition on the socket surface. Also as described above, the morphogen may be provided to the tooth root surface in admixture with one or more cofactors.

The tooth then is implanted into a fresh or surgically prepared tooth socket. A surgically prepared surface is prepared by extracting the tooth and removing any scar or other undesired fibrous tissue built up in the socket by standard mechanical and/or chemical procedures well known on the surgical and dental arts. The tooth then is implanted in the site using standard dental and surgical procedures.

The implanted tooth is allowed to grow in the prepared socket for a time sufficient to allow the periodontium to regenerate, e.g., one to several months. The integrity and health of the integrated tooth then may be assessed by a dentist by radiography and visual examination.

For experimental purposes, the integration of an implanted tooth following morphogen treatment can be assessed for integrity and health by removing the entire mandibular area, including the tooth socket and tooth, and examining cross sections of the mandibular area. 5-10 µm cross sections may be prepared for histological evaluation by standard histology procedures, e.g., fixing tissue with formalin, preparing sections for slides and staining with eosin and hematoxylin. The growth and integrity of hard tissues, such as bone, cementum and dentin, also can evaluated radiographically.

Finally, as described in Example 2 below, the morphogens of this invention also induce dentin tissue morphogenesis when provided to an area of lost or damaged dentin. Accordingly, using the procedures described herein and in international application (US92/01968 (WO92/15323), the morphogen described herein also may be used to repair and regenerate damaged and/or lost dentin tissue in an implanted tooth.

### IV. Examples

### Example 1. Experimental Regeneration of Peridontium in a Dog Model

The following experiment demonstrates successful integration of an implanted demineralized, protein-extracted morphogen-treated tooth in a mammal. Premolar teeth were extracted from a dog and divided into three experimental groups: (a) demineralized teeth; (b) demineralized and guanidine extracted teeth; and (c) demineralized, guanidine extracted, and morphogen-treated teeth. Teeth from each group were tested in "fresh" sockets, e.g., tooth sockets from which the teeth had just been removed, as well as surgically prepared sockets, e.g., sockets from which teeth had been extracted 2 months previously and in which scar tissue had formed. These "healed" sockets were surgically prepared for tooth implantation by removing (e.g., by scraping) scar tissue build up to reveal fresh alveolar bone.

The teeth from all three groups were completely demineralized by placing them in 4 liters of 0.5 M HCl at 4°C for 5 days. The 0.5 M HCl solution was changed every 24 hours during the 5 day period. The teeth then were washed in 4 liters of deionized water at 4°C for 5 days. The water solution was also changed every 24 hours during the 5 day period. Teeth from group (a) then were lyophilized until dry and set aside and maintained at 4°C until ready for use.

Teeth from groups (b) and (c) then were protein-extracted by multiple extractions in 6 M guanidine Hcl, followed by washes with distilled water. Specifically, the teeth were placed in in 2-4 liters of 6 M guanidine-HCl/Tris HCl pH 7.0 at 4°C for 72 hours; then washed and further extracted in 200 ml of the guanidine-HCl solution for 4 hours. The teeth were washed again with 4 liters of distilled dH₂O at 4°C for 48 hours, and 4 liters of dH₂O for an additional 12 hours with 3 changes of dH₂O. The teeth were then lyophilized until dry. Teeth from group (b) were then set aside and maintained at 4°C until ready for use.

Teeth from group (c) then were treated with the morphogen OP-1 as follows. 1.15 mg of OP-1 was resuspended in 4 ml of 47.5% ethanol/0.09% trifluoroacetic acid (TFA). The concentration was determined to be 0.273 mg/ml. Approximately 50 µg of OP-1 (183 µl of the OP-1 solution) was dispensed into an eppendorf tube, and the total volume brought to 300 µl of 47.5% ethanol/0.09% TFA. Each tooth then was placed in an eppendorf tube such that the OP-1 solution just covered the tooth. The tube was placed at ⁻70°C until the OP-1 solution was frozen, and lyophilized until dry. During lyophilization, care was taken to keep the tube cold. Approximately 50-70% of the OP-1 can be expected to remain in or on the tooth after lyophilization.

The teeth from each of groups (a), (b), and (c) were then implanted into a freshly prepared tooth socket or surgically prepared socket using standard dental surgery procedures known in the art.

The implanted teeth in all three groups were allowed to remain in the socket for two months. The dog then was sacrificed, the mandible cross-sectioned and x-rayed, and histology performed. The results are described below and follows.

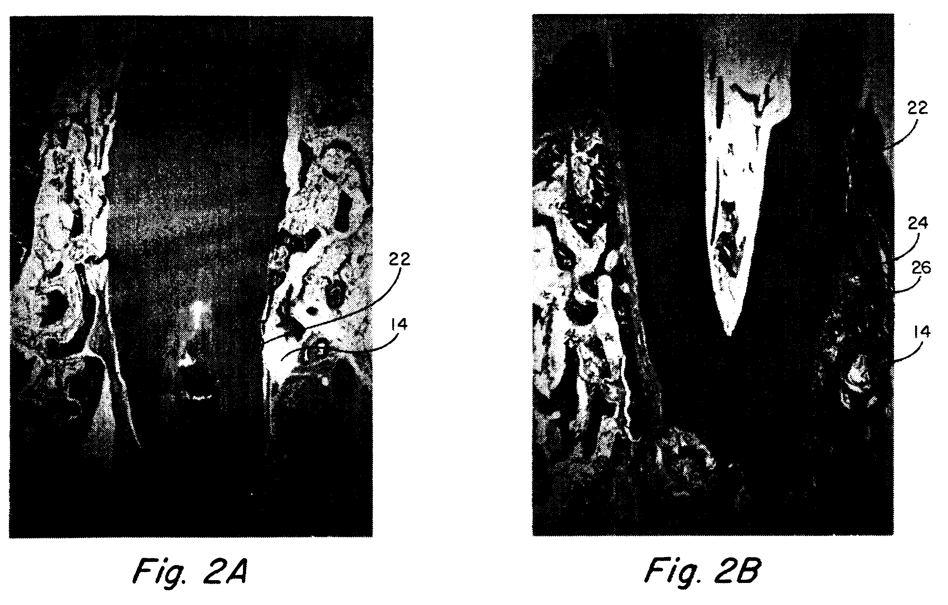

Ankylosis formed in the group (a) implants, where demineralized tooth matrix was implanted alone. Cross-sections of the group (a) mandible revealed that the demineralized tooth was surrounded by bone directly attaching to the root or dentin surface. In addition, there was little new tissue growth between the tooth and the bone. Representative histology is illustrated in the photomicrograph of Fig. 2A where bone tissue 14 grows directly into dental tissue 22 in the implanted tooth.

In the group (b) implants, cross-sections revealed formation of unorganized fibrous tissue around the implanted demineralized, guanidine extracted tooth. The periodontal ligament was loose and disorganized, as was the surrounding bony tissue. Examination of the tooth root surface where cementum matrix normally appears revealed resorption of cementum in the upper coronal surface of the tooth. Histological sections also revealed inflammation as evidenced by the presence of macrophages.

As is evident in Fig. 2b, group (c) implant cross-sections revealed formation of newly formed, organized cementum 24 and periodontal ligament tissue 26 around the morphogen-treated tooth matrix, and growth of new bone connecting the newly formed periodontium to the mandible. The tooth was firmly anchored in the tooth socket. The tissues surrounding the tooth, i.e., the newly-formed cementum growing perpendicular to the newly-formed periodontal ligament, and the alveolar bony tissue, all were healthy and organized much as the tooth and tooth socket shown schematically in Fig. 1. The newly-formed cementum comprised immature columnar cell layers which were beginning to flatten into mature cementoblasts, and the newly-formed periodontal ligament comprised a thick layer of tissue to anchor and cushion the tooth within the tooth socket.

The results of this experiment demonstrate that morphogens promote tooth integration into a tooth socket, and induce morphogenesis of periodontium, including morphogenesis of the regeneration and formation of the periodontium, new cementum and periodontal ligament.

Without being limited to any particular theory, the morphogens may act in the tooth socket environment by inducing a differentiation of primary fibroblasts on the alveolar surface to differentiate into cementoblasts which then induct other primary fibroblasts to form periodontal ligament.

### Example 2. Morphogen-Induced Dentinogenesis

The examples presented below demonstrate the efficacy of morphogens in inducing dentin tissue morphogenesis in an animal model. Further details of the first experiment and the implications of this biological activity of morphogens are disclosed in international application (US92/01968 (WO92/15323).

To date, the unpredictable response of dental pulp tissue to injury is a basic clinical problem in dentistry. Cynomolgus monkeys were chosen as primate models for the reparative dentine/pulp capping examples described below.

Using standard dental surgical procedures, small areas (e.g., 2mm) of dental pulps were surgically exposed by removing the enamel and dentin immediately above the pulp (by drilling) of sample teeth, performing a partial amputation of the coronal pulp tissue, inducing hemostasis, application of the pulp treatment, and sealing and filling the cavity by standard procedures.

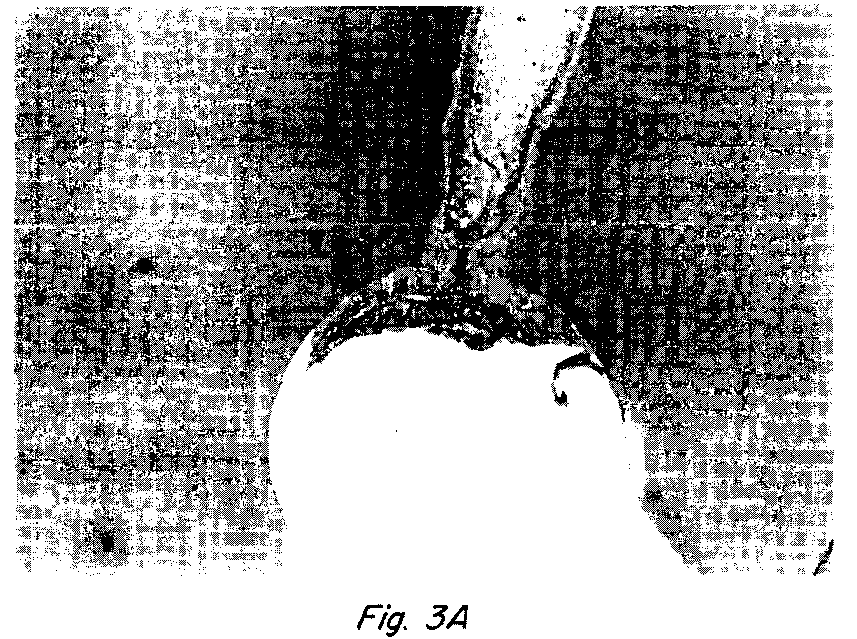

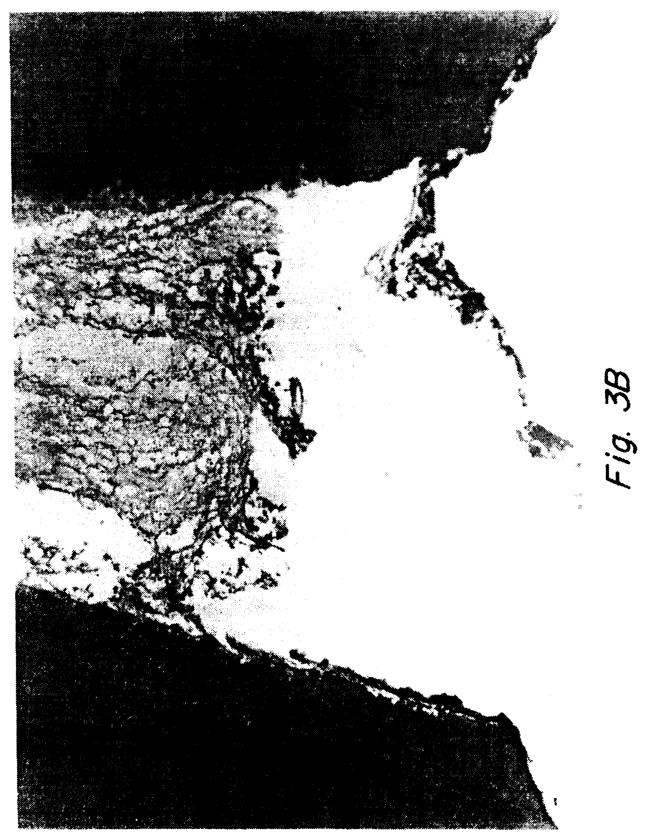

Pulp treatments used were: OP1 dispersed in a carrier matrix; carrier matrix alone and no treatment. Twelve teeth per animal (four for each treatment) were prepared, and two animals were used. At four weeks, teeth were extracted and processed histologically for analysis of dentin formation, and/or ground to analyze dentin mineralization. Morphogen treatment produced dramatic effects: Control treatments with carrier alone or with no treatment (PBS) showed little or no reparation of the lost tissue. By contrast, morphogen-treated teeth showed significant dentin tissue formation in the area where dentin tissue had been surgically removed. The experimental results show that morphogen treatment reliably induced formation of reparative or osteodentin bridges on surgically exposed healthy dental pulps. See, for example, Fig. 3A, where OP1 dispersed in a carrier (demineralized, guanidine-extracted bone collagen matrix prepared as described in U.S. Patent No. 4,975,526) constituted the pulp treatment. As is evident from the micrograph new dentine formation effectively bridges or "caps" the surgically exposed dental pulp, maintaining the integrity and viability of the pulp tissue. By contrast, pulps treated with carrier matrix alone, or not treated, failed to form reparative dentin. See, for example Fig. 3B where carrier alone (demineralized, guanidine-extracted bone collagen matrix prepared as described in U.S. Patent No. 4,975,526) constituted the pulp treatment. As is evident from the micrograph, minimal reparative dentin formed, insufficient to bridge the exposed pulp tissue. Without further treatment such exposed, unprotected pulp tissue will become infected and die.

In a supplemental experiment, a range of morphogen concentrations were tested. In all cases, human OP-1, prepared as described in Sampath et al. (1992) J. Biol. Chem. 267: 20352-20362, was the morphogen tested, and bone collagen matrix, prepared as described in U.S. Patent No. 4,975,526 was the carrier material/delivery vehicle ("CM"). Briefly, cortical bone powder was prepared from freshly obtained bovine femurs. The epiphyses, adherent flesh and marrow were removed and residual lipids extracted with hexane, isopropanol, and ethyl ether. The resulting material was ground and sieved to a described particle size of 75-425 µm. The cortical bone powder then was demineralized in acid, and soluble proteins extracted with guanidine hydrochloride. The demineralized, extracted bone powder then was subjected to a thermal acid treatment, washed with water, and lyophilized. The final dry powder was sieved to remove particles >425 µm and stored at 4°C.

The hOP-1/CM samples were prepared by combining hOP-1 with the CM and drying under vacuum. The batch used in these experiments contained 2.5µg hOP-1/mg CM. Prior to implant the sample was moistened with a sterile aqueous solution, preferably saline, to form a paste-like substance. CM controls were prepared using the same procedure, omitting the morphogen. The samples were stored at -20°C until used.

The pulp capping experiments were conducted using 4 adult female non-human primates (Macaca fasicularis) of approximately 4 kg each. The animals were sedated using standard procedures, e.g., with ketamine (15 mg/kg body wt.) and acepromazine (0.55 mg/kg body wt.) supplemented with local intraoral infiltration anesthesia.

Thirty premolar and molar teeth in four animals were isolated by rubber dam and the pulps exposed using standard dentistry procedures, e.g., using sterile high speed rotary cutting instruments with water spray coolant. The pulp exposures made were approximately 1-1.5 by 2-2.5 mm. Partial hemostasis was achieved with sterile cotton pellets but the teeth were not dried extensively prior to treatment. The exposed pulps were treated with: hOP-1/CM (2.5 µg hOP-1/mg CM) at 1.5, 3.0 or 6.0 mg/tooth; or one of three controls: Ca(OH)₂ paste, a standard pulp capping agent used in the art ("Dycal", L.D. Caulk, Milford, DE); CM alone, 3.0 mg/tooth; or no treatment material. The teeth then were sealed with a standard adhesive, e.g., Temp-Bond NE™ (Kerr U.S.A., Romulus, MI). The teeth were allowed to heal for six weeks. No changes in behavior were noted by any of the animals during the healing period.

The animals were sacrificed six weeks following surgery and prepared for histomorphometric analysis using standard procedures. For example, teeth were fixed by immersion in 10% formalin in phosphate buffered saline (pH 7.2) and decalcified in formic acid/sodium citrate at room temperature for 6-8 days. The specimens were processed, imbedded in paraffin, serial sectioned (5 µm) and stained.

In all teeth treated with hOP-1/CM and for all OP1 concentrations tested, reparative dentine sufficient to bridge the surgically created gap that exposed the underlying pulp tissue was formed. As in the previous experiment, the morphogen/CM device was resorbed in the healed teeth, and replaced with reparative dentine, fully integrated with the cut dentine at the exposure site. Also as in the previous experiment, the pulp tissue beneath the cap appeared normal, with intact odontoblasts lining the pulp chamber. The amount of new dentine tissue increased as the amount of OP-1 provided in a sample was increased, indicating that the amount of reparative dentine formed was related to the mass of OP1/CM administered. Pulp treatments using CM alone or no treatment did not succeed in bridging the exposure site and in several cases resulted in necrotic pulp tissue. Treatments using Ca(OH)₂ succeeded in bridging the gap, but the paste remained and the bridge created lies within the pulp chamber itself.

### Example 3. Screening Assay for Candidate Compounds which Alter Endogenous Morphogen Levels

Candidate compound(s) which may be administered to affect the level of a given morphogen may be found using the following screening assay, in which the level of morphogen production by a cell type which produces measurable levels of the morphogen is determined with and without incubating the cell in culture with the compound, in order to assess the effects of the compound on the cell. This can be accomplished by detection of the morphogen either at the protein or RNA level. A more detailed description also may be found in international application US92/07359 (WO93/05172), incorporated hereinabove by reference.

### 3.1 Growth of Cells in Culture

Cell cultures of kidney, adrenals, urinary bladder, brain, or other organs, may be prepared as described widely in the literature. For example, kidneys may be explanted from neonatal or new born or young or adult rodents (mouse or rat) and used in organ culture as whole or sliced (1-4 mm) tissues. Primary tissue cultures and established cell lines, also derived from kidney, adrenals, urinary, bladder, brain, mammary, or other tissues may be established in multiweii plates (6 well or 24 well) according to conventional cell culture techniques, and are cultured in the absence or presence of serum for a period of time (1-7 days). Cells may be cultured, for example, in Dulbecco's Modified Eagle medium (Gibco, Long Island, NY) containing serum (e.g., fetal calf serum at 1%-10%, Gibco) or in serum-deprived medium, as desired, or in defined medium (e.g., containing insulin, transferrin, glucose, albumin, or other growth factors).

Samples for testing the level of morphogen production includes culture supernatants or cell lysates, collected periodically and evaluated for OP-1 production by immunoblot analysis (Sambrook et al., eds., 1989, Molecular Cloning, Cold Spring Harbor Press, Cold Spring Harbor, NY), or a portion of the cell culture itself, collected periodically and used to prepare polyA+ RNA for RNA analysis. To monitor de novo OP-1 synthesis, some cultures are labeled according to conventional procedures with an ³⁵S-methionine/³⁵S-cysteine mixture for 6-24 hours and then evaluated for OP-1 synthesis by conventional immunoprecipitation methods.

### 3.2 Determination of Level of Morphogenic Protein

In order to quantitate the production of a morphogenic protein by a cell type, an immunoassay may be performed to detect the morphogen using a polyclonal or monoclonal antibody specific for that protein. For example, OP-1 may be detected using a polyclonal antibody specific for OP-1 in an ELISA, as follows.

1 µg/100 µl of affinity-purified polyclonal rabbit IgG specific for OP-1 is added to each well of a 96-well plate and incubated at 37°C for an hour. The wells are washed four times with 0.167M sodium borate buffer with 0.15 M NaCl (BSB), pH 8.2, containing 0.1% Tween 20. To minimize non-specific binding, the wells are blocked by filling completely with 1% bovine serum albumin (BSA) in BSB and incubating for 1 hour at 37°C. The wells are then washed four times with BSB containing 0.1% Tween 20. A 100 µl aliquot of an appropriate dilution of each of the test samples of cell culture supernatant is added to each well in triplicate and incubated at 37°C for 30 min. After incubation, 100 µl biotinylated rabbit anti-OP-1 serum (stock solution is about 1 mg/ml and diluted 1:400 in BSB containing 1% BSA before use) is added to each well and incubated at 37°C for 30 min. The wells are then washed four times with BSB containing 0.1% Tween 20. 100 µl strepavidin-alkaline (Southern Biotechnology Associates, Inc. Birmingham, Alabama, diluted 1:2000 in BSB containing 0.1% Tween 20 before use) is added to each well and incubated at 37°C for 30 min. The plates are washed four times with 0.5M Tris buffered Saline (TBS), pH 7.2. 50µl substrate (ELISA Amplification System Kit, Life Technologies, Inc., Bethesda, MD) is added to each well incubated at room temperature for 15 min. Then, 50 µl amplifier (from the same amplification system kit) is added and incubated for another 15 min at room temperature. The reaction is stopped by the addition of 50 µl 0.3 M sulphuric acid. The OD at 490 nm of the solution in each well is recorded. To quantitate OP-1 in culture media, a OP-1 standard curve is performed in parallel with the test samples.

Polyclonal antibody may be prepared as follows. Each rabbit is given a primary immunization of 100 ug/500 µl E. coli produced OP-1 monomer (amino acids 328-431 in SEQ ID NO:5) in 0.1% SDS mixed with 500 µl Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Additional boosts and test bleeds are performed at monthly intervals until antibody against OP-1 is detected in the serum using an ELISA assay. Then, the rabbit is boosted with 100 µg of antigen and bled (15 ml per bleed) at days seven and ten after boosting.

Monoclonal antibody specific for a given morphogen may be prepared as follows. A mouse is given two injections of E. coli produced OP-1 monomer. The first injection contains 100µg of OP-1 in complete Freund's adjuvant and is given subcutaneously. The second injection contains 50 µg of OP-1 in incomplete adjuvant and is given intraperitoneally. The mouse then receives a total of 230 µg of OP-1 (amino acids 307-431 in SEQ ID NO:5) in four intraperitoneal injections at various times over an eight month period. One week prior to fusion, the mouse is boosted intraperitoneally with 100 µg of OP-1 (307-431) and 30 µg of the N-terminal peptide (Ser₂₉₃-Asn₃₀₉-Cys) conjugated through the added cysteine to bovine serum albumin with SMCC crosslinking agent. This boost was repeated five days (IP), four days (IP), three days (IP) and one day (IV) prior to fusion. The mouse spleen cells are then fused to myeloma (e.g., 653) cells at a ratio of 1:1 using PEG 1500 (Boeringer Mannheim), and the cell fusion is plated and screened for OP-1-specific antibodies using OP-1 (307-431) as antigen. The cell fusion and monoclonal screening then are according to standard procedures well described in standard texts widely available in the art.

### Example 4. Soluble Morphogen Complexes

A currently preferred form of the morphogen useful in therapeutic formulations for systemic administration, having improved solubility in aqueous solutions and consisting essentially of amino acids, is a dimeric morphogenic protein comprising at least the 100 amino acid peptide sequence having the pattern of seven or more cysteine residues characteristic of the morphogen family complexed with a peptide comprising part or all of a pro region of a member of the morphogen family, or an allelic, species or other sequence variant thereof. Preferably, the dimeric morphogenic protein is complexed with two peptides. Also, the dimeric morphogenic protein preferably is noncovalently complexed with the pro region peptide or peptides. The pro region peptides also preferably comprise at least the N-terminal eighteen amino acids that define a given morphogen pro region. In a most preferred embodiment, peptides defining substantially the full length pro region are used.

Other soluble forms of morphogens include dimers of the uncleaved pro forms of these proteins, as well as "hemi-dimers" wherein one subunit of the dimer is an uncleaved pro form of the protein, and the other subunit comprises the mature form of the protein, including truncated forms thereof, preferably noncovalently associated with a cleaved pro domain peptide.

As described above, useful pro domains include the full length pro regions, as well as various truncated forms hereof, particularly truncated forms cleaved at proteolytic Arg-Xaa-Xaa-Arg cleavage sites. For example, in OP-1, possible pro sequences include sequences defined by residues 30-292 (full length form); 48-292; and 158-292. Soluble OP-1 complex stability is enhanced when the pro region comprises the full length form rather than a truncated form, such as the 48-292 truncated form, in that residues 30-47 show sequence homology to the N-terminal portions of other morphogens, and are believed to have particular utility in enhancing complex stability for all morphogens. Accordingly, currently preferred pro sequences are those encoding the full length form of the pro region for a given morphogen. Other pro sequences contemplated to have utility include biosynthetic pro sequences, particularly those that incorporate a sequence derived from the N-terminal portion of one or more morphogen pro sequences.

As will be appreciated by those having ordinary skill in the art, useful sequences encoding the pro region may be obtained from genetic sequences encoding known morphogens. Alternatively, chimeric pro regions can be constructed from the sequences of one or more known morphogens. Still another option is to create a synthetic sequence variant of one or more known pro region sequences.

In another preferred aspect, useful pro region peptides include polypeptide chains comprising an amino acid sequence encoded by a nucleic acid that hybridizes under stringent conditions with a DNA or RNA sequence encoding at least the N-terminal eighteen amino acids of the pro region sequence for OP1 or OP2, e.g., nucleotides 136-192 and 152-211 of Seq. ID No. 16 and 20, respectively.

### 4.1 Isolation of Soluble morphogen complex from conditioned media or body fluid

Morphogens are expressed from mammalian cells as soluble complexes. Typically, however the complex is disassociated during purification, generally by exposure to denaturants often added to the purification solutions, such as detergents, alcohols, organic solvents, chaotropic agents and compounds added to reduce the pH of the solution. Provided below is a currently preferred protocol for purifying the soluble proteins from conditioned media (or, optionally, a body fluid such as serum, cerebro-spinal or peritoneal fluid), under non-denaturing conditions. The method is rapid, reproducible and yields isolated soluble morphogen complexes in substantially pure form.

Soluble morphogen complexes can be isolated from conditioned media using a simple, three step chromatographic protocol performed in the absence of denaturants. The protocol involves running the media (or body fluid) over an affinity column, followed by ion exchange and gel filtration chromatographies. The affinity column described below is a Zn-IMAC column. The present protocol has general applicability to the purification of a variety of morphogens, all of which are anticipated to be isolatable using only minor modifications of the protocol described below. An alternative protocol also envisioned to have utility an immunoaffinity column, created using standard procedures and, for example, using antibody specific for a given morphogen pro domain (complexed, for example, to a protein A-conjugated Sepharose column.) Protocols for developing immunoaffinity columns are well described in the art, (see, for example, Guide to Protein Purification, M. Deutscher, ed., Academic Press, San Diego, 1990, particularly sections VII and XI.)

In this experiment OP-1 was expressed in mammalian CHO (chinese hamster ovary) cells as described in the art (see, for example, international application US90/05903 (WO91/05802).) The CHO cell conditioned media containing 0.5% FBS was initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC step separates the soluble OP-1 from the bulk of the contaminating serum proteins that elute in the flow through and 35 mM imidazole wash fractions. The Zn-IMAC purified soluble OP-1 is next applied to an S-Sepharose cation-exchange column equilibrated in 20 mM NaPO₄ (pH 7.0) with 50 mM NaCl. This S-Sepharose step serves to further purify and concentrate the soluble OP-1 complex in preparation for the following gel filtration step. The protein was applied to a Sephacryl S-200HR column equilibrated in TBS. Using substantially the same protocol, soluble morphogens also may be isolated from one or more body fluids, including serum, cerebro-spinal fluid or peritoneal fluid.

IMAC was performed using Chelating-Sepharose (Pharmacia) that had been charged with three column volumes of 0.2 M ZnSO₄. The conditioned media was titrated to pH 7.0 and applied directly to the ZN-IMAC resin equilibrated in 20 mM HEPES (pH 7.0) with 500 mM NaCl. The Zn-IMAC resin was loaded with 80 mL of starting conditioned media per mL of resin. After loading, the column was washed with equilibration buffer and most of the contaminating proteins were eluted with 35 mM imidazole (pH 7.0) in equilibration buffer. The soluble OP-1 complex then is eluted with 50 mM imidazole (pH 8.0) in 20 mM HEPES and 500 mM NaCl.

The 50 mM imidazole eluate containing the soluble OP-1 complex was diluted with nine volumes of 20 mM NaPO₄ (pH 7.0) and applied to an S-Sepharose (Pharmacia) column equilibrated in 20 mM NaPO₄ (pH 7.0) with 50 mM NaCl. The S-Sepharose resin was loaded with an equivalent of 800 mL of starting conditioned media per mL of resin. After loading the S-Sepharose column was washed with equilibration buffer and eluted with 100 mM NaCl followed by 300 mM and 500 mM NaCl in 20 mM NaPO₄ (pH 7.0). The 300 mM NaCl pool was further purified using gel filtration chromatography. Fifty mls of the 300 mm NaCl eluate was applied to a 5.0 X 90 cm Sephacryl S-200HR (Pharmacia) equilibrated in Tris buffered saline (TBS), 50 mM Tris, 150 mM NaCl (pH 7.4). The column was eluted at a flow rate of 5 mL/minute collecting 10 mL fractions. The apparent molecular of the soluble OP-1 was determined by comparison to protein molecular weight standards (alcohol dehydrogenase (ADH, 150 kDa), bovine serum albumin (BSA, 68 kDa), carbonic anhydrase (CA, 30 kDa) and cytochrome C (cyt C, 12.5 kDa). The purity of the S-200 column fractions was determined by separation on standard 15% polyacrylamide SDS gels stained with coomassie blue. The identity of the mature OP-1 and the pro-domain was determined by N-terminal sequence analysis after separation of the mature OP-1 from the pro-domain using standard reverse phase C18 HPLC.

The soluble OP-1 complex elutes with an apparent molecular weight of 110 kDa. This agrees well with the predicted composition of the soluble OP-1 complex with one mature OP-1 dimer (35-36 kDa) associated with two pro-domains (39 kDa each). Purity of the final complex can be verified by running the appropriate fraction in a reduced 15% polyacrylamide gel.

The complex components can be verified by running the complex-containing fraction from the S-200 or S-200HR columns over a reverse phase C18 HPLC column and eluting in an acetonitrile gradient (in 0.1% TFA), using standard procedures. The complex is dissociated by this step, and the pro domain and mature species elute as separate species. These separate species then can be subjected to N-terminal sequencing using standard procedures (see, for example, Guide to Protein Purification, M. Deutscher, ed., Academic Press, San Diego, 1990, particularly pp. 602-613), and the identity of the isolated 36kD, 39kDa proteins confirmed as mature morphogen and isolated, cieaved pro domain, respectively. N-terminal sequencing of the isolated pro domain from mammalian cell produced OP-1 revealed 2 forms of the pro region, the intact form (beginning at residue 30 of Seq. ID No. 16) and a truncated form, (beginning at residue 48 of Seq. ID No. 16.) N-terminal sequencing of the polypeptide subunit of the isolated mature species reveals a range of N-termini for the mature sequence, beginning at residues 293, 300, 313, 315, 316, and 318, of Seq. ID No. 16, all of which are active as demonstrated by the standard bone induction assay.

### 4.2 In Vitro Soluble Morphogen Complex Formation

As an alternative to purifying soluble complexes from culture media or a body fluid, soluble complexes may be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea of GuHCl, which then preferably can be diluted into a physiological buffer. Protein purification/renaturing procedures and considerations are well described in the art, and details for developing a suitable renaturing protocol readily can be determined by one having ordinary skill in the art. One useful text one the subject is Guide to Protein Purification, M. Deutscher, ed., Academic Press, San Diego, 1990, particularly section V. Complex formation also may be aided by addition of one or more chaperone proteins.

### 4.3 Stability of Soluble Morphogen Complexes

The stability of the highly purified soluble morphogen complex in a physiological buffer, e.g., tris-buffered saline (TBS) and phosphate-buffered saline (PBS), can be enhanced by any of a number of means. Currently preferred is by means of a pro region that comprises at least the first 18 amino acids of the pro sequence (e.g., residues 30-47 of Seq. ID NO. 16 for OP-1), and preferably is the full length pro region. Residues 30-47 show sequence homology to the N-terminal portion of other morphogens and are believed to have particular utility in enhancing complex stability for all morphogens. Other useful means for enhancing the stability of soluble morphogen complexes include three classes of additives. These additives include basic amino acids (e.g., L-arginine, lysine and betaine); nonionic detergents (e.g., Tween 80 or NonIdet P-120); and carrier proteins (e.g., serum albumin and casein). Useful concentrations of these additives include 1-100 mM, preferably 10-70 mM, including 50 mM, basic amino acid;, 0.01-1.0%, preferably 0.05-0.2%, including 0.1% (v/v) nonionic detergent;, and 0.01-1.0%, preferably 0.05-0.2%, including 0.1% (w/v) carrier protein.

## Claims

1. A composition for use in therapy (e.g. for inhibiting loss of, or stimulating regeneration of, periodontal tissue in a mammal), said composition comprising a therapeutic concentration of a morphogen in association with a cofactor that mitigates symptoms associated with periodontal tissue damage selected from antiseptics, antibiotics with the proviso that tetracycline is excluded, anesthetics non-steroidal anti-inflammatory agents with the proviso that salicyclic acid is excluded and analgesics with the proviso that salicyclic acid is excluded said concentration of morphogen being sufficient to induce periodontal tissue morphogenesis.

2. The composition of claim 1 wherein
(I) said morphogen is dispersed in an acellular matrix, for example an acellular matrix derived from dentin, bone, periodontal ligament or cementum tissue, and/or
(II) said composition comprises a solution of high viscosity.

3. The composition of claims 1 or 2 wherein said morphogen is a dimeric protein comprising a pair of polypeptides, the amino acid sequence of each of which comprises:
(a) a sequence sharing at least 70% homology (e.g. at least 80% homology) with the C-terminal seven cysteine domain of human OP-1, residues 38-139 Seq. ID No. 5 or greater than 60% identity (e.g. greater than 65 identity) with the C-terminal six cysteine domain of human OP-1, residues 43-139 of Seq. ID No.5; or
(b) a sequence defined by Generic Sequence 6, Seq. ID No. 31.

4. The composition of claim 3 wherein the amino acid sequences of at least one of said morphogen polypeptides comprises a sequence selected from the C-terminal seven cysteine domain of human OP-1, residues 38-139 of Seq. ID No. 5, and allelic, species and biosynthetic variants thereof.

5. The composition of claim 3 wherein the amino acid sequences of said morphogen polypeptides comprise a sequence defined by OPX, Seq. ID No. 29.

6. The composition of any one of claims 1-5 wherein said morphogen in complexed with at least one prodomain peptide selected from the prodomains of members of the morphogen family, including allelic, species and biosynthetic sequence variants thereof, said prodomain peptide further being selected from the full length and proteolytic fragment peptides of said prodomains.

7. Use of a morphogen for the manufacture of a pharmaceutical for:
(a) inducing mammalian periodontal tissue morphogenesis, or
(b) the treatment of periodontal disease (e.g. for inducing periodontal tissue morphogenesis to inhibit tissue loss in periodontal disease), or
(c) inducing periodontal tissue morphogenesis to regenerate lost, damaged or nonviable periodontal tissue, or
(d) enhancing integration of a tooth in a mammalian tooth socket, or
(e) inhibiting periodontal tissue damage and tooth loss associated with periodontal and other gum diseases, or
(f) enhancing integration of a tooth (e.g. an implanted, allogenic and/or autologous tooth) in a mammalian tooth socket (e.g. a tooth socket comprising lost, damaged or nonviable periodontal tissue) comprising the step of providing a therapeutically effective concentration of the morphogen to the tooth socket surface or to the exterior tooth root surface, said concentration being sufficient to induce periodontal tissue morphogenesis on said surface, or (g) preparing a tooth (e.g. a prosthetic, allogenic and/or autologous tooth) or a mammalian tooth socket for implantation of said tooth into said socket, said socket being significantly reduced in viable periodontal tissue, comprising the steps of: (i) preparing said tooth socket to receive said tooth by exposing a surface of fresh alveolar bone therein and (ii) providing a therapeutically effective concentration of a morphogen to a surface selected from the exterior surface of the root of said tooth (which is e.g. partially demineralized) and the exposed surface of fresh alveolar bone in said prepared socket, said concentration being sufficient to induce periodontal tissue morphogenesis on said surface; or
(h) implanting a tooth (e.g. a prosthetic, allogenic and/or autologous tooth) in a mammalian tooth socket, said socket being significantly reduced in viable periodontal tissue, the method comprising the steps of: (i) preparing said tooth socket to receive said tooth by exposing a fresh surface of alveolar bone therein; and (ii) providing a therapeutically effective concentration of a morphogen to a surface selected from the exterior surface of the root of said tooth (which is e.g. partially demineralized) and the exposed surface of fresh alveolar bone in said prepared socket, said concentration being sufficient to induce periodontal tissue morphogenesis on said surface; and (iii) implanting said tooth in said prepared socket; or
(i) regenerating periodontal tissue in a mammalian tooth socket, the method comprising the step of providing a therapeutically effective concentration of a morphogen to said tooth socket, said concentration being sufficient to induce periodontal tissue morphogenesis therein; or
(j) inhibiting the tissue damage associated with periodontal disease, the method comprising the step of providing a therapeutically effective concentration of a morphogen to periodontal tissue at risk of said damage, said concentration being sufficient to inhibit inflammation of said tissue or to maintain the differentiated periodontal phenotype of said tissue.

8. Use according to claim 7 wherein said pharmaceutical is for topical administration or local injection, e.g. being dispersed in an acellular matrix material.

9. Use according to claim 8 wherein said matrix material is derived from dentin, periodontal ligament, bone or cementum tissue.

10. Use according to any one of claims 7 to 9 wherein said pharmaceutical comprises morphogen at a concentration of less than about 50 µg (e.g. less than about 25 µg).

11. Use according to any one of claims 7 to 10 wherein said pharmaceutical comprises: (a) a cofactor that mitigates symptoms associated with periodontal tissue damage, and is e.g. suitable for topical administration or local injection to periodontal tissue, or (b) a biocompatible, porous carrier material for topical application to damaged dentin tissue.

12. Use according to any one of claims 7 to 11 wherein said morphogen is as defined in any one of claims 3-6.

13. A prosthetic or replacment tooth having a morphogen adsorbed onto an exterior root surface thereof such that, when disposed in a mammalian tooth socket comprising residual living periodontal tissue, said morphogen induces periodontal tissue regeneration (for. example, regeneration sufficient to anchor said tooth in said socket).

14. The tooth of claim 13 which is:
(a) a complete prosthesis of natural or synthetic non dentin-containing material; or
(b) a natural tooth from which the root has been removed and replaced with an inert, biocompatible material;
(c) an autologous or allogenic natural tooth, which may e.g. be at least partially demineralized.

15. A composition comprising a morphogen and either:
(a) a cofactor that mitigates symptoms associated with periodontal tissue damage selected from antiseptics, antibiotics with the proviso that tetracycline is excluded anesthetics, non-steroidal anti-inflammatory agents with the proviso that salicyclic acid is excluded and analgesics with the proviso that salicylic is excluded, wherein said morphogen being present at a concentration sufficient to induce periodontal tissue morphogenesis, or
(b) a biocompatible, porous carrier material is a solution of high viscosity for anchoring odontoblasts and for topical application to damaged dentin tissue, wherein the morphogen is adsorbed thereon in an amount sufficient to induce regeneration of dentin tissue when the composition in disposed in vivo at a tooth locus comprising exposed pulp tissue.

## Patentansprüche

1. Zusammensetzung zur Verwendung in der Therapie (beispielsweise zur Hemmung von Verlust oder zur Stimulierung einer Regeneration von Periodontalgewebe bei einem Säugetier), wobei die Zusammensetzung eine therapeutische Konzentration eines Morphogens in Verbindung mit einem Kofaktor umfaßt, der mit einer Periodontalgewebs-Schädigung in Verbindung stehende Symptome lindert, und der aus Antiseptika, Antibiotika mit dem Vorbehalt, daß Tetracyclin ausgeschlossen ist, Anästhetika, nicht steroidalen entzündungshemmenden Mitteln mit dem Vorbehalt, daß Salicylsäure ausgeschlossen ist und Azialgetika mit dem Vorbehalt, daß Salicylsäure ausgeschlossen ist, ausgewählt ist, wobei die Morphogenkonzentration ausreicht, um eine Periodontalgewebs-Morphogenese zu induzieren.

2. Zusammensetzung nach Anspruch 1,
bei der
(I) das Morphogen in einer azellulären Matrix, beispielsweise einer aus Dentin, Knochen, Periodontium oder Zahnzement-Gewebe gewonnenen azellulären Matrix, dispergiert wird, und/oder
(II) die Zusammensetzung eine Lösung mit hoher Viskosität umfaßt.

3. Zusammensetzung nach Anspruch 1 oder 2,
bei der das Morphogen ein dimeres Protein mit zwei Polypeptiden ist, wobei die Aminosäuresequenz von jedem folgendes umfaßt:
(a) eine Sequenz, die zumindest 70% Homologie (beispielsweise zumindest 80% Homologie mit der C-terminalen sieben-Cystein-Domäne von menschlichem OP-1, Reste 38-139 Seq. ID Nr. 5 oder mehr als 60% Identität (beispielsweise mehr als 65% Identität) mit der C-terminalen sechs-Cystein-Domäne von menschlichem OP-1, Reste 43-139 von Seq. ID Nr. 5 teilt; oder
(b) eine durch die generische Sequenz 6, Seq. ID Nr. 31 definierte Sequenz.

4. Zusammensetzung nach Anspruch 3,
bei der die Aminosäuresequenzen zumindest eines der Morphogen-Polypeptide eine Sequenz umfaßen, die aus der C-terminalen sieben-Cystein-Domäne von menschlichem OP-1, Reste 38-139 von Seq. ID Nr. 5 und aus Allel-Spezies und deren biosynthetischen Varianten ausgewählt ist.

5. Zusammensetzung nach Anspruch 3,
bei der die Aminosäuresequenzen der Morphogen-Polypeptide eine durch OPX, Seq. ID Nr. 29, definierte Sequenz umfassen.

6. Zusammensetzung nach einem der Ansprüche 1 - 5, bei der das Morphogen mit zumindest einem Prodomänen-Peptid komplexiert ist, das aus den Prodomänen von Mitgliedern der Morphogen-Familie, einschließlich von Allel-Spezies und deren biosynthetischen Sequenz-Varianten ausgewählt ist, wobei das Prodomänen-Peptid weiterhin aus Peptiden in voller Länge und proteolytischen Bruchstück-Peptiden dieser Prodomänen ausgewählt ist.

7. Verwendung eines Morphogens zur Herstellung eines Arzneimittels zur:
(a) Induktion einer Periodontalgewebs-Morphogenese beim Säugetier, oder
(b) Behandlung einer periodontalen Erkrankung (beispielsweise zur Induktion einer Periodontalgewebs-Morphogenese zur Hemmung eines Gewebsverlust bei einer Periodontalerkrankung) oder
(c) Induktion einer Periodontalgewebs-Morphogenese zur Regenerierung von verlorenem, geschädigtem oder nicht lebensfähigem Periodontalgewebe oder
(d) Erhöhung der Integration eines Zahns in ein Säugetier-Zahnfach, oder
(e) Hemmung einer Periodontalgewebs-Schädigung und eines Zahnverlustes, der mit periodontalen und anderen Zahnfleisch-Erkrankungen in Verbindung steht, oder
(f) Erhöhung der Integration eines Zahnes (beispielsweise eines implantierten allogenen und/oder autogenen Zahnes) in ein Zahnfach eines Säugetieres (beispielsweise ein Zahnfach, das verlorenes, geschädigtes oder nicht lebensfähiges Periodontalgewebe umfaßt), das den Schritt umfaßt, der Zahnfachoberfläche oder der äußeren Zahnwurzel-Oberfläche eine therapeutisch wirksame Konzentration des Morphogens zu verabreichen, wobei die Konzentration ausreicht, um eine Periodontalgewebs-Morphogenese auf der Oberfläche zu induzieren, oder
(g) Vorbereitung eines Zahnes (beispielsweise eines Prothesen-, allogenen und/oder autogenen Zahnes) oder eines Säugetier-Zahnfaches zur Implantation des Zahnes in das Zahnfach, wobei das Zahnfach bezüglich lebensfähigem Periodontalgewebe signifikant vermindert ist, mit den folgenden Schritten: (i) Vorbereitung eines Zahnfaches zur Aufnahme des Zahnes, indem darin eine Oberfläche eines frischen Alveolarknochens freigelegt wird, und (ii) Verabreichung einer therapeutisch wirksamen Konzentration eines Morphogens an eine Oberfläche, die aus der Außenoberfläche der Wurzel des Zahnes (die beispielsweise teilweise demineralisiert ist) und der freigelegten Oberfläche eines frischen Alveolarknochens in dem vorbereiteten Zahnfach ausgewählt ist, wobei die Konzentration ausreicht, um eine Periodontalgewebs-Morphogenese auf der Oberfläche zu induzieren; oder
(h) Implantieren eines Zahnes (beispielsweise eines Prothesen-, allogenen und/oder autogenen Zahnes) in ein Zahnfach eines Säugetiers, wobei das Zahnfach signifikant verringertes lebensfähiges Periodontalgewebe aufweist, wobei das Verfahren die folgenden Schritte umfaßt: (i) Vorbereitung des Zahnfaches zur Aufnahme des Zahnes durch Darin-Freilegen einer frischen Oberfläche eines Alveolarknochens; und (ii) Verabreichen einer therapeutisch wirksamen Konzentration eines Morphogens an eine Oberfläche, die aus der Außenoberfläche der Wurzel des Zahnes (die beispielsweise teilweise demineralisiert ist) und der freigelegten Oberfläche eines frischen Alveolarknochens in dem vorbereiteten Zahnfach ausgewählt ist, wobei die Konzentration ausreicht, um eine Periodontalgewebs-Morphogenese auf der Oberfläche zu induzieren; und (iii) Implantieren des Zahns in das vorbereitete Zahnfach; oder
(i) Regenerieren von Periodontalgewebe in einem Säugetierzahnfach, wobei das Verfahren den Schritt umfaßt, eine therapeutisch wirksame Konzentration eines Morphogens dem Zahnfach zu verabreichen, wobei die Konzentration ausreicht, um darin eine Periodontalgewebs-Morphogenese zu induzieren; oder
(j) Hemmung der mit einer Periodontalerkrankung verbundenen Gewebsschädigung, wobei das Verfahren den Schritt umfaßt, eine therapeutisch wirksame Konzentration eines Morphogens an ein Periodontalgewebe zu verabreichen, das dem Risiko der Schädigung ausgesetzt ist, wobei die Konzentration ausreicht, um eine Entzündung des Gewebes zu hemmen oder den differenzierten periodontalen Phänotyp des Gewebes aufrechtzuerhalten.

8. Verwendung nach Anspruch 7,
bei der das Arzneimittel zur topischen Verabreichung oder lokalen Injektion vorgesehen ist, indem es beispielsweise in einem azellulären Matrixmaterial dispergiert wird.

9. Verwendung nach Anspruch 8,
bei der das Matrixmaterial aus Dentin, Periodontium, Knochen- oder Zahnzement-Gewebe gewonnen wird.

10. Verwendung nach einem der Ansprüche 7 - 9,
bei der das Arzneimittel ein Morphogen in einer Konzentration von weniger als ungefähr 50 µg (beispielsweise weniger als ungefähr 25 µg) umfaßt.

11. Verwendung nach einem der Ansprüche 7 - 10,
bei der das Arzneimittel folgendes umfaßt:
(a) einen Kofaktor, der mit einer Periodontalgewebs-Schädigung verbundene Symptome lindert und beispielsweise zur topischen Verabreichung an oder lokalen Injektion in Periodontalgewebe geeignet ist, oder
(b) ein biokompatibles, poröses Trägermaterial zur topischen Verabreichung an geschädigtes Dentin-Gewebe.

12. Verwendung nach einem der Ansprüche 7 - 11, bei der das Morphogen wie in einem der Ansprüche 3 bis 6 definiert ist.

13. Prothesen- oder Ersatzzahn, der an seiner äußeren Wurzeloberfläche ein Morphogen adsorbiert hat, derartig, daß das Morphogen, wenn es in einem Zahnfach eines Säugetiers angeordnet wird, das einen Rest von lebenden Periodontalgewebe umfaßt, eine Periodontalgewebs-Regeneration (beispielsweise eine Regeneration, die ausreicht, den Zahn in dem Zahnfach zu verankern) induziert.

14. Zahn nach Anspruch 13,
der folgendes ist:
(a) eine vollständige Prothese eines natürlichen oder synthetischen nicht Dentin-enthaltenden Materials; oder
(b) ein natürlicher Zahn, aus dem die Wurzel entfernt und durch ein inertes, biokompatibles Material ersetzt wurde;
(c) ein autogener oder allogener natürlicher Zahn, der beispielsweise zumindest teilweise demineralisiert sein kann.

15. Zusammensetzung mit einem Morphogen und entweder:
(a) einem Kofaktor, der die mit einer Periodontalgewebs-Schädigung verbundenen Symptome lindert, der aus Antiseptika, Antibiotika mit dem Vorbehalt, daß Tetracyclin ausgeschlossen ist, Anästhetika, nicht steroidalen entzündungshemmenden Mitteln mit dem Vorbehalt, daß Salicylsäure ausgeschlossen ist und Analgetika mit dem Vorbehalt, daß Salicylsäure ausgeschlossen ist, ausgewählt ist, wobei das Morphogen in einer Konzentration vorliegt, die ausreicht, um eine Periodontalgewebs-Morphogenese zu induzieren, oder
(b) einem biokompatiblen, porösen Trägermaterial in einer Lösung mit hoher Viskosität zur Verankerung von Odontoblasten und zur topischen Verabreichung an geschädigtes Dentin-Gewebe, wobei das Morphogen darin in einer ausreichenden Menge adsorbiert wird, um eine Regeneration von Dentingewebe zu induzieren, wenn die Zusammensetzung in vivo am Zahnort, der freigelegtes Pulpagewebe umfaßt, angeordnet wird.

## Revendications

1. Composition à usage thérapeutique (par exemple, pour inhiber la déperdition, ou stimuler la régénération, du tissu périodontique chez un mammifère), ladite conposition comprenant une concentration thérapeutique d'un morphogène en association avec un cofacteur qui atténue les symptômes associés aux altérations du tissu périodontique, choisi parmi les antiseptiques, les antibiotiques, à condition que la tétracycline soit exclue, les anesthésiques, les anti-inflammatoires non stéroïdiens, à condition que l'acide salicylique soit exclu, et les analgésiques, à condition que l'acide salicylique soit exclu, ladite concentration de morphogène étant suffisante pour induire la morphogenèse du tissu périodontique.

2. Composition selon la revendication 1, dans laquelle
(I) ledit morphogène est dispersé dans une matrice acellulaire, par exemple, une matrice acellulaire dérivée de la dentine, de l'os alvéolaire, du desmodonte ou du cément, et/ou
(II) ladite composition comprend une solution à viscosité élevée.

3. Composition selon les revendications 1 ou 2, dans laquelle ledit morphogène est une protéine dinère comprenant une Paire de polypeptides, la séquence d'acides aminés de chacun d'eux comprenant :
(a) une séquence partageant une homologie d'au moins 70 % (par exemple, une homologie d'au moins 80 %) avec le domaine à sept cystéines C-terminal du OP-1 humain, résidus 38-139 de la Séq. N° ID 5, ou une identité de plus de 60 % (par exemple, une identité de plus de 65 %) avec le domaine à sept cysteines C-terminal du OP-1 humain, résidus 43-139 de la Séq. N° ID 5 ; ou
(b) une séquence définie par la séquence générique 6, Séq. N° ID 31.

4. Composition selon la revendication 3, dans laquelle les séquences d'acides aminés d'au moins un desdits polypeptides du morphogène comprennent une séquence choisie dans le domaine à sept cystéines C-terminal du OP-1 humain, résidus 38-139 de la Séq. N° ID 5, et ses variantes alléliques, d'espèces et biosynthétiques.

5. Composition selon la revendication 3, dans laquelle les séquences d'acides aminés desdits polypeptides du morphogène comprennent une séquence définie par OPX, Séq. N° ID 29.

6. Composition selon l'une quelconque des revendications 1 à 5, dans laquelle ledit morphogène forme un complexe avec au moins un peptide de prodomaines choisi parmi les prodomaines des membres de la famille du morphogène, comprenant les variantes de séquences alléliques, d'espèces et biosynthétiques de celle-ci, ledit peptide de prodomaines étant, en outre, choisi parmi les peptides complets et à fragments protéolytiques desdits prodomaines.

7. Utilisation d'un morphogène pour la préparation d'une composition pharmaceutique destinée à :
(a) induire la morphogenèse du tissu périodontique des mammifères, ou
(b) traiter la paradontolyse (par exemple, induire la morphogenèse du tissu périodontique afin d'inhiber la perte tissulaire dans la paradontolyse), ou
(c) induire la morphogenèse du tissu périodontique, ou régénérer le tissu périodontique en déperdition, altéré ou non viable, ou
(d) favoriser l'intégration d'une dent dans une alvéole dentaire de mammifères, ou
(e) inhiber l'altération du tissu périodontique et la déperdition de substance dentaire associée aux paradontolyses et autres maladies gingivales, ou
(f) favoriser l'intégration d'une dert (par exemple, une dent implantée, allogénique, et/ou autologue) dans une alvéole dentaire de mammifères (par exemple, une alvéole dentaire dont le tissu périodontique est en déperdition, altéré ou non viable), le procédé comprenant l'étape qui consiste à appliquer une concentration thérapeutiquement efficace du morphogène sur la surface de l'alvéole dentaire ou sur la surface extérieure de la racine de la dent, ladite concentration étant suffisante pour induire la morphogenèse du tissu périodontique sur ladite surface, ou
(g) préparer une dent (par exemple, une fausse dent, une dent allogènique et/ou autologue) ou une alvéole dentaire de mammifères pour l'implantation de ladite dent dans ladite alvéole, ladite alvéole étant significativement appauvrie en tissu périodontique viable, le procédé comprenant les étapes qui consistent à : (i) préparer ladite alvéole dentaire à recevoir ladite dent en exposant une surface d'os alvéolaire frais et (ii) appliquer une concentration thérapeutiquement efficace d'un morphogène sur une surface choisie sur la surface extérieure de la racine de ladite dent (qui est, par exemple, partiellement, déminéralisée) et la surface exposée d'os alvéolaire frais dans ladite alvéole prèparée, ladite concentration étant suffisante pour induire le morphogenèse du tissu périodontique sur ladite surface ; ou
(h) implanter une dent (par exemple, une fausse dent, une dent allogénique et/ou autologue) dans une alvéole dentaire de mammifères, le tissu périodontique viable de ladite alvéole étant significativement réduit, le procédé comprenant les étapes qui consistent à : (i) préparer ladite alvéole dentaire à recevoir ladite dent en exposant une surface fraîche d'os alvéolaire ; et (ii) appliquer une concentration thérapeutiquement efficace d'un morphogène sur une surface choisie sur la surface extérieure de la racine de lacite dent (qui est, par exemple, partiellement, déminéralisée) et la surface exposée d'os alvéolaire frais dans ladite alvéole préparée, ladite concentration étant suffisante pour induire le morphogenèse du tissu périodontique sur ladite surface ; et (iii) implanter ladite dent dans ladite alvéole préparée ; ou
(i) regénérer le tissu périodontique dans une alvéole dentaire de mammifères, le procédé comprenant l'étape qui consiste à appliquer une concentration thérapeutiquement efficace d'un morphogène sur ladite alvéole dentaire, ladite concentration étant suffisante pour induire le morphogenèse du tissu périodontique ; ou
(j) inhiber l'altération tissulaire associée à la paradontolyse, le procédé comprenant l'étape qui consiste à appliquer une concentration thérapeutiquement efficace d'un morphogène sur le tissu périodontique victime de ladite altération, ladite concentration étant suffisante pour inhiber l'inflammation dudit tissu ou pour maintenir le phénotype périodontique différencié dudit tissu.

8. Utilisation selon la revendication 7, dans laquelle ladite composition pharmaceutique est destinée à être administrée localement ou injectée localement, en étant, par exemple, dispersée dans un matériau de matrice acellulaire.

9. Utilisation selon la revendication 8, dans laquelle ledit matériau de matrice est dérivé de la dentine; du desmodonte, de l'os alvéolaire ou du cément.

10. Utilisation selon l'une quelconque des revendications 7 à 9, dans laquelle ladite composition pharmaceutique comprend le morphogène à une concentration inférieure à environ 50 µg (par exemple, inférieure à environ 25 µg).

11. Utilisation selon l'une quelconque des revendications 7 à 10 dans laquelle ladite composition pharmaceutique comprend : (a) un cofacteur qui atténue les symptômes associés à l'altération du tissu périodontique, et est, par exemple, utilisable pour une administration locale ou une injection locale dans le tissu périodontique, ou (b) un matériau de support biocompatible, poreux pour l'application locale sur la dentine altérée.

12. Utilisation selon l'une quelconque des revendications 7 à 11, dans laquelle ledit morphogène est tel que défini dans l'une quelconque des revendications 3 à 6.

13. Fausse dent ou dent de remplacement ayant un morphogène adsorbé sur une surface extérieure de sa racine pour que, une fois logée dans une alvéole dentaire de mammifères comprenant du tissu périodontique vivant résiduel, ledit morphogène induise une régénération du tissu périodontique (par exemple, une régénération suffisante pour ancrer ladite dent dans ladite alvéole).

14. Dent selon la revendication 13 qui est :
(a) une fausse dent complète en un matériau naturel ou synthétique ne contenant pas de dentine ; ou
(b) une dent naturelle dont, la racine a été enlevée et remplacée par un matériau inerte, biocompatible ;
(c) une dent naturelle autologue ou allogénique, qui peut, par exemple, être au moins partiellement déminéralisée.

15. Composition comprenant un morphogène et soit :
(a) un cofacteur qui atténue les symptômes associés à l'altération du tissu périodontique, choisi parni les antiseptiques, les antibiotiques, à condition que la tétracycline soit exclue, les anesthésiques, les anti-inflammatoires non stéroïdiens, à condition que l'acide salicylique soit exclu, et les analgésiques, à condition que l'acide salicylique soit exclu, dans laquelle ledit morphogène est présent à une concentration suffisante pour induire la morphogenèse du tissu périodontique, soit
(b) un matériau de support biocompatible, poreux dans une solution à viscosité élevée afin d'ancrer des odontoblastes et destiné à être appliqué localement sur la dentine altérée, dans lequel le morphogène est adsorbé en une quantité suffisante pour induire une régénération de la dentine lorsque la composition est appliquée in vivo sur un locus dentaire comprenant de la pulpe exposée.
